# EUROPEAN PATENT APPLICATION

(11) **EP 2 468 887 A1**
(43) Date of publication of application: **27.06.2012**
(21) Application number: 11185104.4
(22) Date of filing: 12.12.2007
(51) Int. Cl.: C12Q 1/68

(54) **TTK as tumor marker and therapeutic target for lung cancer**

(30) Priority: 13.12.2006 US 874791 P
(62) Divisional of application: 07859848.9
(71) Applicant: Oncotherapy Science, Inc., Kawasaki-shi Kanagawa 213-0012 (JP)
(72) Inventor: Nakamura, Yusuke, Tokyo, 113-8654 (JP); Daigo, Yataro, Tokyo, 113-8654 (JP); Nakatsuru, Shuichi, Kanagawa, 213-0012 (JP)
(74) Representative: Vossius & Partner

(57) **Abstract**

Disclosed herein is a method for determining kinase activity of TTK for EGFR and methods of screening for modulators of this kinase activity. Also disclosed are methods and pharmaceutical compositions for preventing and/or treating lung cancer that use or include such modulators. Methods for diagnosing lung cancer using the kinase activity of TTK for EGFR protein as an index as well as methods for assessing and prognosing lung cancer are also provided.

## Description

### PRIORITY

The present application claims the benefit of U.S. Provisional Application No. 60/874,791, filed December 13, 2006, the entire disclosure of which is hereby incorporated herein by reference for all purposes.

### FIELD OF THE INVENTION

The present invention relates to lung cancer, more particularly the diagnosis and treatment thereof.

### BACKGROUND OF THE INVENTION

Lung cancer is one of the most common causes of cancer death worldwide, and non-small cell lung cancer (NSCLC) accounts for nearly 80% of those cases (Greenlee, R. T., et al., (2001) CA Cancer J Clin, 51: 15-36.). Small cell lung cancer (SCLC) comprises 15-20% of all lung cancers (Chute JP et al., (1999) J Clin Oncol.; 17:1794-801, Simon GR et al., (2003) Chest.; 123(1 Suppl):259S-271S). Although many genetic alterations associated with the development and progression of lung cancer have been reported, but precise molecular mechanisms remain unclear (Sozzi, G. Eur J Cancer, (2001) 37 Suppl 7: S63-73.). Over the last decade, newly developed cytotoxic agents, including paclitaxel, docetaxel, gemcitabine, and vinorelbine, have emerged to offer multiple therapeutic choices for patients with advanced NSCLC; however, each of the new regimens can provide only modest survival benefits as compared to cisplatin-based therapies (Schiller, J. H. et al. (2002) N Engl J Med, 346: 92-8.; Kelly, K., et al. (2001) J Clin Oncol, 19: 3210-8.). Hence, the development of new therapeutic strategies, such as molecular-targeted agents and antibodies, and cancer vaccines, are eagerly anticipated.

Systematic analysis of expression levels of thousands of genes using cDNA microarray technology provides an effective approach for identifying unknown molecules involved in pathways of carcinogenesis, and can reveal candidate targets for the development of novel therapeutics and diagnostics. Attempts to isolate novel molecular targets for diagnosis, treatment and prevention of NSCLC by analyzing genome-wide expression profiles of NSCLC cells on a cDNA microarray containing 27,648 genes, using pure populations of tumor cells prepared from 101 lung cancer tissues by laser-capture microdissection, are ongoing (Kikuchi T, et al. Oncogene. 2003 Apr 10;22(14):2192-205.; Kikuchi T, et al. Int J Oncol. 2006 Apr; 28(4):799-805.; Kakiuchi S, et al., Mol Cancer Res. 2003 May;1(7):485-99.; Hum Mol Genet. 2004 Dec 15;13(24):3029-43. Epub 2004 Oct 20.; Taniwaki M, et al, Int J Oncol. 2006 Sep;29(3):567-75.). In the course of this genome wide cDNA microarray analysis, 642 up-regulated genes and 806 down-regulated genes have been identified as diagnostic markers and therapeutic targets for NSCLC (See WO 2004/31413, the contents of which are incorporated by reference herein).

Epidermal growth factor receptor (EGFR) plays a critical role in the growth and survival of human cancers in various tissues by stimulation of ligands such as EGF that, in turn, leads to autophosphorylation of EGFR and thus activates the EGFR signaling pathway. Through cDNA and tissue microarrays analyses, TTK has been identified as over-expressed in the great majority of lung cancers and has further been shown to be associated with poor prognosis. Furthermore, suppression of endogenous TTK expression by treatment with siRNA has been shown to cause significant growth inhibition of non-small cell lung cancer cells. Screening of potential substrates for TTK kinase using a panel of antibodies against phospho-proteins related to cancer-cell signaling resulted in the identification of an EGFR as an intracellular target of TTK. It was further discovered that phosphorylation at Tyr-992 and Ser-967 of EGFR by TTK occurred independently of EGF stimulation, and led to the activation of PLCgamma and phosphorylation of MAPK. In addition, point mutations were identified in the tyrosine kinase domain of the TTK gene in two patients with metastatic brain tumors derived from primary lung adenocarcinoma and in a lung-cancer cell line RERF-LC-AI. *In vitro,* the TTK mutant increased the invasive ability of mammalian cells. Together, these data imply that TTK functions as oncogene and its activation is likely to play an important role in the intracellular stimulation of EGFR-MAPK signaling in cancer cells, and that a novel intracellular signaling pathway between TTK kinase and EGFR, independent from the presence of EGF, plays a significant role in pulmonary carcinogenesis. Thus, the present invention suggests that targeting the TTK enzymatic activity will be a promising therapeutic strategy for treatment of lung-cancer patients.

### SUMMARY OF THE INVENTION

In the course of screening for novel molecular targets for diagnosis, treatment and prevention of human cancers, genome-wide expression profile analyses of 101 lung cancers was performed on cDNA microarray containing 27,648 genes, coupled with laser microdissection (Kikuchi T, et al. Oncogene. 2003 Apr 10;22(14):2192-205.; Kikuchi T, et al. Int J Oncol. 2006 Apr;28(4):799-805.; Kakiuchi S, et al., Mol Cancer Res. 2003 May; 1(7):485-99.; Kakiuchi S, et al., Hum Mol Genet. 2004 Dec 15;13(24):3029-43. Epub 2004 Oct 20.; Taniwaki M, et al, Int J Oncol. 2006 Sep;29(3):567-75.). The results demonstrate that the gene encoding the TTK protein kinase (alias hMps1) is frequently over-expressed in the great majority of primary lung cancers.

Epidermal growth factor receptor (EGFR) has been recognized as an important mediator of growth signaling pathways (Carpenter G. Annu Rev Biochem. 1987;56:881-914.; Wells C. Int J Biochem Cell Biol. 1993 Jun;31(6):637-43.). Aberrant EGFR activity, arising from genetic and epigenetic changes, has been shown to enhance cell proliferation and cause tumor progression in many tumors (Salomon DS, et al., Crit Rev Oncol Hematol. 1995 Jul;19(3):183-232.; Mendelson J. Clin Cancer Res. 2000 Mar;6(3):747-53.). Therefore, agents that selectively block EGFR signaling have been under development; to that end, anti-EGFR monoclonal antibody, cetuximab (Erbitux), and small-molecule inhibitors of EGFR tyrosine kinase such as gefitinib (Iressa) and erlotinib (Tarceva), have been used in clinical practice (Dowell J, et al., Nat Rev Drug Discov. 2005 Jan;4(1):13-4.; Herbst RS, et al., Nat Rev Cancer. 2004 Dec;4(12):956-65.). Stimulation of its ligands, such as EGF, causes EGFR to undergo a conformational change and autophosphorylation that activates the EGFR signaling pathways includes the MAPK (mitogen activated protein kinase) cascade and the c-Src (cellular Src) cascade (Yarden Y. Eur J Cancer. 2001 Sep;37 Suppl 4:S3-8.; Pal SK & Pegram M. Anticancer Drugs. 2005 Jun;16(5):483-94.; Tice DA, et al., Proc Natl Acad Sci U S A. 1999 Feb 16;96(4):1415-20.). c-Src phosphorylates the cytoplasmic tail of EGFR in the presence of EGF and activates the EGFR signals (Yarden Y. Eur J Cancer. 2001 Sep;37 Suppl 4:S3-8.; Pal SK & Pegram M. Anticancer Drugs. 2005 Jun;16(5):483-94.; Tice DA, et al., Proc Natl Acad Sci U S A. 1999 Feb 16;96(4):1415-20.). However, no kinase that phosphorylates EGFR and consequently activates the EGFR pathways in an EGF-independent manner has yet been reported.

The evidence disclosed herein demonstrates that TTK plays a significant role in pulmonary carcinogenesis through EGF-independent phosphorylation of EGFR Tyr-992 and Ser-967, and subsequent activation of downstream MAPK signals that are considered to be indispensable for tumor growth/survival. These data suggest that novel signaling between TTK and EGFR, independent of the presence of EGF, represents a potential target for development of novel therapeutic drugs for lung cancer.

Also disclosed herein are variant TTK proteins composed of various amino acid substitutions, for example, an exchange from leucine to proline at codon 72 (L72P), serine to threonine at codon 76 (S76T), tyrosine to cysteine at codon 574 (Y574C), proline to Glutamine at codon 789 (P789Q) and lysine to Isoleusine at codon 856 (K856I) (**Table 4**). A missense mutation at codon 574 (Y574C) on the TTK kinase domain found in a RERF-LC-AI cell line was not present in the SNP databases (JSNP: http://snp.ims.u-tokyo.ac.jp/index_ja.html; DBSNP: http://www.ncbi.nlm.nih.gov/projects/SNP/). In addition, two missense mutations were identified in the TTK kinase domain in clinical samples of two metastatic brain tumors derived from primary lung adenocarcinoma. The mutations resulted in two amino acid substitution, namely the substitution of Valine to Phenylalanine at codon 610 (V610F) and Glutamine to Histidine at codon 753 (Q753H). Matched normal brain tissue was available for these two patients and showed only the wild-type DNA sequence, indicating that the mutations had arisen somatically during tumor formation or progression.

The mutant-TTK (Y574C) transfected cells showed a high-autophosphorylation level as compared to non-transfected cells, indicating that the mutation could promote the TTK kinase activity. Furthermore, it was confirmed that the invasive ability of mutant-TTK (Y574C) transfected cells was significantly enhanced by matrigel invasion assay using the mutant-TTK construct. These results doubtlessly indicate that the TTK mutation originated from RERF-LC-AI cell could be an activating mutation involved in lung carcinogenesis.

**Table 4. List of TTK mutation in lung-cancer cell lines.**

| **Histol ogy** | **Cell line** | **Nucleotide location (*) (Amino acid)** | | | | | | | | |
|---|---|---|---|---|---|---|---|---|---|---|
| | | T288C (L72P) | G302C (S76T) | T581C (A169A) | T1655A (!) (1527I) | A1796G # (Y574C) | C2441A # (!) (P789Q) | A2641T # (K856I) | A2671G (!) (**) | T2823C (!) (**) |
| ADC | A427 | | | | Hetero. | | Hetero. | | Hetero. | Homo. |
| | A549 | | | | Homo. | | Homo. | | Homo. | Homo. |
| | **LC174** | | | | | | Homo. | | | |
| | **LC176** | | | | | | Homo. | | | |
| | LC319 | | | | Homo. | | Homo. | Hetero. | Homo. | Homo. |
| | PC-3 | | | | Homo. | | Homo. | | Homo. | Homo. |
| | PC-9 | | | | Homo. | | Homo. | Hetero. | Homo. | Homo. |
| | PC14 | Hetero. | Hetero. | | Homo. | | Homo. | | Homo. | Homo. |
| | **PC14- PE6** | | | | Homo. | | Homo. | | | |
| | **SK-LU-1** | | | | | | Homo. | | | |
| | **NCI- H23** | | | | Homo. | | Homo. | | | |
| | **NCI-H522** | | | | | | | | | |
| | NCI-H1373 | | | | Homo. | | Homo. | | Homo. | Homo. |
| | **NCI-H1435** | | | | | | | | | |
| | **NCI-H1793** | | | | Homo. | | Homo: | | | |
| BAC | **SW1573** | | | | | | Homo. | | | |
| | **NCI-H358** | | | | | | | | | |
| | **NCI-H1650** | | | | | | Homo. | | | |
| | NCI-H1666 H1666 | | | | Homo. | | Homo. | Hetero. | Homo. | Homo. |
| | NCI-H1781 | | | | Homo. | | Homo. | Hetero. | Homo. | Homo. |
| SCC | RERF-LC-AI | | | | Homo. | Homo. | Homo. | Hetero. | Homo. | Homo. |
| | SK-MES-1 | | | | Homo. | | Homo. | | Homo. | Homo. |
| | EBC-1 | | | | Homo. | | Homo. | Hetero. | Homo. | Homo. |
| | LU61 | | | | Homo. | | Homo. | Hetero. | Homo. | Homo. |
| | **SW900** | | | | | | Homo. | | | |
| | NCI-H520 | | | | Homo. | | Homo. | | Homo. | Homo. |
| | NCI-H1703 | | | | Hetero. | | Hetero. | Hetero. | Hetero. | Homo. |
| | NCI- H2170 | | | | Hetero. | | Hetero. | | Hetero. | Hetero. |
| ASC | NCI-H226 | | | | Hetero. | | Hetero. | | Hetero. | Hetero. |
| | **NCI- H596** | | | | Homo. | | | | | |
| | NCI-H647 | | | | Homo. | | Homo. | | Homo. | Homo. |
| LCC | LX1 | | | Hetero. | Homo. | | Homo. | Hetero. | Homo. | Homo. |
| SCLC | DMS11 4 | | | | Homo. | | Homo. | Hetero. | Homo. | Homo. |
| | 3 DMS27 | | | | Homo. | | Homo. | Hetero. | Homo. | Homo. |
| | SBC-3 | | | | Homo. | | Homo. | Hetero. | Homo. | Homo. |
| | SBC-5 | | | | Homo. | | Homo. | | Homo. | Homo. |

| | | | | | | | | | | |
|---|---|---|---|---|---|---|---|---|---|---|
| (*) : location from transcription start site (**) : non coding region # : location in kinase domain (!): previously reported | | | | | | | | | | |

Thus, the present invention is based, in part, on the discovery of the EGF-independent phosphorylation of EGFR Tyr-992 and Ser-967 by TTK, and subsequent activation of downstream MAPK signals that are considered to be indispensable for tumor growth and/or survival.

Accordingly, the present invention provides a method of diagnosing lung cancer or a predisposition for developing lung cancer in a subject, including the step of determining TTK expression level and a level of kinase activity of TTK for EGFR in a biological sample derived from the subject, wherein an increase in said level as compared to a normal control level indicates that the subject suffers from or is at risk of developing lung cancer. In particular, the kinase activity of TTK for EGFR is EGF-independent and one of the phosphorylation sites of EGFR is Tyr-992 or Ser-967.

The present invention also provides methods of assessing or determining a lung cancer prognosis. In some embodiments, the method includes the steps of:
a. detecting a TTK expression level and/or phospho-EGFR level in a specimen collected from a subject whose lung cancer prognosis is to be assessed or determined, and
b. indicating a poor prognosis when an elevated level of TTK expression and/or phospho-EGFR level is detected.

In particular, the kinase activity of TTK for EGFR is EGF-independent and the phosphorylation site of EGFR is Tyr-992 or Ser-967.

In a further embodiment, the present invention features a method of measuring TTK kinase activity, the method involving the incubation of polypeptides under conditions suitable for a phosphorylation of EGFR by TTK. Suitable polypeptides include a TTK polypeptide or functional equivalent thereof and an EGFR polypeptide or functional equivalent thereof.

For example, the TTK polypeptide may possess the amino acid sequence of SEQ ID NO: 2. Alternatively, the TTK polypeptide may possess an amino acid sequence of SEQ ID NO: 2, where one or more amino acids are modified by substitution, deletion or insertion, so long as the resulting polypeptide retains the biological activity of the polypeptide of SEQ ID NO: 2. Biological activities of the polypeptide of SEQ ID NO: 2 include, for example, the promotion of cell proliferation and the kinase activity of TTK for EGFR. Additionally, the polypeptide may take the form of an 857-amino acid protein encoded by the open reading frame of SEQ. ID. NO. 1, or a polynucleotide that hybridizes under stringent conditions, e.g., low or high, to the nucleotide sequence of SEQ ID NO: 1, so long as the resulting polynucleotide encodes a protein that retains the biological activity of the polypeptide of SEQ ID NO: 2, e.g. the region including Asp-647 of SEQ ID NO: 2.

The EGFR polypeptide may possess the amino acid sequence of SEQ ID NO; 4 (GenBank Accession No. NP_005219). In the cells, EGFR is cleavage at the N-terminal domain and forms an 1186 residue protein (SEQ ID NO: 42). The EGFR polypeptide may possess an amino acid sequence of SEQ ID NO: 4, wherein one or more amino acids are modified by substitution, deletion or insertion, so long as the resulting polypeptide retains the target region of TTK kinase on SEQ ID NO: 4, e.g. the region including Tyr-992 and Ser-967 at cleavage type of EGFR. Additionally, the EGFR polypeptide may take the form of a 1210-amino acid protein encoded by the open reading frame of SEQ. ID. NO. 3 (GenBank Accession No. NM_005228), or a polynucleotide that hybridizes under stringent conditions, e.g. low or high, to the nucleotide sequence of SEQ ID NO: 3, so long as the resulting polynucleotide encodes a protein that retains the target site of TTK kinase on SEQ ID NO: 4.

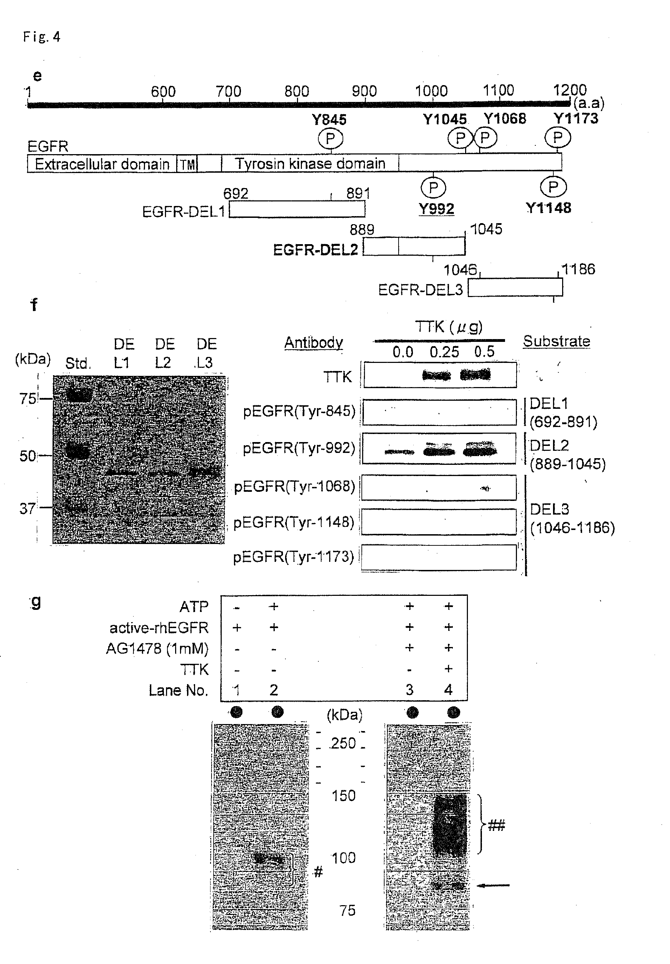

In the context of the present invention, the kinase activity of TTK for EGFR can be defined by the detection of the phospho-EGFR, especially phosphorylated at Tyr-992 (**Figure 4e**). The kinase activity of TTK for EGFR may be detected by conventional methods, such as western-blot analysis using an antibody for phospho-EGFR. The phosphorylation, may occur either *in vitro* or *in vivo.* In the context of *in vitro* phosphorylation, purified recombinant TTK polypeptide can be incubated with whole extracts prepared from cell lines or recombinant EGFR polypeptide with ATP as a phosphate donor. In the context of *in vivo* phosphorylation, cells that endogenously or exogenously co-expressing TTK and EGFR may be used. Suitable conditions for synthesis include, for example, basic buffer conditions know in the art such as Tris-HCl.

The present invention further provides methods of identifying an agent that modulates (e.g., increases or decreases) kinase activity of TTK for EGFR is detected by incubating a TTK polypeptide, or functional equivalent thereof and EGFR polypeptide, or functional equivalent thereof in the presence of ATP as a phosphate donor and determining the phospho-EGFR level. A decrease in the phospho-EGFR level as compared to a normal control level indicates that the test agent is an inhibitor of TTK kinase. Compounds that inhibit (*e.g*., decreases) kinase activity of TTK for EGFR are useful for treating, preventing or alleviating a symptom of lung cancer. For example, such compounds may inhibit the proliferation of lung cancer cells. Alternatively, an increase in the level or activity as compared to a normal control level indicates that the test agent is an enhancer of kinase activity of TTK for EGFR. Herein, the phrase normal control level refers to a level of kinase activity of TTK for EGFR detected in the absence of the test compound. For example, phosphorylation of EGFR by TTK is EGF-independent and examples of the phosphorylates sites of EGFR are Tyr-992 and Ser-967, and so on.

The present invention also encompasses compositions and methods for treating or preventing of lung cancer by contacting a lung cancer cell with a compound identified as described above. In a further embodiment, the present invention provides for the use of a compound identified as described above, for manufacturing a pharmaceutical composition suitable for treating or preventing lung cancer. For example, a method of treating lung cancer may involve the step of administering to a mammal, *e.g*. a human patient having been diagnosed with such a disease state, with a composition containing a pharmaceutically effective amount of a compound identified as described above and a pharmaceutical carrier.

The present invention also provides a kit for the detecting the kinase activity of TTK for EGFR. The reagents are preferably packaged together in the form of a kit. The reagents may be packaged in separate containers and may include, for example, a TTK polypeptide, an EGFR polypeptide, reagent for detecting a phospho-EGFR, e.g. phospho-EGFR (Tyr992) or phospho-EGFR (Ser967), a control reagent (positive and/or negative), and/or a detectable label. Instructions (*e.g*., written, tape, VCR, CD-ROM, *etc*.) for carrying out the assay are preferably included in the kit. The assay format of the kit may include any kinase assay known in the art.

The present invention is based in part on the discovery of the various amino acid substitutions of TTK at kinase domain, especially the mutations resulted in the amino acid substitution; Valine to Phenylalanine at codon 610 (V610F) and Glutamine to Histidine at codon 753 (Q753H), which are available for these two patients respectively, and a missense mutation Tyrosine to Cysteine at codon 574 (Y574C) on the TTK kinase domain found in a RERF-LC-AI cell line, which was not present in the SNP databases. Because the mutation of TTK (Y574C or Q753H) could promote the TTK kinase activity and the invasive ability, the mutation of TTK (Y574C or Q753H) originated from RERF-LC-AI cell could be an activating mutation involved in lung carcinogenesis. Accordingly, the present invention provides the method of predicting a metastasis of lung cancer, especially a brain metastasis of lung cancer, by using the mutations of kinase domain of TTK, *e.g*. Y574C or Q753H as index.

In other embodiment, the present invention also provides A method for treating or preventing lung cancer comprising administering to subject a composition comprising a double-stranded molecule which reduces TTK (SEQ ID NO: 1) or EGFR (SEQ ID NO: 3) gene expression, wherein the double-stranded molecule comprises a sense nucleic acid and an anti-sense nucleic acid, wherein the sense nucleic acid comprises a ribonucleotide sequence corresponding to a sequence of SEQ ID NO: 62 or 63 as the target sequence.

Alternatively, the present invention also provides a composition for treating or preventing lung cancer comprising a pharmaceutically effective amount of a double-stranded molecule which reduces TTK (SEQ ID NO: 1) or EGFR (SEQ ID NO: 3) gene expression, and a pharmaceutically acceptable carrier.

In addition, the present invention provides inhibitory polypeptides that are selected from group of ISSILEKGERLPQPPICTI (SEQ ID NO: 4-4), DVYMIMVKCWMIDADSRPK (SEQ ID NO. 45) and FRELIIEFSKMARDPQRYL (SEQ ID NO. 46). The present invention further provides pharmaceuticals or methods using these inhibitory polypeptides for prevention and/or treatment of lung cancer.

The present invention also relates to methods for treatment and/or prevention of lung cancer comprising the step of administering an inhibitory polypeptide that is selected from group of ISSILEKGERLPQPPICTI (SEQ ID NO: 44), DVYMIMVKCWMIDADSRPK (SEQ ID NO. 45) and FRELIIEFSKMARDPQRYL (SEQ ID NO. 46), or a polynucleotide encoding the same.

These and other objects and features of the invention will become more fully apparent when the following detailed description is read in conjunction with the accompanying figures and examples. However, it is to be understood that both the foregoing summary of the invention and the following detailed description are of a preferred embodiment, and not restrictive of the invention or other alternate embodiments of the invention. In particular, while the invention is described herein with reference to a number of specific embodiments, it will be appreciated that the description is illustrative of the invention and is not constructed as limiting of the invention. Various modifications and applications may occur to those who are skilled in the art, without departing from the spirit and the scope of the invention, as described by the appended claims. Likewise, other objects, features, benefits and advantages of the present invention will be apparent from this summary and certain embodiments described below, and will be readily apparent to those skilled in the art. Such objects, features, benefits and advantages will be apparent from the above in conjunction with the accompanying examples, data, figures and all reasonable inferences to be drawn therefrom, alone or with consideration of the references incorporated herein.

Regarding the specific aims and objectives recited above, it will be understood by those skilled in the art that one or more aspects of this invention can meet certain objectives, while one or more other aspects can meet certain other objectives. Each objective may not apply equally, in all its respects, to every aspect of this invention. As such, the objects herein can be viewed in the alternative with respect to any one aspect of this invention.

### BRIEF DESCRIPTION OF THE DRAWINGS

Various aspects and applications of the present invention will become apparent to the skilled artisan upon consideration of the brief description of the figures and the detailed description of the present invention and its preferred embodiments which follows:

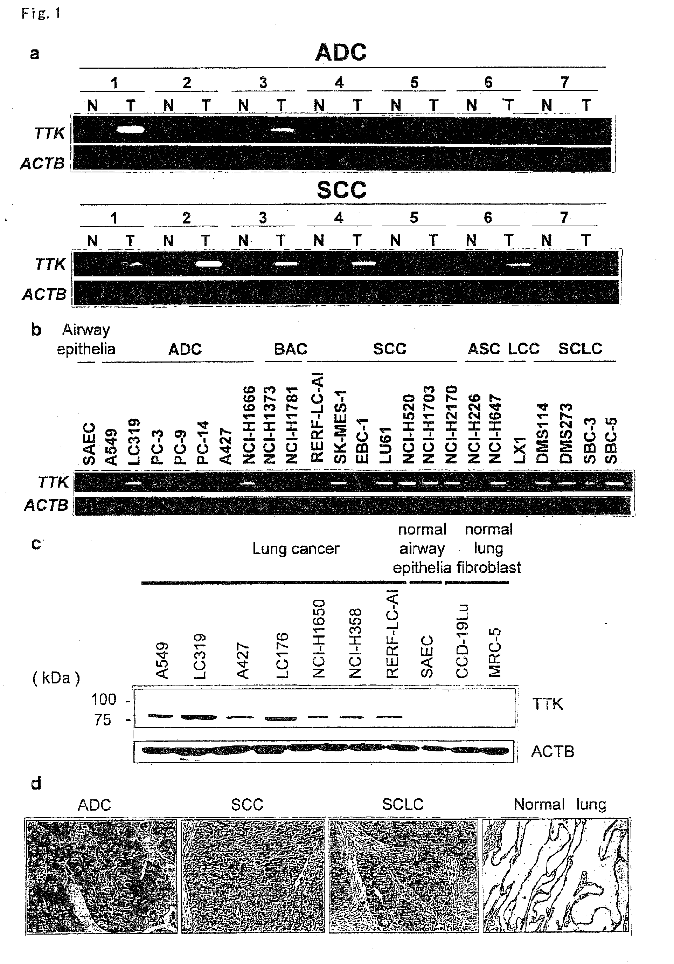

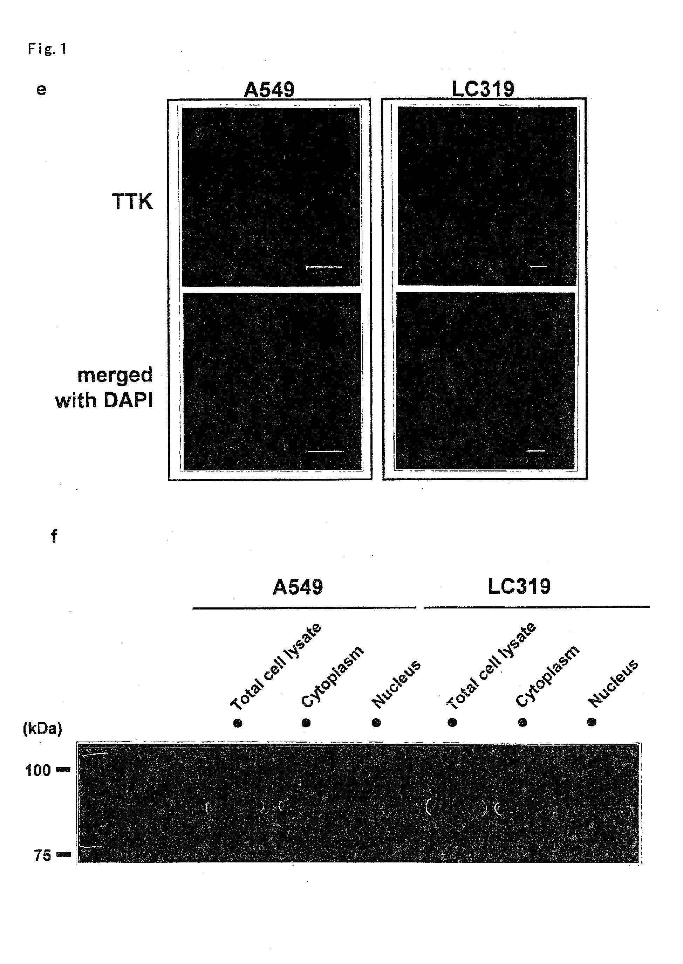

**Figure 1** **depicts the validation of TTK expression in primary lung cancers and cell lines.**
   **Part a** depicts the expression of TTK in clinical samples of NSCLC (T) and corresponding normal lung tissues (N), examined by semiquantitative RT-PCR.
   **Part b** depicts the expression of TTK in lung-cancer cell lines by semiquantitative RT-PCR.
   **Part c** depicts the expression of TTK protein in 7 lung-cancer cell lines, normal airway epithelial cells (SAEC), and two normal lung fibroblast cells, (CCD19Lu and MRC-5) detected by western-blot analysis.
   **Part d** provides representative images of immunohistochemical analysis of TTK protein in lung adenocarcinomas (ADC), squamous-cell carcinomas (SCC), and small cell lung cancer (SCLC) tissues. Magnification, x200.
   **Part e** depicts the expression of TTK in A549 (**left panels**) and LC319 cells (**right panels**) by immunocytochemical analyses. Cells were fixed and stained using anti-TTK antibody and Alexa Fluor 488-conjugated goat anti-mouse IgG as secondary antibody. TTK was visualized in green and the cell nuclei in blue (DAPI).
   **Part f** depicts the results of western blot analyses with anti-TTK antibody, confirming that TTK localizes in the cytosolic and nuclear fraction of A549 (**left panels**) and LC319 cells (**right panels**).

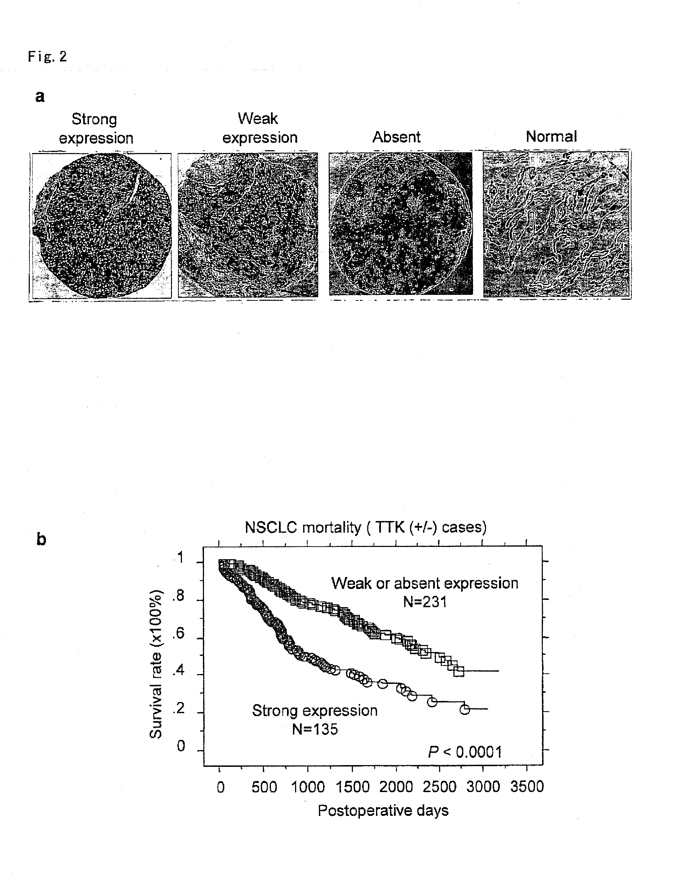

**Figure 2** **depicts the TTK expression in primary lung cancers and its prognostic value.**
   **Part a** depicts the results of immunohistochemical evaluation of TTK protein expression on tissue microarrays. Examples are shown for strong, weak, or absent TTK expression in lung SCCs, and for no expression in normal lung. Magnification, x100.
   **Part b** depicts the results of Kaplan-Meier analysis of tumor-specific survival in patients with non-small cell lung cancer (NSCLC) according to TTK expression (P < 0.0001 by the Log-rank test).

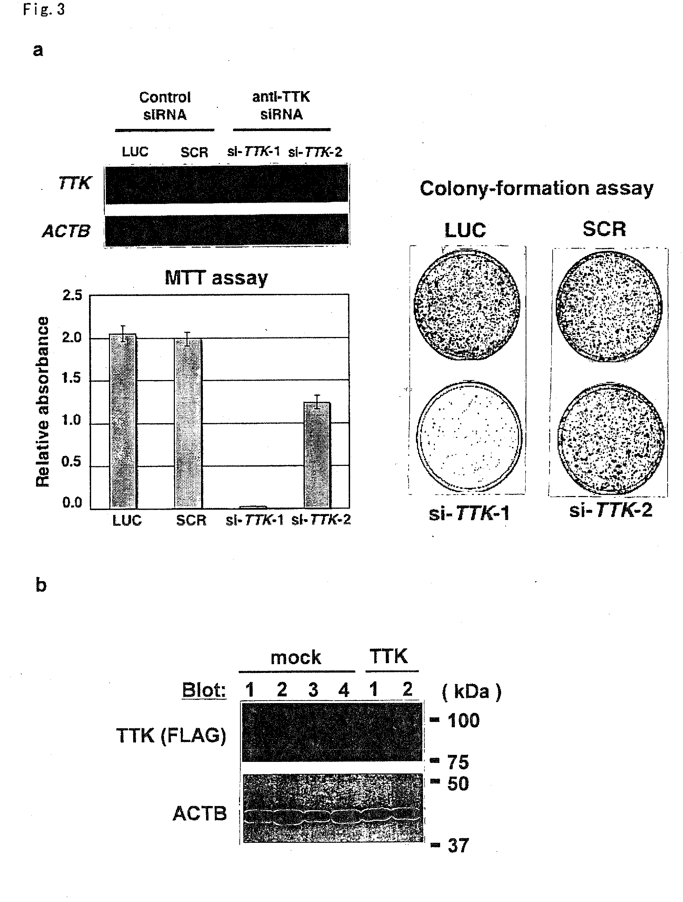

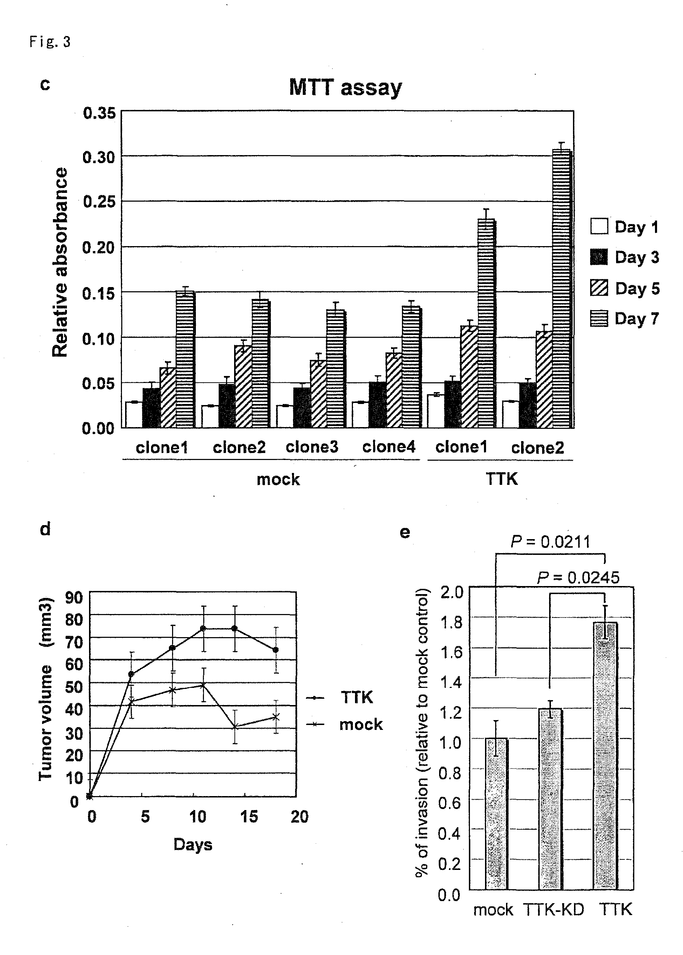

**Figure 3** **depicts the growth-promoting effect of TTK and activation of cellular invasive activity by TTK.**
   **Part a** depicts the expression of TTK in response to si-TTKs (si-TTK-1, -2) or control siRNAs (luciferase (LUC), or scramble (SCR)) in LC319 cells, analyzed by semiquantitative RT-PCR (**left upper panels**). Viability of LC319 cells evaluated by MTT assay in response to si-TTKs, - LUC, or -SCR (**left lower panels**). Colony-formation assays of LC319 cells transfected with specific siRNAs or control plasmids (**right lower panels**).
   **Part b** depicts the expression of TTK protein in TTK-stable transfectants of HEK293 cells on western-blot analysis.
   **Part c** depicts the transfectants expressing low levels (clone 1) or high levels (clone 2) of TTK, or controls cells transfected with mock vector were each cultured in triplicate; at each time point, the cell viability was evaluated by MTT assay.
   **Part d** depicts the growth curves of TTK-stable transfectants of HEK293 cells or mock-HEK293 cells transplanted to subcutaneous of nude mice (5 x 10⁶ TTK-transfected HEK293 cells/mouse).
   **Part e** depicts the results of Matrigel invasion assay demonstrating the increased invasive ability of NIH-3T3 cells transfected with TTK-, the catalytically inactive TTK-KD (kinase dead)-, or mock-vector. The number of invading cells through Matrigel-coated filters was shown.

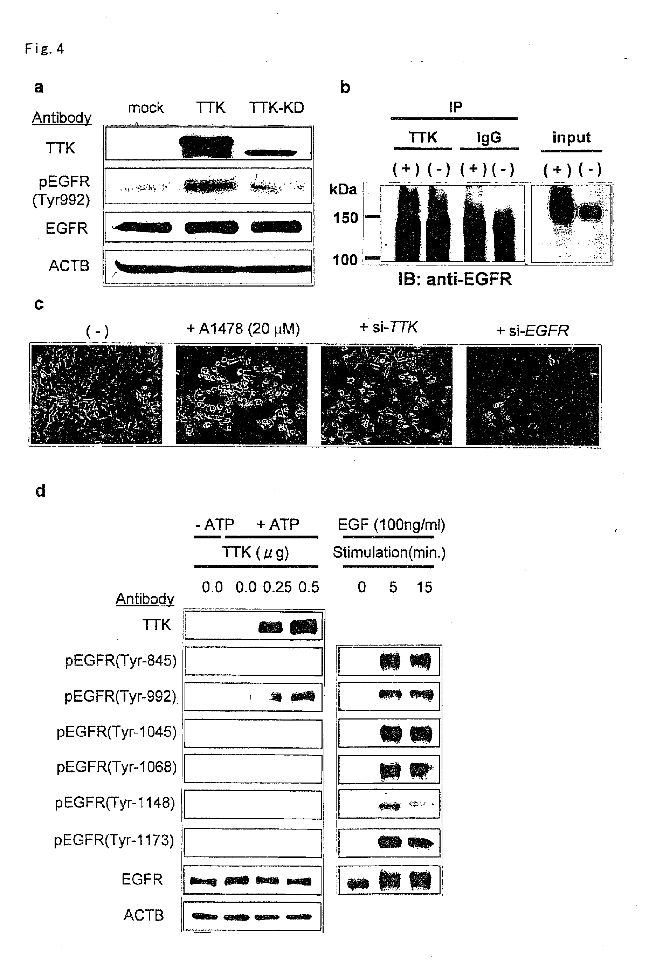

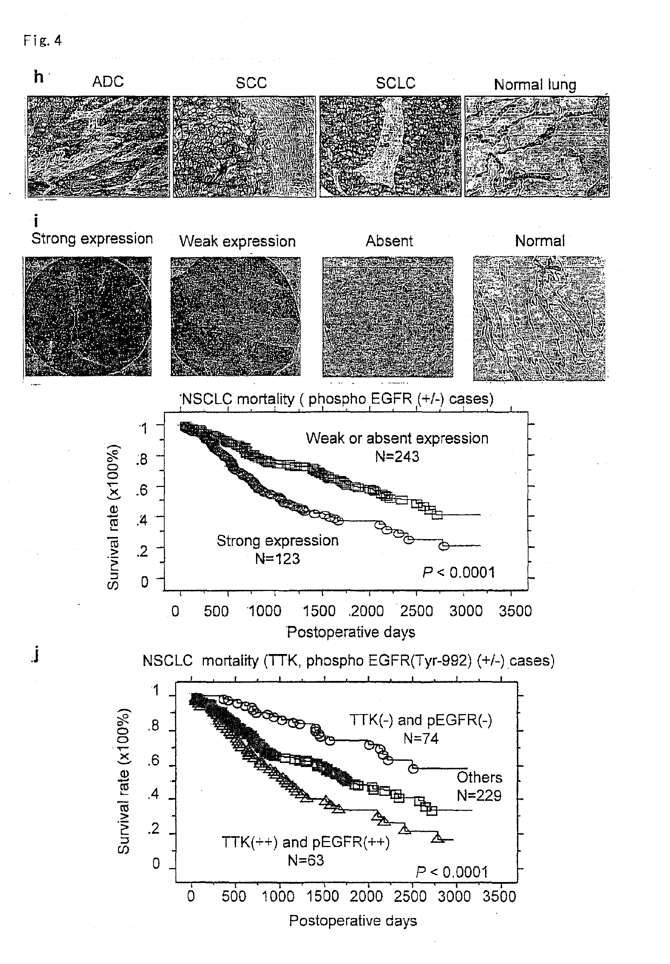

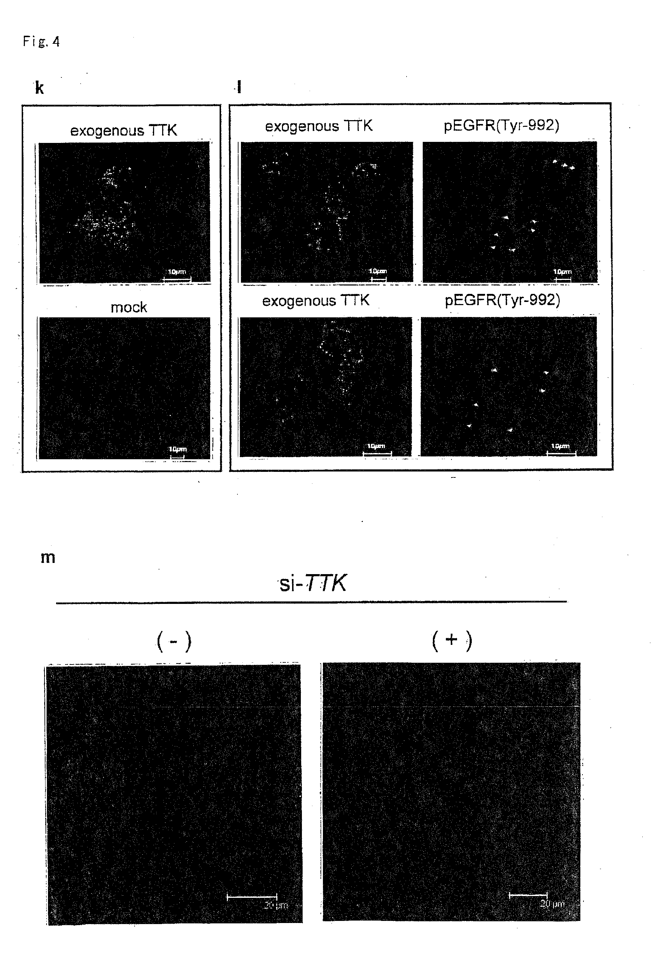

**Figure 4** **relates to the direct phosphorylation of EGFR on Tyr-992 by TTK protein kinase.**
   **Part a** depicts results of Tyr-992 phosphorylation of EGFR in COS-7 cells that transiently over-expressed TTK. COS-7 cells that scarcely expressed endogenous TTK were transfected with the TTK-expression vector, the catalytically inactive TTK-KD (D647A)-expression vector, or mock vector. Whole cell extracts from these cells were used for western-blot analysis using a total of 31 antibodies against various phospho-proteins involved in cancer-cell signaling (see **Table 2**). Blots were stripped and re-probed for total EGFR or ACTB to verify equal loading; all bands of EGFR are about 175 kDa.
   **Part b** depicts the results of interaction of endogenous TTK with EGFR in lung cancer cells. Immunoprecipitations were performed using anti-TTK antibodies and extracts from A549 cells in the absence or presence of EGF (100 nM). Immunoprecipitates were subjected to western blot analysis to detect endogenous EGFR. IP, immunoprecipitation; IB, immunoblot.
   **Part c** depicts the results of phase contrast images of A549 cells treated with AG1478, or transfected with siRNA (oligo) against TTK, or siRNA (oligo) against EGFR. Non-treated A549 cells were served as controls.
   **Part d** depicts the results of in vitro kinase assay by incubating purified recombinant TTK protein with whole cell lysates isolated from COS-7 cells. After in vitro kination reaction, the samples were subjected to western-blot analysis with anti-phospho-EGFR antibodies (Tyr-845, Tyr-992, Tyr-1045, Tyr-1068, Tyr-1148, and Tyr-1173). Blots were stripped and re-probed for total EGFR or ACTB to verify equal loading (**left panels**). COS-7 cells maintained in serum-free medium for 24 hours were exposed to EGF (100 nM) for 5 or 15 min at 37°C. COS-7 cells without exposure to the EGF treatment were served as a control. Whole cell extracts from these cells were used for western-blot analysis with these anti-phospho-EGFR antibodies (**right panels**); all bands shown are around 175 kDa.
   **Part e** is a schemaic representation of the EGFR-deletion mutants (DELs). GST fusion proteins with three partial EGFR sequences at cytoplasmic region were constructed (deletion mutants). Individual mutants are shown as a bar with amino acid residue number at both ends. Locations of the extracellular domain, transmembrane region (TM), tyrosine kinase domain, Src phosphorylation site (Tyr-845), and tyrosine autophosphorylation sites (Tyr-992, Tyr-1045, Tyr-1068, Tyr-1148, and Tyr-1173) are indicated. All three EGFR deletion mutants (EGFR DELs) are kinase-deficient constructs.
   **Part f** depicts the results of three recombinant EGFR-DELs loaded on SDS-PAGE were visualized by Coomassie Brilliant Blue staining (**upper panel**). In vitro kinase assay by incubating purified recombinant TTK with three deletion mutants of EGFR as substrates (**lower panels**). After the kinase reaction, samples were subjected to western-blot analysis with anti-phospho-EGFR antibodies. Blots were stripped and re-probed for GST to verify equal loading.
   **Part g** depicts the results of in vitro kinase assays by incubating the recombinant TTK (as kinase) and catalytically active recombinant GST-tagged EGFR (active-rhEGFR as substrates; Upstate). After the kinase reaction, samples were subjected to western-blot analysis with anti-phospho-tyrosine antibodies. The kinase activity of active-rhEGFR was detected in the presence of ATP (lane 2, # indicates the autophosphorylation of active-rhEGFR). The phosphorylation of active-rhEGFR pre-treated with EGFR tyrosine kinase inhibitor (AG1378) was not detected in the absence of recombinant TTK (lane 3), while it was detected in the presence of recombinant TTK (lane 4). Autophosphorylated form of recombinant TTK (arrow) and phosphorylated form of active-rhEGFR by recombinant TTK were indicated.
   **Part h** depicts the results of immunohistochemical staining of representative surgically-resected samples including NSCLC (lung-ADC and -SCC) and SCLC as well as normal lung, using anti-phospho-EGFR (Tyr-992) antibody on tissue microarrays (x200).
   **Part i** depicts the results of Kaplan-Meier analysis of tumor-specific survival in patients with NSCLC according to phospho-EGFR (Tyr-992) expression (P < 0.0001 by the Log-rank test).
   **Part j** depicts the results of association of co-activation of TTK and phospho-EGFR (Tyr-992) with poor prognosis of NSCLC patients. The 366 NSCLC cases were divided into three groups; group-1 for cases with strong-positive staining for both TTK and phospho-EGFR (Tyr-992) (63 patients), group-2 for cases with negative staining for both markers (74 patients), group-3 for any other cases (229 patients, shown as others).
   **Part k** and **l** depict the results of immunofluorescence analysis of phospho-EGFR (Tyr-992) in COS-7 cells that were transiently over-expressed TTK. COS-7 cells that scarcely expressed endogenous TTK, were transfected with TTK-expressing vector or with empty-vector (mock), and were maintained in serum-free medium for 12 hours, and subsequently washed and fixed; The TTK-Alexa488, phospho-EGFR (Tyr-992)-Alexa594, or cell nuclei (DAPI) were visualized in green, red, or blue, respectively. Internalization of phospho-EGFR (Tyr-992) was observed in cells transfected with TTK-expressing plasmids (k). Arrows indicate localization of phospho-EGFR (Tyr-992) (I).
   **Part m** depicts the levels of phospho-EGFR (Tyr-992) detected by immunofluorescence analysis in A549 cells transfected with the RNAi (oligo) against TTK (si-TTK). RNAi mediated suppression of TTK reduced the phosphorylation of EGFR at Tyr-992.

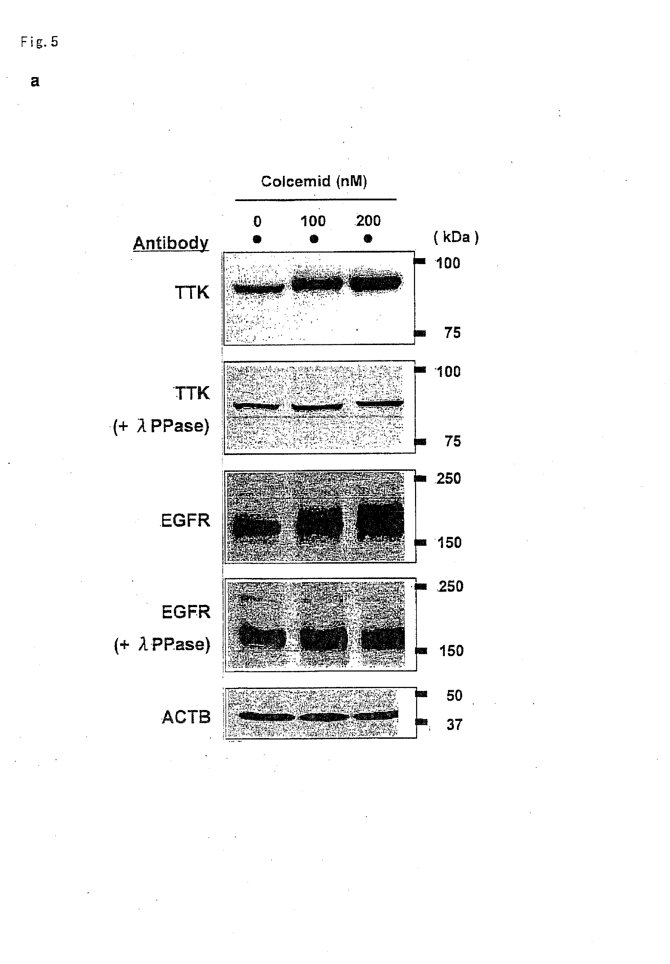

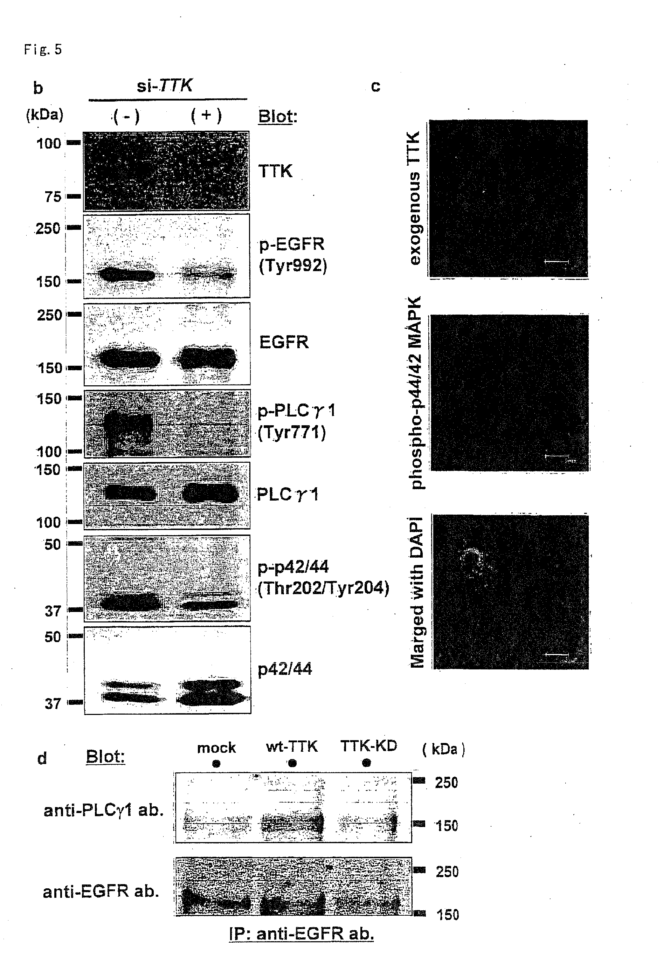

**Figure 5** **depicts the results of induction of phospho-EGFR (Tyr-992) and activation of downstream signals in a TTK-dependent oncogenic pathway.**
   **Part a** depicts the expression levels of TTK and the phosphorylation levels of EGFR (Tyr-992) in A549 cells that had been arrested at mitosis with colcemid treatment (0, 100, 200 nM) (WAKO) for 24 hours, was detected by western-blot analysis using anti-TTK or pEGFR (Tyr-992) antibody (**top** and **third panels**). To assess the mobility-shift of TTK or EGFR band by phosphorylation, the cell lysate was treated or untreated with Lambda Protein Phosphatase (λ-PPase; New England Biolabs) in phosphatase buffer or buffer alone for 1 hour at 37°C. The treatment abolished the mobility-shift of TTK and EGFR bands detected by western-blotting (**second** and **forth panels**).
   **Part b** depicts the expression levels of TTK, phospho-EGFR (Tyr-992), total EGFR, phospho-PLCγ1 (Tyr-771), total PLCγ1, phospho-p44/42 MAPK (Thr202/Tyr204), and total p44/42 MAPK, detected by western-blot analysis in A549 cells transfected with the RNAi (oligo) against TTK. RNAi mediated suppression of TTK reduced the phosphorylation of both EGFR (Tyr-992), PLCγ1 (Tyr-771) and p44/42 MAPK (Thr202/Tyr204).
   **Part c** depicts results of immunofluorescence analysis of phospho-p44/42 MAPK in COS-7 cells transiently over-expressing TTK. Transfected cells were maintained in serum-free medium for 12 hours, and subsequently washed and fixed; The Flag-TTK-Alexa488, phospho-p44/42 MAPK (Thr202/Tyr204)-Alexa594, or cell nuclei (DAPI) were visualized in green (**upper panel**), red (**middle panel**), or blue, respectively. Phosphorylation of p44/42 MAPK was observed only in TTK-transfected cells, but it was not detected in TTK-non-transfected cells (**lower panel**).
   **Part d** depicts the results of co-immunoprecipitaion of PLCγ1 with EGFR in COS-7 cells that were transfected with the TTK-, TTK-KD- or empty-vector (mock). The EGFR was immunoprecipitated from whole cell extracts of these cells by using anti-EGFR antibody. Immunoprecipitates were analyzed by western-blotting.

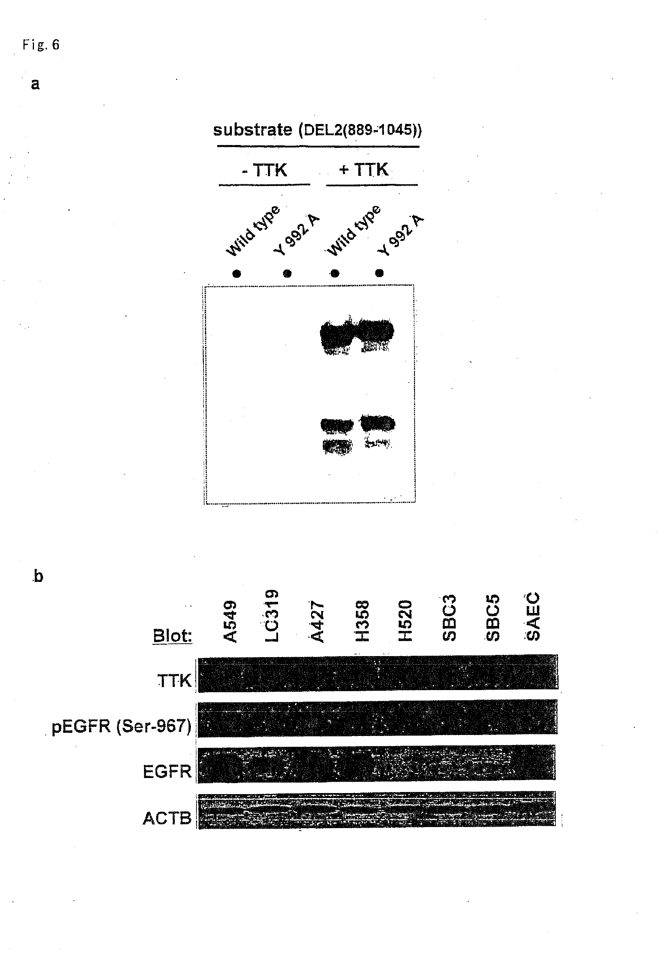

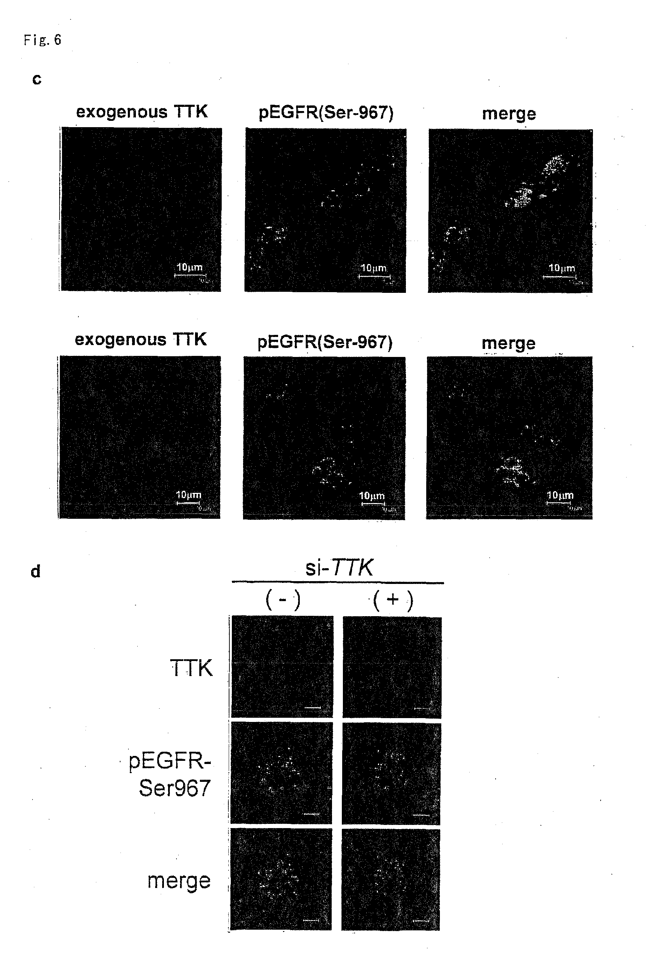

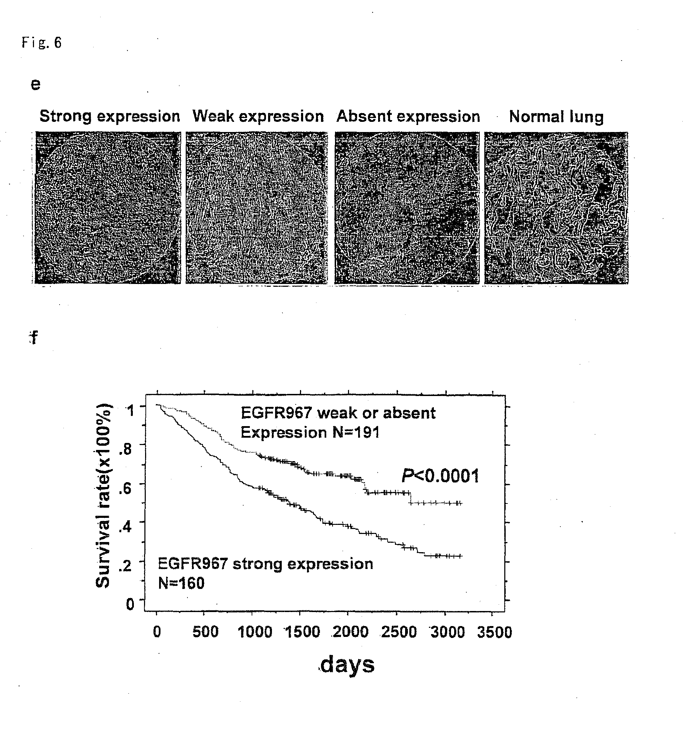

**Figure 6** **depicts the results of direct phosphorylation, of EGFR on Ser-967 by TTK protein kinase.**
   **Part a** depicts the results of in vitro kinase assay performed by incubating recombinant EGFR-DEL-2 (wild type) or EGFR-DEL-2 mutant whose Tyr-992 residue was replaced with an alanine (Y992A), with recombinant TTK in the presence of [γ-³²P] ATP. The products were separated on SDS-PAGE and phosphorylation was visualized by autoradiography.
   **Part b** depicts the levels ofTTK, phospho-EGFR (Ser-967), and total EGFR proteins in lung-cancer cell lines, detected by western-blot analysis.
   **Part c** depicts the results of phospho-EGFR (Ser-967) in COS-7 cells that transiently over-expressed TTK, detected by immunofluorescence analysis. COS-7 cells that scarcely expressed endogenous TTK were transfected with the TTK-expression vector. The TTK-Alexa594, phospho-EGFR (Ser-967)-Alexa488, or cell nuclei (DAPI) were visualized in red (**right panels**), green (**middle panels**), or blue (**left panels**), respectively. TTK over-expression induced the phosphorylation of ECFR (Ser-967).
   **Part d** depicts the phosphorylation level of EGFR (Ser-967) detected by immunofluorescence analysis of A549 cells transfected with the RNAi (oligo) against TTK. RNAi mediated suppression of TTK reduced the phosphorylation of EGFR (Ser-967).
   **Part e** depicts the results of immunohistochemical evaluation of phospho-EGFR (Ser-967) expression on tissue microarrays. Examples are shown for strong, weak, or absent phospho-EGFR (Ser-967) expression. Magnification, x100.
   **Part f** depicts the results of Kaplan-Meier analysis of tumor-specific survival in patients with NSCLC according to phospho-EGFR (Ser-967) expression (P < 0.0001 by the Log-rank test).

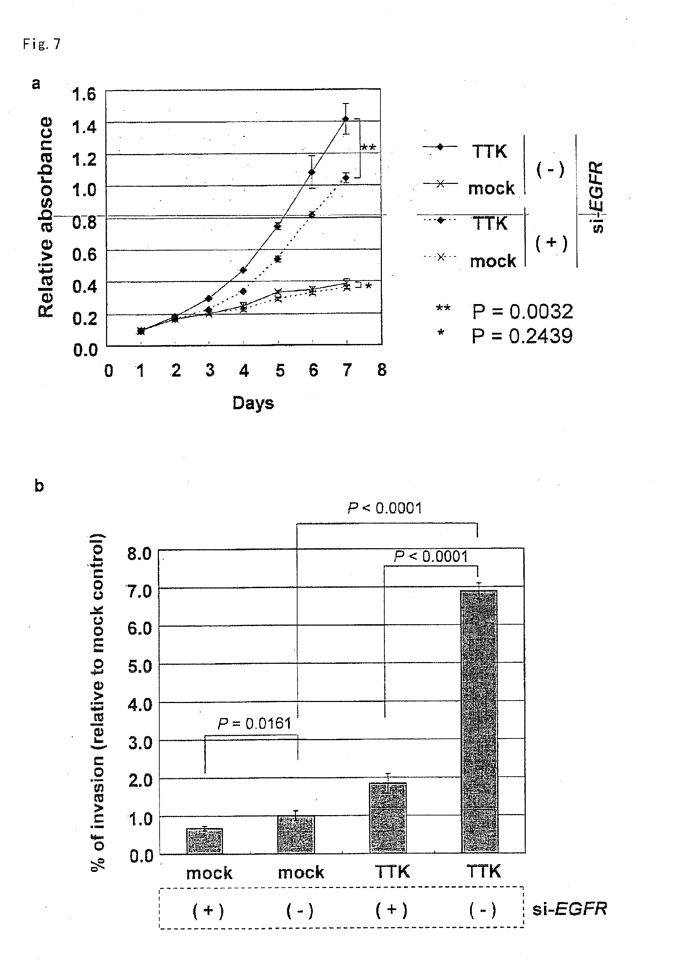

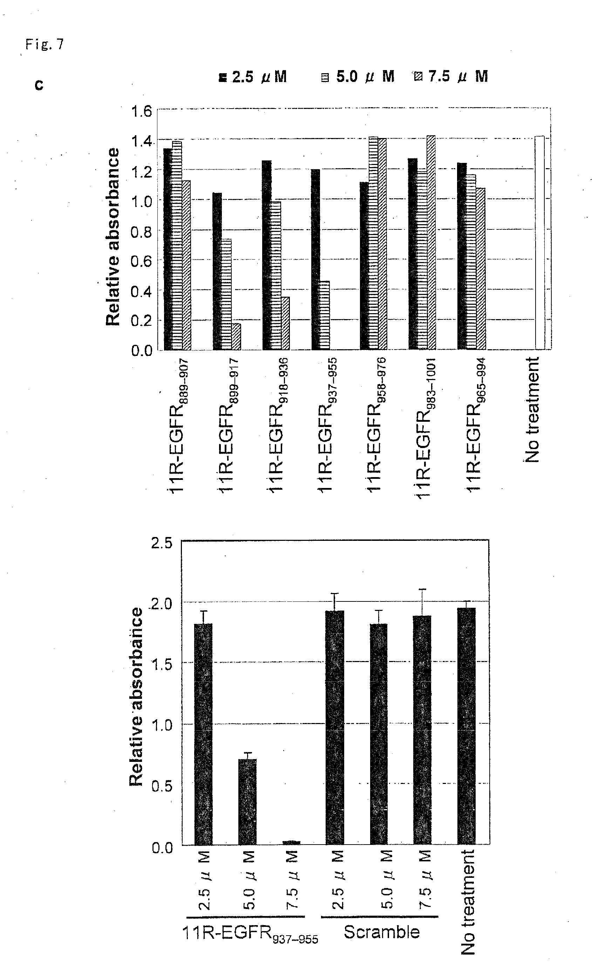

**Figure 7** **depicts the results of inhibition of cell growth/invasion by targeting TTK-EGFR pathway.**
   **Part a** depicts the results of MTT assay demonstrating the increased growth promoting effect of TTK-stable transfectants of HEK293. TTK- or mock-stable transfectants of HEK293 cells were each cultured in triplicate; at each time point, the cell viability was evaluated by MTT assay. These stable transfectants were transfected with the RNAi (oligo) against EGFR (si-EGFR).
   **Part b** depicts the results of Matrigel invasion assay demonstrating the increased invasive ability of TTK- or mock-stable transfectants of HEK293. These stable transfectants were transfected with si-EGFR.
   **Part c** depicts the results of inhibition of growth of lung cancer cells by cell-permeable EGFR peptides (11R-EGFR) detected by MTT assay. Peptides that were introduced into TTK-over-expressing A549 cells. Growth-suppressive effect of 11R-EGFR899-917, 11R-EGFR918-936, or 11R-EGFR937-955 that was derived from TTK-binding region in EGFR (**left panel**). The treatment with scramble peptides derived from the most effective 11R-EGFR937-955 peptides resulted in no growth-suppressive effect in cell viability as measured by MTT assay (**right panel**). Columns, relative absorbance of triplicate assays; bars, SD.

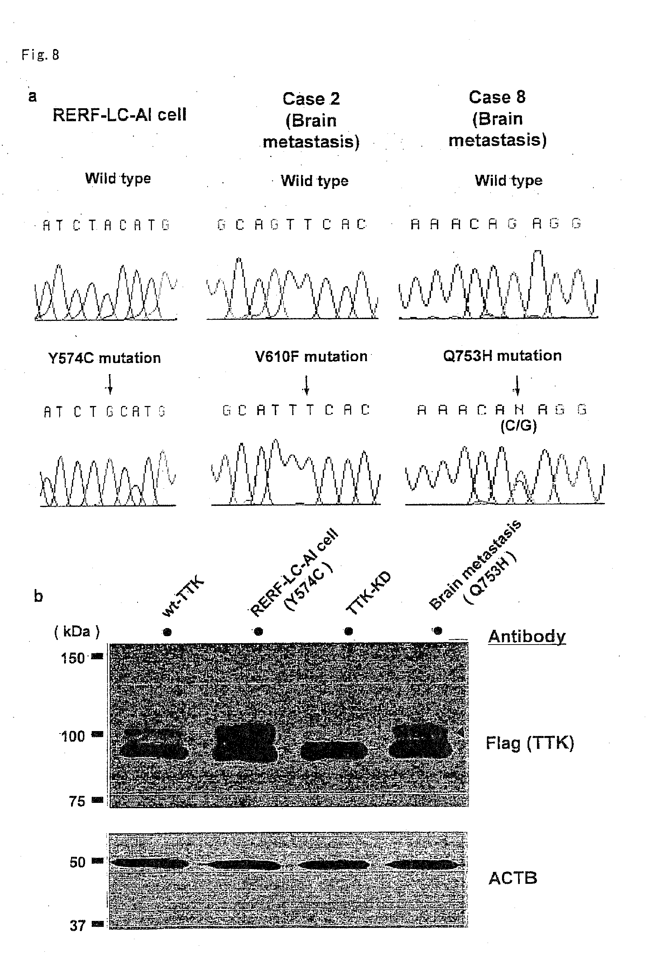

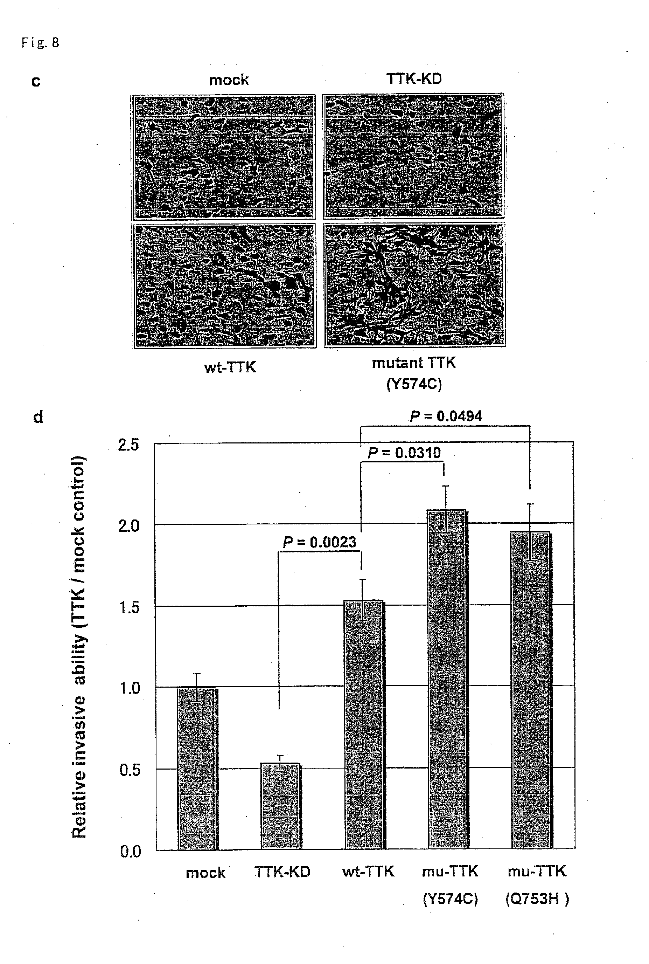

**Figure 8** **relates to the activating mutation of TTK in human lung cancer.**
   **Part a** depicts the results of Sequence analyses of TTK mutations in lung cancer. **Left panels, a** point mutation (Y574C; arrow) resulting in amino acid substitution within the tyrosine kinase domain of TTK found in a lung cancer cell line, RERF-LC-AI. Representative wild type sequence is shown as a reference. **Middle** and **right panels,** Point mutations (arrows) resulting in amino acid substitution within the tyrosine kinase domain of TTK in two metastatic brain tumors derived from primary lung adenocarcinoma (V610F in Case 2 and Q753H in Case 8). The corresponding part of wild type DNA sequence from paired normal brain tissues is also shown.
   **Part b** depicts the results of western-blot analysis using anti-Flag antibody detecting exogenously expressed mutant TTKs (Y574C) and (Q753H) in NIH-3T3 cells. Phospho-TTK was indicated by arrowhead.
   **Part c and d** depict the results of Matrigel invasion assay demonstrating the increased invasive ability of NIH-3T3 cells transfected wt-TTK-, TTK-KD-, mutant TTK (Y574C)-, or mutant TTK (Q753H)-, or mock-vector. Invading cells through Matrigel-coated filters evaluated by Giemsa staining (x200) (**c**) and the cell numbers counted (**d**).

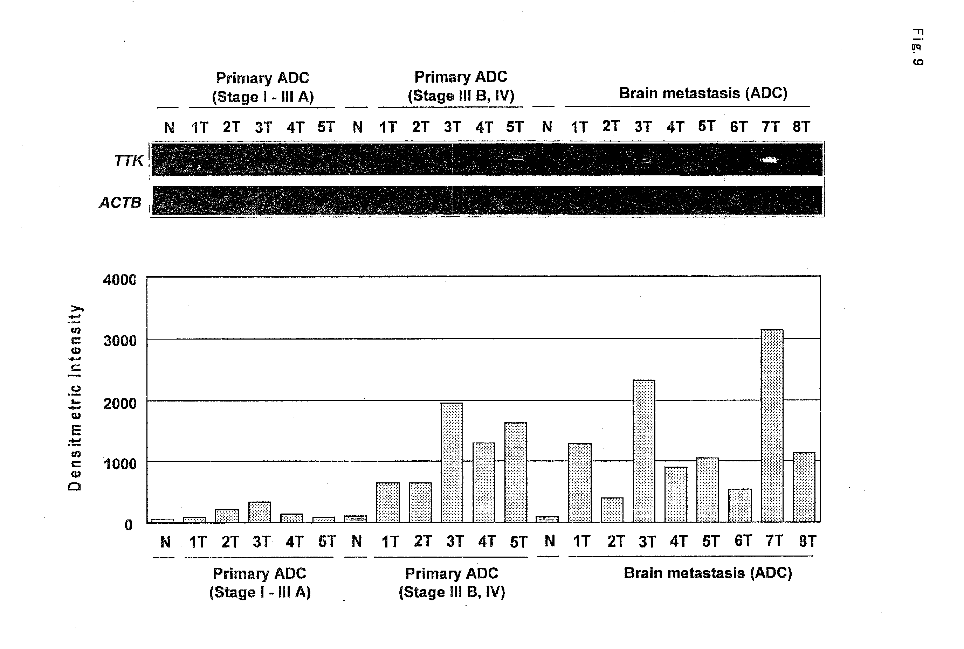

**Figure 9** **depicts the expression of TTK in clinical samples.**
   Expression of TTK in clinical samples of early primary NSCLC (stage I - III A), advanced primary NSCLC (stage III B - IV), and metastatic brain tumor from ADC (T) and normal lung tissues (N), examined by semiquantitative RT-PCR. (**upper panel**). Densitmetric intensity of PCR product was quantified by image analysis software (**lower panel**).

### DETAILED DESCRIPTION OF THE INVENTION

It is to be understood that the present invention is not limited to the specific methodologies and protocols herein described, as these may vary in accordance with routine experimentation and optimization. It is also to be understood that the terminology used in the description is for the purpose of describing the particular versions or embodiments only, and is not intended to limit the scope of the present invention which will be limited only by the appended claims. It must be noted that as used herein and in the appended claims, the singular forms "a", "an", and "the" include plural reference unless the context clearly dictates otherwise. Thus, for example, reference to a "cell" is a reference to one or more cells and equivalents thereof known to those skilled in the art, and so forth.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the present invention, the following definitions apply:

In the context of the present invention, the TTK polypeptide may be a polypeptide having the amino acid sequence of SEQ ID NO: 2 or a polypeptide having the amino acid sequence of SEQ ID NO: 2 in which one or more amino acids are modified by substitution, deletion or insertion, provided the resulting polypeptide is functionally equivalent to the polypeptide of SEQ ID NO: 2. Additionally, the TTK polypeptide may take the form of an 857-amino acid protein encoded by the open reading frame of SEQ. ID. NO. 1, or a polynucleotide that hybridizes under stringent conditions, e.g., low or high, to the nucleotide sequence of SEQ ID NO: 1, provided the resulting polynucleotide encodes a protein that is functionally equivalent to the polypeptide of SEQ ID NO: 2.

In the context of the present invention, the term "functionally equivalent" means that the subject protein retains a biological activity of the original protein. Biological activities of the polypeptide of SEQ ID NO: 2 include, for example, the promotion of cell proliferation and the kinase activity of TTK for EGFR. In the context of the present invention, a protein that is functionally equivalent to TTK preferably has kinase activity for EGFR. Whether or not a subject protein has the target activity can be determined in accordance with the present invention. For example, kinase activity for EGFR can be determined by incubating a polypeptide under conditions suitable for phosphorylation of EGFR and detecting the phosphor-EGFR level. For example, phosphorylation site of EGFR by TTK is Tyr992 or Ser967.

Proteins that are functionally equivalent to the human TTK protein, encoded by the DNA isolated through the above hybridization techniques or gene amplification techniques, normally have a high homology to the amino acid sequence of the human TTK protein. In the context of the present invention, the term "high homology" refers to a homology of 40% or higher, preferably 60% or higher, more preferably 80% or higher, even more preferably 95% or higher. The homology of a protein can be determined by following the algorithm in "Wilbur, W. J. and Lipman, D. J. (1983) Proc. Natl. Acad. Sci. USA 80, 726-30".

In the context of the present invention, the term "stringency" in the context of hybridization refers to the relative rigor of hybridization standards utilized. Examples of suitable low stringency conditions include, for example, 42°C, 2x SSC, 0.1% SDS, or preferably 50°C, 2x SSC, 0.1% SDS. Preferably, a high stringency condition is used. An example of a suitable high stringency condition includes, for example, washing 3 times in 2x SSC, 0.01% SDS at room temperature for 20 min, then washing 3 times in 1x SSC, 0.1% SDS at 37°C for 20 min, and washing twice in 1x SSC, 0.1% SDS at 50°C for 20 min. However, several factors, such as temperature and salt concentration, can influence the stringency of hybridization and one skilled in the art can suitably select the factors to achieve the requisite stringency.

In the context of the present invention, the phrase "control level" refers to an mRNA or protein expression level detected in a control sample and may include any of (a) a normal control level or (b) a lung cancer specific control level. A control level can be a single expression pattern from a single reference population or composed from a plurality of expression patterns. For example, in the context of the present invention, the control level can be a database of expression patterns from previously tested cells. The phrase "normal control level" refers to a level of gene expression detected in a normal, healthy individual or in a population of individuals known not to be suffering from cancer, such as lung cancer. A normal individual is one with no clinical symptoms of cancer, particularly lung cancer. On the other hand, a "lung cancer control level" refers to a level of gene expression found in a population suffering from lung cancer.

In the context of the present invention, an expression level of a particular gene is deemed "increased" when the expression of the gene or the activity of its gene product is increased by at least 0.1, at least 0.2, at least 1, at least 2, at least 5, or at least 10 or more fold as compared to a control level. TTK gene expression can be determined by detecting mRNA of TTK from a tissue sample from a patient, e.g., by RT-PCR or Northern blot analysis, or detecting a protein encoded by TTK, *e.g*., by immunohistochemical analysis of a tissue sample from a patient.

In the context of the present invention, the specimen obtained from a subject may be any biological sample for example, a solid tissue or bodily fluid sample, obtained from a test subject, *e.g*., a patient known to or suspected of having cancer, more particularly lung cancer. For example, in the context of tissue specimen, the tissue can contain epithelial cells. More particularly, the tissue can be epithelial cells from lung cancer cells, e.g. non-small cell lung cancer or small cell lung cancer. Alternatively, the specimen may be a bodily fluid, such a blood, serum, or plasma.
The present invention relates to cancer therapy and prevention. In the context of the present invention, therapy against cancer or prevention of the onset of cancer includes any of the following steps, including inhibition of the growth of cancerous cells, involution of cancer, and suppression of the occurrence of cancer. A decrease in mortality and morbidity of individuals having cancer, decrease in the levels of tumor markers in the blood, alleviation of detectable symptoms accompanying cancer, and such are also included in the therapy or prevention of cancer. Such therapeutic and preventive effects are preferably statistically significant. For example, in observation, at a significance level of 5% or less, wherein the therapeutic or preventive effect of a pharmaceutical composition against cell proliferative diseases is compared to a control without administration. For example, Student's t-test, the Mann-Whitney U-test, or ANOVA can be used for statistical analysis.

Furthermore, in the context of the present invention, the term "prevention" encompasses any activity which reduces the burden of mortality or morbidity from disease. Prevention can occur at primary, secondary and tertiary prevention levels. While primary prevention avoids the development of a disease, secondary and tertiary levels of prevention encompass activities aimed at preventing the progression of a disease and the emergence of symptoms as well as reducing the negative impact of an already established disease by restoring function and reducing disease-related complications.

In the context of the present invention, an "efficacious" treatment is one that leads to a reduction in the level of TTK or the phosphorylation levels of EGFR or a decrease in size, prevalence, or metastatic potential of lung cancer in a subject. When a treatment is applied prophylactically, "efficacious" means that the treatment retards or prevents occurrence of lung cancer or alleviates a clinical symptom of lung cancer. The assessment of lung cancer can be made using standard clinical protocols. Furthermore, the efficaciousness of a treatment can be determined in association with any known method for diagnosing or treating lung cancer. For example, lung cancer is routinely diagnosed histopathologically or by identifying symptomatic anomalies.

Additional definitions are interspersed in the subsequent text, where applicable.

### Overview:

Although advances have been made in development of molecular-targeting drugs for cancer therapy, the ranges of tumor types that respond as well as the effectiveness of the treatments remain very limited (Ranson, M., et al. (2002) J Clin Oncol, 20: 2240-50.; Blackledge, G. and Averbuch, S. (2004) Br J Cancer, 90: 566-72.). Hence, there is an urgent need to develop new anti-cancer agents that are highly specific to malignant cells with minimal or no adverse reactions. A powerful strategy toward these ends would combine the screening of up-regulated genes in cancer cells, identified on the basis of genetic information obtained on cDNA microarrays with high-throughput screening of their effect on cell growth, by inducing loss-of-function phenotypes with RNAi systems, with validation of the potential drug targets by analyzing hundreds of clinical samples on tissue microarray (Sauter, G., et al. (2003) Nat Rev Drug Discov, 2: 962-72.; Kononen, J., et al. (1998) Nat Med, 4: 844-7.). Following such a strategy, it is herein demonstrated that TTK is not only frequently co-over-expressed in clinical NSCLC samples and cell lines, but also that the high levels of expression of the gene products are indispensable for the disease progression as well as the growth of NSCLC cells.

Epidermal growth factor receptor (EGFR) has been recognized as an important mediator of various growth signaling pathways (Carpenter G. Annu Rev Biochem. 1987;56:881-914.; Wells C. Int J Biochem Cell Biol. 1999 Jun;31(6):637-43.). Aberrant EGFR activity arising from genetic and epigenetic changes, has been shown to enhance cell proliferation and drive tumor progression in many tumors (Salomon DS, et al., Crit Rev Oncol Hematol. 1995 Jul;19(3):183-232.; Mendelson J. Clin Cancer Res. 2000 Mar;6(3):747-53.). Therefore, agents that selectively block EGFR signaling have been under development; examples currently in clinical use include anti-EGFR monoclonal antibody, cetuximab (Erbitux), and small-molecule inhibitors of EGFR tyrosine kinase such as gefitinib (Iressa) and erlotinib (Tarceva) (Dowell J, et al., Nat Rev Drug Discov. 2005 Jan;4(1):13-4.; Herbst RS, et al., Nat Rev Cancer. 2004 Dec;4(12):956-65.). Stimulation of its ligands, such as EGF; causes EGFR to undergo a conformational change and autophosphorylation that activates the EGFR signaling pathways includes the MAPK (mitogen activated protein kinase) cascade and c-Src (cellular Src) cascade (Yarden Y. Eur J Cancer. 2001 Sep;37 Suppl 4:S3-8.; Pal SK & Pegram M. Anticancer Drugs. 2005 Jun;16(5):483-94.; Tice DA, et al., Proc Natl Acad Sci U S A. 1999 Feb 16;96(4):1415-20.). c-Src phosphorylates the cytoplasmic tail of EGFR in the presence of EGF and activates the EGFR signals (Yarden Y. Euro J Cancer. 2001 Sep;37 Suppl 4:S3-8.; Pal SK & Pegram M. Anticancer Drugs. 2005 Jun;16(5):483-94.; Tice DA, et al., Proc Natl Acad Sci U S A. 1999 Feb 16;96(4):1415-20.). However, to date, no kinase that phosphorylates EGFR and consequently activates the EGFR pathways in an EGF-independent manner has been reported.

However, evidence is provided herein that TTK plays a significant role in pulmonary carcinogenesis through EGF-independent phosphorylation of EGFR Tyr-992 or Ser-967 and subsequent activation of downstream MAPK signals that are considered to be indispensable for tumor growth/survival. Thus, the data suggest that a novel signaling between TTK and EGFR, independent of the presence of EGF, represents a potential target for development of novel therapeutic drugs for lung cancer.

### Assessing a prognosis of lung cancer:

As noted above, the present invention is based, in part, on the discovery of a novel intracellular target molecule of a TTK kinase, EGFR. The present invention is also based on the finding that a high expression level of TTK and/or a high level of phospho-EGFR is associated with a poor prognosis in lung cancer patients. Especially, the lung cancer is non-small cell lung cancer (NSCLC). In view of the evidence provided herein, that TTK expression and/or a kinase activity of TTK for EGFR or the phosphorylation level of EGFR is associated with poor prognosis of cancer patients, the present invention thus provides methods for assessing or determining a prognosis for lung cancer patients. For example, the pohsphorylation site of EGFR is Tyr-992 or Ser-967. An example of such a method includes the steps of:
a. detecting a TTK expression level or a phosphorylation level of EGFR in a specimen collected from a subject whose lung cancer prognosis is to be assessed or determined, and
b. indicating a poor prognosis when an elevated level of TTK expression or phosphor-EGFR is detected.

In the context of the present method, the specimen is collected from a subject. An example of a preferred specimen for use in the context of the present invention is a lung tissue obtained by biopsy or surgical-resection from lung cancer patients. In the context of the present invention, when the TTK expression level or a phosphorylation level of EGFR detected in a test specimen is higher than a control level, then the test specimen is deemed to have an elevated level of TTK expression or a phosphorylation level of EGFR. An example of a useful control level in the context of the present invention may include a standard value of TTK expression or a Phosphorylation level of EGFR level taken from a group associated with good prognosis. The standard value may be obtained by any method known in the art. For example, a range of mean ± 2 S.D. or mean ± 3 S.D. may be used as the standard value. Alternatively, poor prognosis can be determined, when strong staining is observed by immunohistochemical analysis of sample tissue.

In the context of the present invention, an expression level of TTK may be detected by any one of the method selected from the group consisting of:
(a) detecting the presence of an mRNA encoding the Amino acid sequence of SEQ ID NO: 2,
(b) detecting the presence of a protein having the amino acid sequence of SEQ ID NO: 2, and
(c) detecting the biological activity of a protein having the amino acid sequence of SEQ ID NO: 2.

In the context of the present invention, the mRNA, the protein, or biological activity of the protein may be detected by any method. Methods for detecting a given protein, mRNA or biological activity thereof are well known to those skilled in the art. For example, mRNA may be detected using known PCR or hybridization based technologies. Alternatively, any immunoassay format may be applied for detection of a protein. Furthermore, the biological activity of TTK, *e.g*. a kinase activity of TTK for EGFR, may also detected using any suitable assay method, such as those described herein. For example, the kinase activity of TTK for EGFR may be detected at tyrosine of 992 amino acid residue or serine of 967 amino acid residue in SEQ ID NO: 42.

In the context of the present invention, a phosphorylation level of EGFR may be detected by measuring the amount of phosphorylated EGFR, *e.g*. Tyr-992 or Ser-967 phosphorylated EGFR. The method of detecting the phosphorylated EGFR is well known to those skilled in the art. For example, immunoassay by using a specific antibody may be useful.

In the context of the present invention, determination of a poor prognosis may be used to determine further treatment, *e.g*., to stop further treatments that reduce quality of life, to treat the cancer in a different manner than previously used, or to treat the cancer more aggressively. In other words, the assessment of a prognosis by TTK or phosphorylation of EGFR enables clinicians to choose, in advance, the most appropriate treatment for an individual lung cancer patient without even the information of conventional clinical staging of the disease, using only routine procedures for tissue-sampling.

Further, the methods of the present invention may be used to assess the efficacy of a course of treatment. For example, in a mammal with cancer from which a biological sample is found to contain an elevated level of TTK expression or phosphorylation of EGFR, the efficacy of an anti-cancer treatment can be assessed by monitoring the TTK expression level or the phosphorylation level of EGFR over time. For example, a decrease in TTK expression level or the phosphorylation level of EGFR in a biological sample taken from a mammal following a course of treatment, as compared to a level observed in a sample taken from the mammal before treatment onset, or earlier in, the treatment, may be indicative of efficacious treatment.

Alternatively, according to the present invention, an intermediate result may also be provided in addition to other test results for assessing or determining the prognosis of a subject Such intermediate result may assist a doctor, nurse, or other practitioner to assess, determine, or estimate the prognosis of a subject. Additional information that may be considered, in combination with the intermediate result obtained by the present invention, to assess prognosis includes clinical symptoms and physical conditions of a subject.

As noted above, the present invention also provides kits for assessing or determining lung cancer prognosis, including any one component selected from the group consisting of:
(a) a reagent for detecting the presence of an mRNA encoding the amino acid sequence of SEQ ID NO: 2,
(b) a reagent for detecting the presence of a protein having the amino acid sequence of SEQ NO: 2 or tyrosine of 992 amino acid residue or serine of 967 amino acid residue in SEQ ID NO: 42, and
(c) a reagent for detecting a biological activity of the protein having the amino acid sequence of SEQ ID NO: 2.

TTK has a kinase activity for EGFR, and its expression level and phospho-EGFR level (Tyr-992 or Ser-957) revealed a shorter tumor-specific survival period. Furthermore, the phosphorylation of EGFR at Tyr-992 or Ser-967 by TTK is independent from the EGF stimulation. Thus, TTK-mediated phosphorylation of EGFR is useful as a diagnostic parameter of lung cancer, e.g. non-small cell lung cancer.

The present invention also provides kits for assessing or determining lung cancer or a predisposition for developing lung cancer in a subject, wherein the kit includes a reagent for detecting the kinase activity of TTK for EGFR. The kit is also useful as a diagnostic of lung cancer, e.g. non-small cell lung cancer. Furthermore, the kinase activity of TTK for EGFR, *e.g.* an EGF-independent phosphorylation of EGFR by TTK, may also detected using any suitable reagent.

### Kinase activity of TTK for EGFR:

The selective phosphorylation of EGFR by TTK is revealed herein. Consequently, in another aspect, the present invention provides a method of measuring a kinase activity of TTK for EGFR. Such a method may include the steps of:
a. incubating EGFR or functional equivalent thereof and TTK under conditions suitable for the EGFR phosphorylation by TTK, wherein the TTK is selected from the group consisting of:
   i. a polypeptide having the ammo acid sequence of SEQ ID NO: 2 (TTK);
   ii. a polypeptide having the amino acid sequence of SEQ ID NO: 2 wherein one or more amino acids are modified by substitution, deletion or insertion, provided the resulting polypeptide has a biological activity equivalent to the polypeptide having the amino acid sequence of SEQ ID NO: 2;
   iii. a polypeptide encoded by a polynucleotide that hybridizes under stringent conditions to a polynucleotide having the nucleotide sequence of SEQ ID NO: 1, provided the resulting polypeptide has a biological activity equivalent to the polypeptide having the amino acid sequence of SEQ ID NO: 2;
b. detecting a phospho-EGFR level; and
c. measuring the kinase activity of TTK by correlating the phosphor-EGFR level detected in step (b).

In the context of present invention, the conditions suitable for the EGFR phosphorylation may be provided with an incubation of EGFR and TTK in the presence of phosphate donor. In the present invention, preferable phosphate donor is ATP. The conditions suitable for the EGFR phosphorylation by TTK also include culturing cells expressing the polypeptides. For example, the cell may be a transformant cell harboring an expression vector that contains a polynucleotide encoding the polypeptide. In another embodiment, the phosphorylation reaction against EGFR is performed by incubation of EGFR and TTK in kinase assay buffer (for example, 50 mM Tris, pH7.4, 10 mM MgCl₂, 2 mM dithiothreitol, 1 mM NaF, 0.2 mM ATP) for 60 min at 30°C. In the context of present invention, functional equivalent of EFGR is fragment of EGFR which may be comprised TTK-mediated phosphorylation site of EGFR, Tyr992 or Ser967. For example, the fragment of EGFR may be comprised amono acid sequence of SEQ ID NO: 43.

After the incubation, a phospho-EGFR level can be detected with an antibody recognizing phosphorylated EGFR. Prior to the detection of phosphorylated EGFR, EGFR may be separated from other elements, or cell lysate of EGFR expressing cells. For instance, gel electrophoresis may be used for separation of EGFR. Alternatively, EGFR may be captured by contacting EGFR with a carrier having an anti-EGFR antibody. When the labeled phosphate donor was used, phospho-EGFR level can be detected via tracing the label. For example, radio-labeled ATP (*e.g*. ³²P-ATP) was used as phosphate donor, radio activity of the separated EGFR correlates with phospho-EGFR level.

In the context of present invention, kinase activity of TTK in biological samples may be estimated. For example, the biological sample of the present invention may include cancer tissues obtained from a patient or cancer cell lines. The kinase activity of TTK in such biological samples is useful as credible marker for indicating lung cancer, or assessing or determining prognosis. The present invention further provides a reagent for measuring a kinase activity of TTK for EGFR. Examples of such reagents include EGFR and phosphate donor. In the present invention, the kit for measuring a kinase activity of TTK for EGFR is also provided. Such a kit may include the reagent of the present invention and detecting agent for detecting phospho-EGFR level. Preferable detecting agent is an antibody specifically recognizing phosphorylated EGFR from unphosphorylated EGFR. For example, in the present invention, preferable antibody recognizes phosphorylated EGFR at Tyr992 or Ser967.

### Diagnosing Method:

The present invention also provides a method of diagnosing lung cancer or a predisposition for developing lung cancer in a subject, such a method including the step of determining a level of the TTK expression or the phosphorylation of EGFR in a biological sample derived from the subject, wherein an increase in said level, as compared to a normal control level, indicates that said subject suffers from or is at risk of developing lung cancer. In the present invention, any sample derived from a subject to be diagnosed may be used. An example of a preferred sample for use in the context of the present invention is a lung tissue obtained by biopsy or surgical-resection. For example, the phosphorylation site of EGFR by TTK is Tyr992 or Ser967.

Alternatively, according to the present invention, an intermediate result for examining the condition of a subject may be provided. Such intermediate result may be combined with additional information to assist a doctor, nurse, or other practitioner to determine that a subject suffers from lung cancer. Further, the present invention relates to a method for screening a person who is required to be further diagnosed for lung cancer. After the screening, persons indicating positive result are recommended to be submitted further screening test, or medical treatment to confirm whether they truly suffer from lung cancer.

Alternatively, the present invention may be used to detect cancerous cells in a subject-derived tissue, and provide a doctor with useful information to determine that the subject suffers from lung cancer. Accordingly, the present invention involves determining (*e.g*., measuring) a level of a kinase activity of TTK for EGFR in subject derived samples. In the present invention, a method for diagnosing lung cancer also includes a method for testing or detecting lung cancer. Alternatively, in the present invention, diagnosing lung cancer also refers to showing a suspicion, risk, or possibility of lung cancer in a subject.

The diagnostic method of the present invention involves the step of determining (*e.g*., measuring) the expression of TTK. Using sequence of TTK gene can be detected and measured using conventional techniques well known to one of ordinary skill in the art. For example, northern blot hybridization analyses can be used for determining the expression of TTK gene. Hybridization probes typically include at least 10, at least 20, at least 50, at least 100, or at least 200 consecutive nucleotides of TTK sequence. As another example, the sequences can be used to construct primers for specifically amplifying the TTK nucleic acid in, *e.g*., amplification-based detection methods, for example, reverse-transcription based polymerase chain reaction (RT-PCR). As another example, an antibody against TTK, *e.g*., an anti-TTK polyclonal antibody or anti-TTK monoclonal antibody, can be used for immunoassay, for example, immunohistochemical analysis, western blot analysis or ELISA, *etc.*

Alternatively, the expression of TTK can be detected by the biological activity. For example, the biological activity is cell proliferative activity or invasion activity or kinase activity against EGFR Tyr997 or Ser967. The method of detecting the kinase activity described in above.

Also, the diagnostic method of the invention involves the step of determining the pohsphorylation level of EGFR. For example, the pohsphorylation site of EGFR is Tyr992 or Ser967. The antibody that specifically recognizes the pohsphorylation without non-phosphorylation type can be used for immunoassay, for example, immunohistochemical analysis, western blot analysis or ELISA, *etc.*

The level of the TTK expression or the pohsphorylation level of EGFR detected in a test cell population, *e.g*., a tissue sample from a subject, can then be compared to that in a reference cell population. The reference cell population may include one or more cells for which the compared parameter is known, *i.e*., lung cancer cells or normal lung epithelial cells (non-lung cancer cells).

Whether or not the level of the TTK expression or the pohsphorylation level of EGFR in a test cell population as compared to a reference cell population indicates the presence of lung cancer or a predisposition thereto depends upon the composition of the reference cell population. For example, if the reference cell population is composed of non-lung cancer cell, a similarity in the level between the test cell population and the reference cell population indicates the test cell population is non-lung cancer. Conversely, if the reference cell population is made up of lung cancer cells, a similarity in gene expression between the test cell population and the reference cell population indicates that the test cell population includes lung cancer cells.

The level of the TTK expression or the pohsphorylation level of EGFR in a test cell population is considered "altered" or deemed to "differ" if it varies from the level in a reference cell population by more than 1.1, more than 1.5, more than 2.0, more than 5.0, more than 10.0 or more fold.

Differential gene expression between a test cell population and a reference cell population can be normalized to a control gene, *e.g*. a housekeeping gene. For example, a control gene is one which is known not to differ depending on the cancerous or non-cancerous state of the cell. The expression level of a control gene can thus be used to normalize signal levels in the test and reference cell populations. Exemplary control genes include, but are not limited to, *e.g*., beta-actin, glyceraldehyde 3- phosphate dehydrogenase and ribosomal protein P1.

The test cell population can be compared to multiple reference cell populations. Each of the multiple reference cell populations can differ in the known parameter. Thus, a test cell population can be compared to a first reference cell population known to contain, *e.g*., lung cancer cells, as well as a second reference cell population known to contain, *e.g*., non-lung cancer cells (normal cells). The test cell population can be included in a tissue or cell sample from a subj ect known to contain, or suspected of containing, lung cancer cells.

The test cell population can be obtained from a bodily tissue or a bodily fluid, *e.g*., biological fluid (for example, blood, sputum, saliva). For example, the test cell population can be purified from lung tissue. Preferably, the test cell population comprises an epithelial cell. The epithelial cell is preferably from a tissue known to be or suspected to be lung carcinoma.

Cells in the reference cell population are preferably from a tissue type similar to that of the test cell population. Optionally, the reference cell population is a cell line, *e.g*. a lung cancer cell line (*i.e.,* a positive control) or a normal non-lung cancer cell line (*i.e*., a negative control). Alternatively, the control cell population can be from a database of molecular information obtained from cells for which the assayed parameter or condition is known.

The subject is preferably a mammal. Exemplary mammals include, but are not limited to, *e.g*., a human, non-human primate, mouse, rat, dog, cat, horse, or cow.

The present invention also provides a kit for detection of kinase activity of TTK for EGFR. Examples of components contained within such kits include, EGFR, an antibody that binds to phospho-EGFR, *e.g*. anti-phospho-EGFR (Tyr992) antibody or anti-phospho-EGFR (Ser967) antibody, and a detectable label for detecting the antibody. An antibody recognizing phosphorylated Tyr992 or Ser967 of EGFR is commercially available. Alternatively, it is well known that such antibody can be obtained by immunization with phosphorylated EGFR at Tyr992 or Ser967, or fragment thereof that includes the Tyr992 or Ser967 residue.

The TTK cDNA consists of 2,984 nucleotides that contain an open reading frame of 2,571 nucleotides as set forth in SEQ. ID. NO.: 1 (GenBank Accession No. NM_003318). The open reading frame encodes an 857-amino acid protein having amino acid sequence as set forth in SEQ. ID. NO.: 2 (GenBank Accession No. NP_003309). Mps1 (TTK is its human homologue) was first discovered in budding yeast as a factor to be required in centrosome duplication and was subsequently shown to have a critical function in the spindle checkpoint.

### Screening Method:

The present invention also relates to the finding that TTK has the kinase activity for EGFR For example, the phosphorylation site of EGFR by TTK is Tyr992 or Ser967 and the phosphorylation is EGF-independent. To that end, one aspect of the invention involves identifying test compounds that regulate TTK-mediated phosphorylation of EGFR. Accordingly, the present invention provides novel methods for identifying compounds that modulates a kinase activity of TTK for EGFR. For instance, the present invention provides a method of identifying an agent that modulates a kinase activity of TTK for EGFR, such a method including the steps of:
a. incubating EGFR or functional equivalent thereof and TTK in the presence of a test compound under conditions suitable for the phosphorylation of EGFR by TTK, wherein the TTK is a polypeptide selected from the group consisting of:
   i. a polypeptide having the amino acid sequence of SEQ ID NO: 2 (TTK);
   ii. a polypeptide having the amino acid sequence of SEQ ID NO: 2 wherein one or more amino acids are modified by substitution, deletion or insertion, provided the resulting polypeptide has a biological activity equivalent to the polypeptide having the amino acid sequence of SEQ ID NO: 2;
   iii. a polypeptide encoded by a polynucleotide that hybridizes under stringent conditions to a polynucleotide having the nucleotide sequence of SEQ ID NO: 1, provided the resulting polypeptide has a biological activity equivalent to the polypeptide having the amino acid sequence of SEQ ID NO: 2;
b. detecting a phospho-EGFR level; and
c. comparing the phospho-EGFR level to a control level, wherein an increase or decrease in the phospho-EGFR level as compared to the control level indicates that the test compound modulates the kinase activity of TTK for EGFR.

Agents identified by the present method constitute candidate compounds that may slow or arrest the progression of, *e.g*., lung cancer, by inhibiting TTK-mediated phosphorylation of EGFR. Accordingly, the invention thus provides a method of screening for a compound that modulates TTK kinase activity for EGFR, *e.g*. EGF independent phosphorylation of EGFR by TTK. For example the phosphorylation site of EGFR is Tyr992 or Ser967. The method is practiced by contacting a TTK, or a functional equivalent thereof having kinase activity for EGFR, and EGFR or functional equivalent thereof capable of phosphorylation by TTK, with one or more candidate compounds, and assaying phospho-EGFR level. For example, the functional equivalent of EGFR that is capable of phosphorylation by TTK may be comprised TTK-mediated phosphorylation site of EGFR, Tyr992 or Ser967. More preferably, the fragment of EGFR may be comprised the region from 889aa to 1045aa of SEQ ID NO: 42. A compound that modulates phosphorylation of EGFR by TTK or functional equivalent is thereby identified. Consequently, the present invention also provides a method of screening for a compound for treating and/or preventing lung cancer, such a method including the steps of:
a. identifying a test compound that modulates kinase activity of TTK for EGFR by the method as mentioned above, and
b. selecting a compound that decreases the phospho-EGFR level as compared to a control level.

The other respect of the invention, a kit for detecting the ability of a test compound to modulate kinase activity of TTK for EGFR also provided. Such a kit may include the components of:
A) a polypeptide selected from the group consisting of:
   i. a polypeptide having the amino acid sequence of SEQ ID NO: 2 (TTK);
   ii. a polypeptide having the amino acid sequence of SEQ ID NO: 2 wherein one or more amino acids are modified by substitution, deletion or insertion, provided the resulting polypeptide has a biological activity equivalent to the polypeptide having the amino acid sequence of SEQ ID NO: 2;
   iii. a polypeptide encoded by a polynucleotide that hybridizes under stringent conditions to a polynucleotide having the nucleotide sequence of SEQ ID NO: 1, provided the resulting polypeptide has a biological activity equivalent to the polypeptide having the amino acid sequence of SEQ ID NO: 2; and
B) EGFR or EGFR or functional equivalent thereof.
C) a reagent for detecting a phospho-EGFR.

Further, this invention also provides a kit for detecting for the ability of a test compound to modulate kinase activity of TTK for EGFR. Such a kit may include the components of:
A) a cell expressing EGFR or functional equivalent thereof a polypeptide selected from the group consisting of:
   i. a polypeptide having the amino acid sequence of SEQ ID NO: 2 (TTK);
   ii. a polypeptide having the amino acid sequence of SEQ ID NO: 2 wherein one or more amino acids are modified by substitution, deletion or insertion, provided the resulting polypeptide has a biological activity equivalent to the polypeptide having the amino acid sequence of SEQ ID NO: 2;
   iii. a polypeptide encoded by a polynucleotide that hybridizes under stringent conditions to a polynucleotide having the nucleotide sequence of SEQ ID NO: 1, provided the resulting polypeptide has a biological activity equivalent to the polypeptide having the amino acid sequence of SEQ ID NO: 2; and
B) a reagent for detecting a phospho-EGFR.

In the present invention, the functional equivalent of EGFR is the fragment consisting of amino acid sequence of SEQ ID NO: 43. Furthermore, the kit may further include phosphate donor. Preferable phosphate donor is ATP. The reagent for detection in the kit of the present invention may also include an antibody recognizes phosphorylated Tyr992 or Ser967 of EGFR as the reagent for detecting a phospho-EGFR.

The present invention further provides a composition for treating or preventing lung cancer, such a composition composed of a pharmaceutically effective amount of a compound that decreases a kinase activity of TTK for EGFR and a pharmaceutically acceptable carrier. As noted above, in the context of the present invention, the term "functionally equivalent." means that the subject protein retains the biological activity of the original protein, in this case the kinase activity for EGFR. Whether or not a subject protein has the target activity can be determined in accordance with the present invention. For example, kinase activity for EGFR can be determined by incubating a polypeptide under conditions suitable for phosphorylation of EGFR and detecting the phosphor-EGFR level. For example, the phosphorylation site of EGFR by TTK is Tyr992 or Ser967.

Methods for preparing proteins functionally equivalent to a given protein are well known to those skilled in the art and include conventional methods of introducing mutations into the protein. For example, one skilled in the art can prepare proteins functionally equivalent to the human TTK protein by introducing an appropriate mutation in the amino acid sequence of the human TTK protein via site-directed mutagenesis (Hashimoto-Gotoh, T. et al. (1995), Gene 152, 271-5; Zoller, MJ, and Smith, M. (1983), Methods Enzymol. 100, 468-500; Kramer, W. et al. (1984), Nucleic Acids Res. 12, 9441-56; Kramer W, and Fritz HJ. (1987) Methods. Enzymol. 154, 350-67; Kunkel, TA (1385), Proc. Natl. Acad. Sci. USA. 82, 488-92; Kunkel (1991), Methods Enzymol. 204, 125-39). Amino acid mutations can occur in nature, too. The proteins suitable for use in the context present invention include those proteins having the amino acid sequences of the human TTK protein in which one or more amino acids are mutated, provided the resulting mutated proteins are functionally equivalent to the human TTK protein. The number of amino acids to be mutated in such a mutant is generally 25 amino acids or less, preferably 10 to 15 amino acids or less, more preferably 5 to 6 amino acids or less, and even more preferably 2 to 3 amino acids or less. To maintain kinase activity for EGFR, it is preferable to conserve the kinase domain in the amino acid sequence of the mutated protein. For example, to maintain the kinase domain of TTK, Asp647 of TTK can not be altered.

Mutated or modified proteins, proteins having amino acid sequences modified by substitution, deletion or insertion of one or more amino acid residues of a certain amino acid sequence, are known to retain the original biological activity (Mark, D. F. et al., Proc. Natl. Acad. Sci. USA (1984) 81, 5662-6, Zoller, M. J. & Smith, M., Nucleic Acids Research (1982) 10, 6487-500, Wang, A. et al., Science (1984) 224, 1431-3, Dalbadie-McFarland, G. et al., Proc. Natl. Acad. Sci. USA (1982) 79, 6409-13).

The amino acid residue to be mutated is preferably mutated into a different amino acid in which the properties of the amino acid side-chain are conserved (a process known in the art as "conservative amino acid substitution"). Examples of properties of amino acid side chains are hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (Q A, V, L, I, P); a hydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Note, the parenthetic letters indicate the one-letter codes of amino acids.

An example of a protein to which one or more amino acids residues are inserted or added to the amino acid sequence of human TTK protein (SEQ ID NO: 2) is a fusion protein containing the human TTK protein. Fusion proteins suitable for use in the context of the present invention include, for example, fusions of the human TTK protein and other peptides or proteins. Fusion proteins can be made using techniques well known to those skilled in the art, for example by linking the DNA encoding the human TTK protein of the invention with DNA encoding other peptides or proteins, so that the frames match, inserting the fusion DNA into an expression vector and expressing it in a host. There is no restriction as to the peptides or proteins to be fused to the protein of the present invention.

Known peptides that can be used as peptides that are fused to the TTK protein include, for example, FLAG (Hopp, T. P. et al., (1988) Biotechnology 6, 1204-10), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, alpha-tubulin fragment, B-tag, Protein C fragment, and the like. Examples of proteins that may be fused to a protein of the invention include GST (glutathione-S-transferase), Influenza agglutinin (HA), immunoglobulin constant region, beta-galactosidase, MBP (maltose-binding protein), and such.

Fusion proteins can be prepared by fusing commercially available DNA, encoding the fusion peptides or proteins discussed above, with the DNA encoding a protein of the present invention and expressing the fused DNA prepared.

An alternative method known in the art to isolate functional equivalent proteins is, for example, the method using a hybridization technique (Sambrook, J. et al., (1989) Molecular Cloning 2nd ed. 9.47-9.58, Cold Spring Harbor Lab. Press). One skilled in the art can readily isolate a DNA having high homology with a whole or part of the TTK DNA sequence (*e.g*., SEQ ID NO: 1) encoding the human TTK protein, and isolate functional equivalent proteins to the human TTK protein from the isolated DNA. The proteins used for the present invention include those that are encoded by DNA that hybridize with a whole or part of the DNA sequence encoding the human TTK protein and are functional equivalent to the human TTK protein. These proteins include mammal homologues corresponding to the protein derived from human or rat (for example, a protein encoded by a monkey, mouse, rabbit and bovine gene). In isolating a cDNA highly homologous to the DNA encoding the human TTK protein from animals, it is particularly preferable to use tissues from testis or lung cancer.

The conditions of hybridization for isolating a DNA encoding a protein functionally equivalent to the human TTK protein can be routinely selected by a person skilled in the art. For example, hybridization may be performed by conducting pre-hybridization at 68°C for 30 min or longer using "Rapid-hyb buffer" (Amersham LIFE SCIENCE), adding a labeled probe, and warming at 68°C for 1 hour or longer. The following washing step can be conducted, for example, for a low stringency condition. Exemplary low stringency conditions include, for example, 42°C, 2x SSC, 0.1% SDS, or preferably 50°C, 2x SSC, 0.1% SDS. More preferably, high stringency conditions are selected. Exemplary high stringency conditions include, for example, washing 3 times in 2x SSC, 0.01% SDS at room temperature for 20 min, then washing 3 times in 1x SSC, 0.1% SDS at 37°C for 20 min and washing twice in 1x SSC, 0.1% SDS at 50°C for 20 min. However, several factors, such as temperature and salt concentration, can influence the stringency of hybridization, Selection of the factors necessary to achieve a requisite level of stringency constitutes routine optimization that is well within the purview of one skilled in the art.

In place of hybridization, a gene amplification method, for example, the polymerase chain reaction (PCR) method, can be utilized to isolate a DNA encoding a protein functionally equivalent to the human TTK protein, using a primer synthesized based on the sequence information of the DNA (SEQ ID NO: 1) encoding the human TTK protein (SEQ ID NO: 2).

As noted above, proteins that are functionally equivalent to the human TTK protein, encoded by the DNA isolated through the above hybridization techniques or gene amplification techniques, normally have a high homology to the amino acid sequence of the human TTK protein. In the context of the present invention, the term "high homology" refers to a homology of 40% or higher, preferably 60% or higher, more preferably 80% or higher, even more preferably 95% or higher. The homology of a protein can be determined by following the algorithm in "Wilbur, W. J. and Lipman, D. J. (1983) Proc. Natl. Acad. Sci. USA 80, 726-30".

A protein useful in the context of the present invention may have variations in amino acid sequence, molecular weight, isoelectric point, the presence or absence of sugar chains, or form, depending on the cell or host used to produce it or the purification method utilized. Nevertheless, so long as it is functionally equivalent to a human TTK protein (SEQ ID NO: 2), it is useful in the context of the present invention.

The proteins useful in the context of the present invention can be prepared as recombinant proteins or natural proteins, by methods well known to those skilled in the art. A recombinant protein can be prepared, for example, by inserting a DNA encoding a protein of the present invention (for example, the DNA having the nucleotide sequence of SEQ ID NO: 1) into an appropriate expression vector, introducing the vector into an appropriate host cell, obtaining the extract, and purifying the protein by subjecting the extract to chromatography, for example, ion exchange chromatography, reverse phase chromatography, gel filtration, or affinity chromatography utilizing a column to which antibodies against the protein of the present invention are fixed, or by combining more than one of aforementioned columns.

In addition, when a protein useful in the context of the present invention is expressed within host cells (for example, animal cells and *E. coli*) as a fusion protein with glutathione-S-transferase protein or as a recombinant protein supplemented with multiple histidines, the expressed recombinant protein can be purified using a glutathione column or nickel column.

After purifying the fusion protein, it is also possible to exclude regions, other than the objective protein, by cutting with thrombin or factor-Xa as required.

A natural protein can be isolated by methods known to those skilled in the art, for example, by contacting an affinity column, in which antibodies binding to the TTK protein described below are bound, with the extract of tissues or cells expressing a protein of the present invention. The antibodies can be polyclonal antibodies or monoclonal antibodies.

In the present invention, a kinase activity of TTK or its functional equivalent can be determined by methods known in the art. For example, TTK and EGFR can be incubated with an ATP, under suitable assay conditions for a phosphorylation of EGFR by TTK. In the present invention, exemplary conditions for the phosphorylation of EGFR include the steps of contacting TTK with EGFR or cell extracts, and incubating them. In the present invention, conditions for the phosphorylation of EGFR may be provided by the incubation of TTK with EGFR in the presence of phosphate donor. An ATP is an example of suitable phosphate donor. For example, a radio labeled ATP can be the phosphate donor. The increased radio-labeled-EGFR may be detected by any suitable method. For example, in a hot assay, the radio-labeled-EGFR is detected by scintillation counter. On the other hand, in a cold assay, the phospho-EGFR is detected using an antibody binding to phospho-EGFR, *e.g*. western blot assay or ELISA. For example, suitable conditions for phosphorylation of EGFR by TTK are set forth below:
Reaction mixture:
   50 mM Tris-HCl (pH 7.4),
   10 mM MgCl₂,
   2 mM dithiothreitol,
   1 mM NaF,
   0.2 mM ATP

The reaction mixture is mixed with a sample containing TTK to be determined, and incubated for 60 min. at 30°C. The reactions were stopped by addition of Laemmli sample buffer and heating at 95°C for 5 min. Proteins were resolved by SDS-PAGE and then western-blot using an antibody binding to phospho-EGFR.

Alternatively, a kinase activity of TTK for EGFR can be estimated based on a radio-labeled-EGFR by scintillation counter. A phospho-EGFR can be detected by an antibody based detection system, *e.g*. ELISA or western blot assay. Alternatively, a phospho-EGFR can be detected with mass spectrometry, *e.g*. MALDI-TOF-MS. For example, the phosphorylation site of EGFR by TTK is Tyr992 or Ser967. The kinase activity of TTK for EGFR at Tyr992 or Ser967 may be detected by using an antibody specific for phospho-EGFR (Tyr992 or Ser967).

Various low-throughput and high-throughput enzyme assay formats are know in the art and can be readily adapted for detection or measuring of the phosphorylation level of EGFR by TTK. For high-throughput assays, the EGFR is preferably immobilized on a solid support, such as a multi-well plate, slide or chip. Following the reaction, the phospho-EGFR can be detected on the solid support. In order to detect phospho-EGFR, for example, an antibody binding to phospho-EGFR can be used. For example, the phosphorylation site of EGFR by TTK is Tyr992 or Ser967. The phosphorylation of EGFR at Tyr992 or Ser967 by TTK may be detected by using an antibody specific for phosphor-EGFR (Tyr992 or Ser967). Alternatively, P³² labeled ATP may be used as a phosphate donor. The phospho-EGFR can be traced with radioactive P³². To facilitate such assays, the solid support may be coated with streptavidin and the EGFR labeled with biotin. The skilled person can determine suitable assay formats depending on the desired throughput capacity of the screen.

Any test compound, including, but not limited to, cell extracts, cell culture supernatant, products of fermenting microorganisms, extracts from marine organisms, plant extracts, purified or crude proteins, peptides, non-peptide compounds, synthetic micromolecular compounds and natural compounds, can be used in the screening methods of the present invention. The test compound of the present invention can be also obtained using any of the numerous approaches in combinatorial library methods known in the art, including (1) biological libraries, (2) spatially addressable parallel solid phase or solution phase libraries, (3) synthetic library methods requiring deconvolution, (4) the "one-bead, one-compound" library method and (5) synthetic library methods using affinity chromatography selection. The biological library methods using affinity chromatography selection are limited to peptide libraries, while the other four approaches are applicable to peptide, non-peptide oligomer or small molecule libraries of compounds (Lam (1997) Anticancer Drug Des. 12: 145-67). Examples of methods for the synthesis of molecular libraries can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37:1233-51.). Libraries of compounds may be presented in solution (see Houghten (1992) Bio/Techniques 13: 412-21.) or on beads (Lam (1991) Nature 354: 82-4.), chips (Fodor (1993) Nature 364: 555-6.), bacteria (US Pat. No. 5,223,409), spores (US Pat. No. 5,571,698; 5,403,484, and 5,223,409), plasmids (Cull et al. (1992) Proc. Natl. Acad. Sci. USA 89: 1865-9.) or phage (Scott and Smith (1990) Science 249: 386-90.; Devlin (1990) Science 249: 404-6.; Cwirla et al. (1990) Proc. Natl. Acad. Sci. USA 87: 6378-78.; Felici (1991) J. Mol. Biol. 222: 301-10.; US Pat. Application 2002103360).

A compound isolated by the screening method of the present invention is a candidate for the development of drugs that inhibit a kinase activity af TTK for EGFR and can be applied to the treatment or prevention of lung cancer. Especially, the kinase activity of TTK for EGFR is ECrF-independent., Alternatively, the phosphorylation site of EGFR is Tyr-992 or Ser-967.

Moreover, a compound in which a part of the structure of the compound inhibiting the kinase activity of TTK for EGFR is converted by addition, deletion and/or replacement are also included in the compounds obtainable by the screening method of the present invention.

### Treating and preventing lung cancer:

The present invention provides compositions for treating or preventing lung cancer containing any of the compounds selected by the screening methods of the present invention.

When administrating a compound isolated by a method of the present invention as a pharmaceutical for humans and other mammals, such as mice, rats, guinea-pigs, rabbits, cats, dogs, sheep, pigs, cattle, monkeys, baboons, and chimpanzees, the isolated compound can be directly administered or, alternatively, can be formulated into a dosage form using conventional pharmaceutical preparation methods. For example, according to the need, the drugs can be taken orally, as sugar-coated tablets, capsules, elixirs and microcapsules, or non-orally, in the form of injections of sterile solutions or suspensions with water or any other pharmaceutically acceptable liquid. For example, the compound can be mixed with pharmaceutically acceptable carriers or media, specifically, sterilized water, physiological saline, plant-oils, emulsifiers, suspending agents, surfactants, stabilizers, flavoring agents, excipients, vehicles, preservatives, binders, and such, in a unit dose form required for generally accepted drug implementation. The amount of active ingredients in these preparations makes a suitable dosage within the indicated range acquirable.

Examples of additives that can be mixed to form tablets and capsules include, for example, binders, such as gelatin, corn starch, tragacanth gum and arabic gum; excipients, such as crystalline cellulose; swelling agents, such as corn starch, gelatin and alginic acid; lubricants, such as magnesium stearate; sweeteners, such as sucrose, lactose or saccharin; and flavoring agents, such as peppermint, Gaultheria adenothrix oil and cherry. When the unit-dose form is a capsule, a liquid carrier, such as an oil, can also be further included in the above ingredients. Sterile composites for injections can be formulated following normal drug implementations using vehicles such as distilled water used for injections.

Physiological saline, glucose, and other isotonic liquids, including adjuvants, such as D-sorbitol, D-mannose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil and soy-bean oil are examples of suitable oleaginous liquids and may be used in conjunction with benzyl benzoate or benzyl alcohol as solubilizers. They may be further formulated with a buffer, such as phosphate buffer or sodium acetate buffer; a pain-killer, such as procaine hydrochloride; a stabilizer, such as benzyl alcohol or phenol; and an anti-oxidant. The prepared injection may be filled into a suitable ampule.

Methods well known to those skilled in the art may be used to administer a pharmaceutical composition of the present invention to patients, for example, as intra-arterial, intravenous, or percutaneous injections and also as intranasal, transbronchial, intramuscular or oral administrations. The dosage and method of administration may vary according to the body-weight and age of the patient and the selected administration method; however, one skilled in the art can routinely select a suitable method of administration and dosage. If said compound is encodable by a DNA, the DNA can be inserted into a vector for gene therapy and the vector can be administered to a patient to perform the therapy. The dosage and method of administration may again vary according to the body-weight, age, and symptoms of the patient; however, one skilled in the art can suitably select them.

For example, although the dose of a compound that binds to TTK and regulates its activity depends on the symptoms, a suitable dose is generally about 0.1 mg to about 100 mg per day, preferably about 1.0 mg to about 50 mg per day and more preferably about 1.0 mg to about 20 mg per day, when administered orally to a normal adult (weight 60 kg).

When administering parenterally, in the form of an injection to a normal adult (weight 60 kg), although there are some differences according to the patient, target organ, symptoms and method of administration, it is convenient to intravenously inject a dose of about 0.01 mg to about 30 mg per day, preferably about 0.1 to about 20 mg per day and more preferably about 0.1 to about 10 mg per day. Also, in the case of other animals too, it is possible to administer an amount converted to 60 kg of body-weight.

In another aspect, the present invention includes pharmaceutical, or therapeutic, compositions containing one or more therapeutic compounds described herein. Pharmaceutical formulations may include those suitable for oral, rectal, nasal, topical (including buccal and sub-lingual), vaginal or parenteral (including intramuscular, subcutaneous and intravenous) administration, or for administration by inhalation or insufflation. The formulations may, where appropriate, be conveniently presented in discrete dosage units and may be prepared by any of the methods conventional in the art of pharmacy. All such Pharmaceutical methods herein include the steps of bringing into association the active compound with liquid carriers or finely divided solid carriers or both as needed and then, if necessary, shaping the product into the desired formulation.

Pharmaceutical formulations suitable for oral administration may conveniently be presented as discrete units, such as capsules, cachets or tablets, each containing a predetermined amount of the active ingredient; as a powder or granules; or as a solution, a suspension or as an emulsion. The active ingredient may also be presented as a bolus electuary or paste, and be in a pure form, *i.e.,* without a carrier. Tablets and capsules for oral administration may contain conventional excipients, such as binding agents, fillers, lubricants, disintegrant or wetting agents. A tablet may be made by compression or molding, optionally with one or more formulational ingredients. Compressed tablets may be prepared by compressing in a suitable machine the active ingredients in a free-flowing form, such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may be coated according to methods well known in the art. Oral fluid preparations may be in the form of, for example, aqueous or oily suspensions, solutions, emulsions, syrups or elixirs, or may be presented as a dry product for constitution with water or other suitable vehicle before use. Such liquid preparations may contain conventional additives, such as suspending agents, emulsifying agents, non-aqueous vehicles (which may include edible oils), or preservatives. Furthermore, the tablets may optionally be formulated so as to provide slow or controlled release of the active ingredient therein.

Formulations for parenteral administration include aqueous and non-aqueous sterile injection solutions which may contain anti-oxidants, buffers, bacteriostats and solutes which render the formulations isotonic with the blood of the intended recipient; and aqueous and non-aqueous sterile suspensions which may include suspending agents and thickening agents. The formulations may be presented in unit dose or multi-dose containers, for example, sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion. Extemporaneous injection solutions and suspensions may be prepared from sterile powders, granules and tablets of the kind previously described.

Formulations for rectal administration may be presented as a suppository with the usual carriers, such as cocoa butter or polyethylene glycol. Formulations for topical administration in the mouth, for example, buccally or sublingually, include lozenges, containing the active ingredient in a flavored base, such as sucrose and acacia or tragacanth, and pastilles containing the active ingredient in a base, such as gelatin and glycerin or sucrose and acacia. For intra-nasal administration, the compounds of the present invention may be used as a liquid spray or dispersible powder or in the form of drops. Drops may be formulated with an aqueous or non-aqueous base also including one or more dispersing agents, solubilizing agents or suspending agents. Liquid sprays are conveniently delivered from pressurized packs.

For administration by inhalation, the compounds of the present invention are conveniently delivered from an insufflator, nebulizer, pressurized pack or other convenient aerosol spray delivery means. Pressurized packs may include a suitable propellant, such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount.

Alternatively, for administration by inhalation or insufflation, the compounds of the present invention may take the form of a dry powder composition, for example a powder mix of the compound and a suitable powder base, such as lactose or starch. The powder composition may be presented in a unit dosage form, in for example, capsules, cartridges, gelatin or blister packs from which the powder may be administered with the aid of an inhalator or insulators.

When desired, the above-described formulations, adapted to give sustained release of the active ingredient, may be employed. The pharmaceutical compositions of the present invention may also contain other active ingredients, such as antimicrobial agents, immunosuppressants or preservatives.

It should be understood that in addition to the ingredients particularly mentioned above, the formulations of the present invention may include other agents conventional in the art, having regard to the type of formulation in question; for example, those suitable for oral administration may include flavoring agents.

Preferred unit dosage formulations are those containing an effective dose, as recited below, or an appropriate fraction thereof, of the active ingredient.

For each of the aforementioned conditions, the compositions may be administered orally or via injection at a dose ranging from about 0.1 to about 250 mg/kg per day. The dose range for adult humans is generally from about 5 mg to about 17.5 g/day, preferably about 5 mg to about 10g/day, and most preferably about 100 mg to about 3 g/day. Tablets or other unit dosage forms of presentation provided in discrete units may conveniently contain an amount which is effective at such dosage or as a multiple of the same, for instance, units containing about 5 mg to about 500 mg, usually from about 100 mg to about 500 mg.

The pharmaceutical composition preferably is administered orally or by injection (intravenous) or subcutaneous), and the precise amount administered to a subject will be the responsibility of the attendant physician. However, the dose employed will depend upon a number of factors, including the age and sex of the subject, the precise disorder being treated, and its severity. In addition, the route of administration may vary depending upon the condition and its severity.

### Diagnosing metastasis of lung cancer

The present invention is based in part on the discovery of the various amino acid substitutions of TTK at kinase domain, especially the mutations resulted in promoting the TTK kinase activity and the invasive ability. In view of the evidence provided herein, that one or more amino acid substitutions of TTK at kinase domain are associated with a metastasis of lung cancer, the present invention thus provides methods for predicting metastasis of lung cancer. An example of such a method includes the steps of: detecting one or more mutations of TTK at kinase domain, and then indicating a high risk of metastasis of lung cancer when a mutation is detected. In the context of the present invention, one or more amino acid substitutions of TTK at kinase domain are Valine to Phenylalanine at codon 610 (V610F), Glutamine to Histidine at codon 753 (Q753H) and Tyrosine to Cysteine at codon 574 (Y574C) of SEQ ID NO: 2. The detection can be accomplished by sequencing, mini-sequencing, hybridization, restriction fragment analysis, oligonucleotide ligation assay or allele specific PCR.

It is revealed herein that the presence of a previously unknown TTK mutant correlates with a high risk of metastasis of lung cancer. Based on this finding, the present invention also provides a method and reagent for detecting such TTK mutant. For instance, the present invention provides a method for detecting one or more mutation of TTK, wherein the mutation is at least one mutation selected from the group consisted of Valine to Phenylalanine at codon 610 (V610F), Glutamine to Histidine at codon 753 (Q753H) and Tyrosine to Cysteine at codon 574 (Y574C) af SEQ ID NO: 2. The method of the present invention includes steps of:
a) contacting a subject polypeptide or cDNA encoding thereto with a binding agent recognizing any one of the mutation of the polypeptide or cDNA encoding thereto,
b) detecting the binding agent with the polypeptide or cDNA encoding thereto, and
c) showing the mutation of TTK when the binding of the agent of step b) is detected.

In the present invention, a binding agent recognizing any one of the mutation of the polypeptide is preferably an antibody that binds to polypeptide having at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, and substantially not binds to the polypeptide of SEQ ID NO: 2. Further, the antibody may be monoclonal antibody, or polyclonal antibody, or fragment thereof that contains the antigen-binding region. In the present invention, such antibodies may be obtained by conventional methods of immunization of TTK polypeptide or immunological-active fragment containing the mutation. Alternatively, a screening of antibody variable region recognizing the TTK mutant from antibody library may be performed using TTK mutant as antigen.

In the present invention, a preferred antibody will specifically recognize the TTK mutant. Such antibody is referred as TTK mutant specific antibody. Alternatively, in the present invention, a preferred TTK mutant specific antibody will substantially not bind to the polypeptide of SEQ ID NO: 2. In the context, antibody which substantially does not bind with wild type of TTK having the amino acid sequence of SEQ ID NO: 2 shows generally 30% or less, preferably 20% or less, more preferably 10% or less of reactivity with wild type of TTK, comparing with that of TTK mutant.

In the present invention, the TTK mutant includes at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2. Such mutant may be prepared by conventional method of introducing site specific mutations into the protein. For example, one skilled in the art can prepare proteins having the mutation by introducing site specific mutation in the amino acid sequence of the human TTK protein via site-directed mutagenesis (Hashimoto-Gotoh, T. et ar. (1995), Gene 152, 271-5; Zoller, MJ, and Smith, M. (1983), Methods Enzymol. 100,468-500; Kramer, W. et al. (1984), Nucleic Acids Res. 12, 9441-56; Framer W, and Fritz HJ. (1987) Methods. Enzymol. 154, 350-67; Kunkel, TA (1985), Proc. Natl. Acad. Sci. USA. 82, 488-92; Kunkel (1991), Methods Enzymol. 204, 125-39).

In the present invention, TTK mutant can also be detected by determining of the nucleotide sequence of cDNA encoding TTK mutant. For example, primers or probes annealing with the cDNA (or RNA) at region may include any one codon of Valine to Phenylalanine at codon 610 (V610F), Glutamine to Histidine at codon 753 (Q753H) and Tyrosine to Cysteine at codon 574 (Y574C) of SEQ ID NO: 2.

Further, the present invention also provides a reagent for detecting one or more mutation of TTK, wherein the mutation is at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, wherein the reagent includes a binding agent recognizing any one of the mutation of the polypeptide or cDNA encoding thereto.

In addition, polynucleotides that encode a TTK mutant or fragment thereof that includes the mutated position are useful as control samples for detecting the mutation. Accordingly, the present invention provides an isolated polynucleotide having the nucleotide sequence of SEQ ID NO: 1, in which one or more mutations selected from group consisting of A1870G (for Y574C), G1977T (for V610F), and G2408C (for Q753H) are included, or fragment thereof containing the one or more mutations. For example, in the detection of the mutation using a hybridization technique, polynucleotide of TTK mutant can be used as positive control. In the present invention, any fragment of the polynucleotide of TTK mutant may be used as such control, unless at least one mutation is included. Preferable length of the fragments of the polynucleotide of TTK mutant is generally 25 or more, 50 or more, 100 or more, and more preferably 200 or more nucleotides.

A polypeptide encoding a TTK mutant or fragment thereof containing the mutated position is also useful as control sample for detecting the mutation. Accordingly, the present invention provides an isolated polypeptide having the amino acid sequence of SEQ ID NO: 2, in which one or more mutations selected from group consisting of V610F, Q753H and for Y574C are included, or fragment thereof containing the one or more mutations. For example, in the detection of the mutation using an immunoassay technique, polypeptide of TTK mutant can be used as positive control. In the present invention, any fragment of the polypeptide of TTK mutant may be used as such control, unless at least one mutation is included. The mutation of TTK polypeptide can also be detected by molecular mass analysis of whole peptide or fragment thereof. For instance, preferable technique for the molecular mass analysis is MALDI-TOF MS. Length of the fragments of the polypeptide of TTK mutant is generally 10 or more, preferably 20 or more, or 50 or more, and more preferably 100 or more amino acid residues.

### Dominant negative protein that inhibits TTK kinase activity for EGFR

The present invention relates to inhibitory polypeptides that contain ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46). In some preferred embodiments, the inhibitory polypeptide comprises ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46); a polypeptide functionally equivalent to the polypeptide; or polynucleotide encoding those polypeptides, wherein the polypeptide lacks the kinase activity of TTK for EGFR. It is a novel finding proved by the present invention that that EGFR fragment inhibits the lung cancer cell proliferation.

The polypeptides comprising the selected amino acid sequence of the present invention, can be of any length, so long as the polypeptide contain the amino acid sequence of ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) and inhibits cancer cell proliferation. For example, the length of the amino acid sequence may range from 19 to 76 residues preferably from 19 to 57, more more preferably from 19 to 38.

The polypeptides of the present invention may contain two or more "selected amino acid sequences". The two or more "selected amino acid sequences" may be the same or different amino acid sequences. Furthermore, the "selected amino acid sequences" can be linked directly. Alternatively, they may be disposed with any intervening sequences among them.

Furthermore, the present invention relates to polypeptides homologous (i.e., share sequence identity) to the ISSILEKGERLPQPPICTI (SEQ ID NO: 44) or DVYMIMVKCWMIDADSRPK (SEQ ID NO: 45) or FRELTIEFSKMARDPQRYL (SEQ ID NO: 46) polypeptide specifically disclosed here. In the present invention, polypeptides homologous to the ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) polypeptide are those which contain any mutations selected from addition, deletion, substitution and insertion of one or several amino acid residues and are functionally equivalent. The phrase "functionally equivalent" refers to having the function to inhibit the kinase activity of TTK for EGFR and inhibit the cell proliferation. Therefore, polypeptides functionally equivalent to the ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) peptide in the present invention preferably have amino acid mutations in sites other than the ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) sequence. Amino acid sequences of polypeptides functionally equivalent to the ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) peptide in the present invention conserve the ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) sequence, and have 60% or higher, usually 70% or higher, preferably 80% or higher, more preferably 90% or higher, or 95% or higher, and further more preferably 98% or higher homology to a "selected amino acid sequence". Amino acid sequence homology can be determined using algorithms well known in the art, for example, BLAST or ALIGN set to their default settings.

The polypeptides of the present invention can be chemically synthesized from any position based on selected amino acid sequences. Methods used in the ordinary peptide chemistry can be used for the method of synthesizing polypeptides. Specifically, the methods include those described in the following documents and Japanese Patent publications:
Peptide Synthesis, Interscience, New York, 1966; The Proteins, Vol. 2, Academic Press Inc., New York, 1976;
Peputido gousei (Peptide Synthesis), Maruzen (Inc.), 1975;
Peputido gousei no kiso to jikken (Fundamental and Experimental Peptide Synthesis), Maruzen (Inc.), 1985;
Tyakohin no kaihatsu (Development of Pharmaceuticals), Sequel, Vol. 14: Peputido gousei (Peptide Synthesis), Hirokawa Shoten, 1991;
International Patent Publication WO99167258.

The polypeptides of the present invention can be also synthesized by known genetic engineering techniques. An example of genetic engineering techniques is as follows. Specifically, DNA encoding a desired peptide is introduced into an appropriate host cell to prepare a transformed cell. The polypeptides of the present invention can be obtained by recovering polypeptides produced by this transformed cell. Alternatively, a desired polypeptide can be synthesized with an in vitro translation system, in which necessary elements for protein synthesis are reconstituted in vitro.

When genetic engineering techniques are used, the polypeptide of the present invention can be expressed as a fused protein with a peptide having a different amino acid sequence. A vector expressing a desired fusion protein can be obtained by linking a polynucleotide encoding the polypeptide of the present invention to a polynucleotide encoding a different peptide so that they are in the same reading frame, and then introducing the resulting nucleotide into an expression vector. The fusion protein is expressed by transforming an appropriate host with the resulting vector. Different peptides to be used in forming fusion proteins include the following peptides:
FLAG (Hopp et al., (1988) BioTechnology 6,1204-10),
6xHis consisting of six His (histidine) residues, 10xHis,
Influenza hemagglutinin (HA),
Human c-myc fragment,
VSV-GP fragment,
p18 HIV fragment,
T7-tag,
HSV-tag,
E-tag,
SV40T antigen fragment,
lck tag,
a-tubulin fragment,
B-tag,
Protein C fragment,
GST (glutathione-S-transferase),
HA (Influenza hemagglutinin),
Immunoglobulin constant region,
β-galactosidase, and
MBP (maltose-binding protein).

The polypeptide of the present invention can be obtained by treating the fusion protein thus produced with an appropriate protease, and then recovering the desired polypeptide. To purify the polypeptide, the fusion protein is captured in advance with affinity chromatography that binds with the fusion protein, and then the captured fusion protein can be treated with a protease. With the protease treatment, the desired polypeptide is separated from affinity chromatography, and the desired polypeptide with high purity is recovered.

The polypeptides of the present invention include modified polypeptides. In the present invention, the term "modified" refers, for example, to binding with other substances. Accordingly, in the present invention, the polypeptides of the present invention may further comprise other substances such as cell-membrane permeable substance. The other substances include organic compounds such as peptides, lipids, saccharides, and various naturally-occurring or synthetic polymers. The polypeptides of the present invention may have any modifications so long as the polypeptides retain the desired activity of inhibiting the kinase activity of TTK for EGFR. In some embodiments, the inhibitory polypeptides can directly compete with EGFR binding to TTK. Modifications can also confer additive functions on the polypeptides of the invention. Examples of the additive functions include targetability, deliverability, and stabilization.

Preferred examples of modifications in the present invention include, for example, the introduction of a cell-membrane permeable substance. Usually, the intracellular structure is cut off from the outside by the cell membrane. Therefore, it is difficult to efficiently introduce an extracellular substance into cells. Cell membrane permeability can be conferred on the polypeptides of the present invention by modifying the polypeptides with a cell-membrane permeable substance. As a result, by contacting the polypeptide of the present invention with a cell, the polypeptide can be delivered into the cell to act thereon.

The "cell-membrane permeable substance" refers to a substance capable of penetrating the mammalian cell membrane to enter the cytoplasm. For example, a certain liposome fuses with the cell membrane to release the content into the cell. Meanwhile, a certain type of polypeptide penetrates the cytoplasmic membrane of mammalian cell to enter the inside of the cell. For polypeptides having such a cell-entering activity, cytoplasmic membranes and such in the present invention are preferable as the substance. Specifically, the present invention includes polypeptides having the following general formula.

[R]-[D];

wherein,
[R] represents a cell-membrane permeable substance; [D] represents a fragment sequence containing ISSILEKGERLPQPPICTI (SEQ ID NO: 44) or DVYMIWKCWVMADSRPK (SEQ ID NO: 45) or FRELIIEFSKMARDPQRYL (SEQ ID NO: 46). In the above-described general formula, [R] and [D] can be linked directly or indirectly through a linker. Peptides, compounds having multiple functional groups, or such can be used as a linker. Specifically, amino acid sequences containing -GGG- can be used as a linker. Alternatively, a cell-membrane permeable substance and a polypeptide containing a selected sequence can be bound to the surface of a minute particle. [R] can be linked to any positions of [D]. Specifically, [R] can be linked to the N terminal or C terminal of [D], or to a side chain of amino acids constituting [D]. Furthermore, more than one [R] molecule can be linked to one molecule of [D]. The [R] molecules can be introduced to different positions on the [D] molecule. Alternatively, [D] can be modified with a number of [R]s linked together.

For example, there have been reported a variety of naturally-occurring or artificially synthesized polypeptides having cell-membrane permeability (Joliot A. & Prochiantz A., Nat Cell Biol. 2004; 6: 189-96). All of these known cell-membrane permeable substances can be used for modifying polypeptides in the present invention. In the present invention, for example, any substance selected from the following group can be used as the above-described cell-permeable substance:
poly-arginine; Matsushita et al., (2003) J. Neurosci.; 21, 6000-7.
[Tat / RKKRRQRRR] (SEQ ID NO: 47) Frankel et al., (1988) Cell 55,1189-93.
Green & Loewenstein (1988) Cell 55,1179-88.
[Penetratin / RQIYIWQNRRMYWKK] (SEQ ID NO: 48)
Derossi et al., (1994) J. Biol. Chem. 269, 10444-50.
[Buforin II / TRSSRAGLQFPVGRVHRLLRK] (SEQ ID NO: 49)
Park et al., (2000) Proc. Natl Acad. Sci. USA 97, 8245-50.
[Transportan / GWTLNSAGYLLGKINLKALAALAKKIL] (SEQ ID NO: 50)
Pooga et al., (1998) FASEB J. 12,67-77.
[MAP (model amphipathic peptide) / KLALKLALKALKAALKLA] (SEQ ID NO: 51)
Oehlke et al., (1998) Biochim. Biophys. Acta. 1414, 127-39.
[K-FGF /AAVALLPAVLLALLAP] (SEQ ID NO: 52)
Lin et al., (1995) J. Biol. Chem. 270, 14255-8.
[Ku70 / VPMLK] (SEQ ID NO: 53)
Sawada et al., (2003) Nature Cell Biol. 5, 352-7.
[Ku70 / PMLKE] (SEQ ID NO: 61)
Sawada et al., (2003) Nature Cell Biol. 5, 352-7.
[Prion / MANLGYWLLALFVTMWTDVGLCKKRPKP] (SEQ ID NO: 54)
Lundberg et al., (2002) Biochem. Biophys. Res. Commun. 299, 85-30.
[pVEC / LLIILRRRIRKQAHAHSK] (SEQ ID NO: 55)
Elmquist et al., (2001) Exp. Cell Res. 269, 237-44.
[Pep-1 / KETWWETWWTEWSQPKKKRKV] (SEQ ID NO: 56)
Morris et al., (2001) Nature Biotechnol. 19, 1173-6.
[SynB1 / RGGRLSYSRRRFSTSTGR] (SEQ ID NO: 57)
Rousselle et al., (2000) Mol. Pharmacol. 57, 679-86.
[Pep-7 / SDLWEMMMVSLACQY] (SEQ ID NO: 58)
Gao et al., (2002) Bioorg. Med. Chef. 10, 4057-65.
[HN-1 / TSPLNIHNGQKL] (SEQ ID NO: 59)
Hong & Clayman (2000) Cancer Res. 60, 6551 -6.
In the present invention, the poly-arginine, which is listed above as an example of cell-membrane permeable substances, is constituted by any number of arginine residues. Specifically, for example, it is constituted by consecutive 5-20 arginine residues. The preferable number of arginine residues is 11 (SEQ ID NO: 60).

### Pharmaceutical compositions comprising ISSILEKGERLPQPPICTI (SEQ ID NO_{:} 44) or DVYAHMVKCWMIDADSRPK (SEQ ID NO: 45) or FRELIIEFSKALARDPQRYL (SEQ ID NO: 46)

The polypeptide of the present invention inhibit proliferation of lung cancer cells. Therefore, the present invention provides therapeutic and/or preventive agents for cancer which comprise as an active ingredient a polypeptide which comprises ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46); or a polynucleotide encoding the same. Alternatively, the present invention relates to methods for treating and/or preventing lung cancer comprising the step of administering a polypeptide of the present invention. Furthermore, the present invention relates to the use of the polypeptides of the present invention in manufacturing pharmaceutical compositions for treating and/or preventing lung cancer. Furthermore, the present invention also relates to a polypeptide selected from peptides comprising ISSILEKGERLPQPPICTI (SEQ ID NO: 44) or DVYMIMVKCWMIDADSRPK (SEQ ID NO: 45) or FRELIIEFSKMARDPQRYL (SEQ ID NO: 46) for treating and/or preventing lung cancer.

Alternatively, the inhibitory polypeptides of the present invention can be used to induce apoptosis of cancer cells. Therefore, the present invention provides apoptosis inducing agents for cells, which comprise as an active ingredient a polypeptide which comprises ISSILEKGERLPQPPICTI (SEQ ID NO: 44) or DVYMIMVKCWMIDADSRPK (SEQ ID NO: 45) or FRELHEFSKMARDPQRYL (SEQ ID NO: 46); or a polynucleotide encoding the same. The apoptosis inducing agents of the present invention may be used for treating cell proliferative diseases such as cancer. Alternatively, the present invention relates to methods for inducing apoptosis of cells which comprise the step of administering the polypeptides of the present invention. Furthermore, the present invention relates to the use of polypeptides of the present invention in manufacturing pharmaceutical compositions for inducing apoptosis in cells.

The inhibitory polypeptides of the present invention induce apoptosis in TTK-expressing cells such as lung cancer. In the meantime, TTK expression has not been observed in most of normal organs. In some normal organs, the expression level af TTK is relatively low as compared with lung cancer tissues. Accordingly, the polypeptides of the present invention may induce apoptosis specifically in lung cancer cells.

When the polypeptides of the present invention are administered, as a prepared pharmaceutical, to human and other mammals such as mouse, rat, guinea pig, rabbit, cat, dog, sheep, Pig, cattle, monkey, baboon and chimpanzee for treating lung cancer or inducing apoptosis in cells, isolated compounds can be administered directly, or formulated into an appropriate dosage form using known methods for preparing pharmaceuticals. For example, if necessary, the pharmaceuticals can be orally administered as a sugar-coated tablet, capsule, elixir, and microcapsule, or alternatively parenterally administered in the injection form that is a sterilized solution or suspension with water or any other pharmaceutically acceptable liquid. For example, the compounds can be mixed with pharmacologically acceptable carriers or media, specifically sterilized water, physiological saline, plant oil, emulsifier, suspending agent, surfactant, stabilizer, corrigent, excipient, vehicle, preservative, and binder, in a unit dosage form necessary for producing a generally accepted pharmaceutical. Depending on the amount of active ingredient in these formulations, a suitable dose within the specified range can be determined.

Examples of additives that can be mixed in tablets and capsules are binders such as gelatin, corn starch, tragacanth gum, and gum arabic; media such as crystalline cellulose; swelling agents such as corn starch, gelatin, and alginic acid; lubricants such as magnesium stearate; sweetening agents such as sucrose, lactose or saccharine; and corrigents such as peppermint, wintergreen oil and cherry. When the unit dosage from is capsule, liquid carriers such as oil can be further included in the above-described ingredients. Sterilized mixture for injection can be formulated using media such as distilled water for injection according to the realization of usual pharmaceuticals.

Physiological saline, glucose, and other isotonic solutions containing adjuvants such as D-sorbitol, D-mannose, D-mannitol, and sodium chloride can be used as an aqueous solution for injection. They can be used in combination with a suitable solubilizer, for example, alcohol, specifically ethanol and polyalcohols such as propylene glycol and polyethylene glycol, non-ionic surfactants such as Polysorbate 80TM and HCO-50.

Sesame oil or soybean oil can be used as an oleaginous liquid, and also used in combination with benzyl benzoate or benzyl alcohol as a solubilizer. Furthermore, they can be further formulated with buffers such as phosphate buffer and sodium acetate buffer; analgesics such as procaine hydrochloride; stabilizers such as benzyl alcohol and phenol; and antioxidants. Injections-thus prepared can be loaded into appropriate ampoules.

Methods well-known to those skilled in the art can be used for administering pharmaceutical compounds of the present invention to patients, for example, by intraarterial, intravenous, or subcutaneous injection, and similarly, by intranasal, transtracheal, intramuscular, or oral administration. Doses and administration methods are varied depending on the body weight and age of patients as well as administration methods. However, those skilled in the art can routinely select them. DNA encoding a polypeptide of the present invention can be inserted into a vector for the gene therapy, and the vector can be administered for treatment. Although doses and administration methods are varied depending on the body weight, age, and symptoms of patients, those skilled in the art can appropriately select them. For example, a dose of the compound which bind to the polypeptides of the present invention so as to regulate their activity is, when orally administered to a normal adult (body weight 60 kg), about 0.1 mg to about 100 mg/day, preferably about 1.0 mg to about 50 mg/day, more preferably about 1.0 mg to about 20 mg/day, although it is slightly varied depending on symptoms.

When the compound is parenterally administered to a normal adult (body weight 60 kg) in the injection form, it is convenient to intravenously inject a dose of about 0.01 mg to about 30 mg/day, preferably about 0.1 mg to about 20 mg/day, more preferably about 0.1 mg to about 10 mg/day, although it is slightly varied depending on patients, target organs, symptoms, and administration methods. Similarly, the compound can be administered to other animals in an amount converted from the dose for the body weight of 60 kg.

### siRNA of TTK or EGFR.

siRNA of TTK or EGFR gene can be used to reduce the expression level of the TTK or EGFR gene. For example, siRNA of TTK or EGFR gene is useful for the treatment of lung cancer. Specifically, siRNA of the present invention can act by binding to mRNAs corresponding thereto, thereby promoting the degradation of the mRNA, and/or inhibiting the expression of protein encoded by the TTK or EGFR gene, thereby, inhibiting the function of the protein.

The term "polynucleotide" and "oligonucleotide" are used interchangeably herein unless otherwise specifically indicated and are referred to by their commonly accepted single-letter codes. The terms apply to nucleic acid (nucleotide) polymers in which one or more nucleic acids are linked by ester bonding. The polynucleotide or oligonucleotide may be composed of DNA, RNA or a combination thereof.

As use herein, the term "double-stranded molecule" refers to a nucleic acid molecule that inhibits expression of a target gene including, for example, short interfering RNA (siRNA; e.g., double-stranded ribonucleic acid (dsRNA) or small hairpin RNA (shRNA)) and short interfering DNA/RNA (siD/R-NA; e.g. double-stranded chimera of DNA and RNA (dsD/R-NA) or small hairpin chimera of DNA and RNA (shD/R-NA)).

Also, an siRNA against the TTK or EGFR gene can be used to reduce the expression level of the TTK or EGFR gene. Herein, term "siRNA" refers to a double stranded RNA molecule which prevents translation of a target mRNA. Standard techniques for introducing siRNA into the cell can be used, including those in which DNA is a template from which RNA is transcribed. In the context of the present invention, the siRNA is composed of a sense nucleic acid sequence and an anti-sense nucleic acid sequence against SEQ ID NO: 62 or 63. The siRNA is constructed such that a single transcript has both the sense and complementary antisense sequences from the target gene, e.g., a hairpin. The siRNA may either be a dsRNA or shRNA.

As used herein, the term "dsRNA" refers to a construct of two RNA molecules having sequences complementary to one another annealed together via the complementary sequences to form a double-stranded RNA molecule. The nucleotide sequence of two strands may include not only the "sense" or "antisense" RNAs selected from a protein coding sequence of target gene sequence, but also RNA molecule having a nucleotide sequence selected from non-coding region of the target gene.

The term "shRNA", as used herein, refers to an siRNA having a stem-loop structure, composed of first and second regions complementary to one another, i.e., sense and antisense strands. The degree of complementarity and orientation of the regions being sufficient such that base pairing occurs between the regions, the first and second regions being joined by a loop region, the loop resulting from a lack of base pairing between nucleotides (or nucleotide analogs) within the loop region. The loop region of an shRNA is a single-stranded region intervening between the sense and antisense strands and may also be referred to as "intervening single-strand".

As use herein, the term "siD/R-NA" refers to a double-stranded polynucleotide molecule which is composed of both RNA and DNA, and includes hybrids and chimeras of RNA and DNA and prevents translation of a target mRNA. Herein, a hybrid indicates a molecule wherein a polynucleotide composed of DNA and a polynucleotide composed of RNA hybridize to each other to form the double-stranded molecule; whereas a chimera indicates that one or both of the strands composing the double stranded molecule may contain RNA and DNA. Standard techniques of introducing siD/R-NA into the cell are used. The siD/R-NA includes a TTK or EGFR sense nucleic acid sequence (also referred to as "sense strand"), a TTK or EGFR antisense nucleic acid sequence (also referred to as "antisense strand") or both. The siD/R-NA may be constructed such that a single transcript has both the sense and complementary antisense nucleic acid sequences from the target gene, e.g., a hairpin. The siD/R-NA may either be a dsD/R-NA or shDIR-NA.

As used herein, the term "dsDIR-NA" refers to a construct of two molecules having sequences complementary to one another annealed together via the complementary sequences to form a double-stranded polynucleotide molecule. The nucleotide sequence of two strands may include not only the "sense" or "antisense" polynucleotides sequence selected from a protein coding sequence of target gene sequence, but also polynucleotide having a nucleotide sequence selected from non-coding region of the target gene. One or both of the two molecules constructing the dsD/R-NA are composed of both RNA and DNA (chimeric molecule), or alternatively, one of the molecules is composed of RNA and the other is composed of DNA (hybrid double-strand).

The term "shD/R-NA", as used herein, refers to an siD/R-NA having a stem-loop structure, including first and second regions complementary to one another, i.e., sense and antisense strands. The degree of complementarity and orientation of the regions being sufficient such that base pairing occurs between the regions, the first and second regions being joined by a loop region, the loop resulting from a lack of base pairing between nucleotides. (or nucleotide analogs) within the loop region. The loop region of an shDIR-NA is a single-stranded region intervening between the sense and antisense strands and may also be referred to as "intervening single-strand"

The double-stranded molecules of the invention may contain one or more modified nucleotides and/or non-phosphodiester linkages. Chemical modifications well known in the art are capable of increasing stability, availability, and/or cell uptake of the double-stranded molecule. The skilled person will be aware of other types of chemical modification which may be incorporated into the present molecules (WO03/070744; WO2005/045037). In one embodiment, modifications can be used to provide improved resistance to degradation or improved uptake. Examples of such modifications include phosphorothioate linkages, 2'-O-methyl-4' linked ribonucleotides, 2'-O-ethyl ribonucleotides (especially on the sense strand of a double-stranded molecule), 2'-deoxy-fluoro ribonucleotides, 2'-deoxy ribonucleotides, "universal base" nucleotides, 5'-C- methyl nucleotides, and inverted deoxyabasic residue incorporation (US20060122137).

In another embodiment, modifications can be used to enhance the stability or to increase targeting efficiency of the double-stranded molecule. Modifications include chemical cross linking between the two complementary strands of a double-stranded molecule, chemical modification of a 3' or 5' terminus of a strand of a double-stranded molecule, sugar modifications, nucleobase modifications and/or backbone modifications, 2-fluoro modified ribonucleotides and 2'-deoxy ribonucleotides (WO2004/029212). In another embodiment, modifications can be used to increased or decreased affinity for the complementary nucleotides in the target RNA and/or in the complementary double-stranded molecule strand (W02005/044976). For example, an unmodified pyrimidine nucleotide can be substituted for a 2-thio, 5-alkynyl, 5-methyl, or 5-propynyl pyrimidine. Additionally, an unmodified purine can be substituted with a 7-deza, 7-alkyi, or 7-alkenyi purine. In another embodiment, when the double-stranded molecule is a double-stranded molecule with a 3' overhang, the 3'- terminal nucleotide overhanging nucleotides may be replaced by deoxyribonucleotides (Elbashir SM et al., Genes Dev 2001 Jan 15, 15(2): 188-200). For further details, published documents such as US20060234970 are available. The present invention is not limited to these examples and any known chemical modifications may be employed for the double-stranded molecules of the present invention so long as the resulting molecule retains the ability to inhibit the expression of the target gene.

Furthermore, the double-stranded molecules of the invention may include both DNA and RNA, e.g., dsD/R-NA or shD/R-NA. Specifically, a hybrid polynucleotide of a DNA strand and an RNA strand or a DNA-RNA chimera polynucleotide shows increased stability. Mixing of DNA and RNA, i.e., a hybrid type double-stranded molecule consisting of a DNA strand (polynucleotide) and an RNA strand (polynucleotide), a chimera type double-stranded molecule including both DNA and RNA on any or both of the single strands (polynucleotides), or the like may be formed for enhancing stability of the double-stranded molecule. The hybrid of a DNA strand and an RNA strand may be the hybrid in which either the sense strand is DNA and the antisense strand is RNA, or the opposite so long as it has an activity to inhibit expression of the target gene when introduced into a cell expressing the gene. Preferably, the sense strand polynucleotide is DNA and the antisense strand polynucleotide is RNA. Also, the chimera type double-stranded molecule may be either where both of the sense and antisense strands are composed of DNA and RNA, or where any one of the sense and antisense strands is composed of DNA and RNA so long as it has an activity to inhibit expression of the target gene when introduced into a cell expressing the gene.

In order to enhance stability of the double-stranded molecule, the molecule preferably contains as much DNA as possible, whereas to induce inhibition of the target gene expression, the molecule is required to be RNA within a range to induce sufficient inhibition of the expression. As a preferred example of the chimera type double-stranded molecule, an upstream partial region (i.e., a region flanking to the target sequence or complementary sequence thereof within the sense or antisense strands) of the double-stranded molecule is RNA. Preferably, the upstream partial region indicates the 5' side (5'-end) of the sense strand and the 3' side (3'-end) of the antisense strand. The upstream partial region preferably is a domain consisting of 9 to 13 nucleotides counted from the terminus of the target sequence or complementary sequence thereto within the sense or antisense strands of the double-stranded molecules. Moreover, preferred examples of such chimera type double-stranded molecules include those having a strand length of 19 to 21 nucleotides in which at least the upstream half region (5' side region for the sense strand and 3' side region for the antisense strand) of the polynucleotide is RNA and the other half is DNA. In such a chimera type double-stranded molecule, the effect to inhibit expression of the target gene is much higher when the entire antisense strand is RNA (US20050004064).

In the present invention, the double-stranded molecule may form a hairpin, such as a short hairpin RNA (shRNA) and short hairpin consisting of DNA and RNA (shD/R-NA). The shRNA or shD/R-NA is a sequence of RNA or mixture of RNA and DNA making a tight hairpin turn that can be used to silence gene expression via RNA interference. The shRNA or shD/R-NA preferably includes the sense target sequence and the antisense target sequence on a single strand wherein the sequences are separated by a loop sequence. Generally, the hairpin structure is cleaved by the cellular machinery into dsRNA or dsD/R-NA, which is then bound to the RNA-induced silencing complex (RISC). This complex binds to and cleaves mRNAs which match the target sequence of the dsRNA or dsD/R-NA.

In another embodiment, halogenated RNAs, RNAs partially replaced with DNAs, or methylated RNAs can be used to confer RNAase resistance to the siRNA. Such nucleic acid derivatives that confer RNAase resistance are also included in the double-stranded RNA. In the present invention, the double stranded molecule may include a double stranded RNA constructed from ribonucleotides, modified ribonucleotides, or ribonucleotide derivatives.

An siRNA of the TTK or EGFR gene hybridizes to target RNA and thereby decreases or inhibits production of the polypeptides encoded by the TTK or EGFR gene by associating with the normally single-stranded mRNA transcript, thereby interfering with translation and thus, expression of the protein. In the context of the present invention, an siRNA is preferably less than 500, 200, 100, 50, or 25 nucleotides in length. More preferably an siRNA is 19-25 nucleotides in length. Exemplary nucleic acid sequence for the production of TTK or EGFR siRNA includes the sequences of nucleotides of SEQ ID NOs: 63 or 64 as the target sequence. In order to enhance the inhibition activity of the siRNA, one or more uridine ("u") nucleotides can be added to 3'end of the antisense strand of the target sequence. The number of "u's" to be added is at least 2, generally 2 to 10, preferably 2 to 5. The added "u's" form a single strand at the 3'end of the antisense strand of the siRNA.

An siRNA of the TTK or EGFR gene can be directly introduced into the cells in a form that is capable of binding to the mRNA transcripts. Alternatively, a DNA encoding the siRNA can be carried in a vector.

Vectors can be produced, for example, by cloning an TTK or EGFR gene target sequence into an expression vector having operatively-linked regulatory sequences flanking the sequence in a manner that allows for expression (by transcription of the DNA molecule) of both strands (Lee, N.S., et al., (2002) Nature Biotechnology 20: 500-5). An RNA molecule that is antisense to mRNA of the TTK or EGFR gene is transcribed by a first promoter (e.g., a promoter sequence 3' of the cloned DNA) and an RNA molecule that is the sense strand for the RNA of the TTK or EGFR gene is transcribed by a second promoter (e.g., a promoter sequence 5' of the cloned DNA). The sense and antisense strands hybridize in vivo to generate siRNA constructs for silencing of the TTK or EGFR gene. Alternatively, the two constructs can be utilized to create the sense and anti-sense strands of a siRNA construct. Cloned TTK or EGFR gene can encode a construct having secondary structure, e.g., hairpins, wherein a single transcript has both the sense and complementary antisense sequences from the target gene.

A loop sequence consisting of an arbitrary nucleotide sequence can be located between the sense and antisense sequence in order to form the hairpin loop structure. Thus, the present invention also provides siRNA having the general formula 5'-[A]-[B]-[A']-3',
wherein [A] is a ribonucleotide sequence corresponding to a sequence of the TTK or EGFR gene,
[B] is a ribonucleotide sequence composed of 3 to 23 nucleotides, and
[A'] is a ribonucleotide sequence having the complementary sequence of [A].

The region [A] hybridizes to [A'], and then a loop composed of region [B] is formed. The loop sequence can be 3 to 23 nucleotides in length. The loop sequence, for example, can be selected from the following sequences (found on the worldwide web at ambion.com/techlib/tb/tb_506.html). Furthermore, a loop sequence consisting of 23 nucleotides also provides active siRNA (Jacque, J. M., et al., (2002) Nature 418 : 435-8.).
CCC, CCACC or CCACACC: Jacque, J. M, et al., (2002) Nature, Vol. 418: 435-8.
UUCG: Lee, N.S., et al., (2002) Nature Biotechnology 20: 500-5.; Fruscoloni, P., et al., (2003) Proc. Natl. Acad. Sci. USA 100(4): 1633-44.
UUCAAGAGA: Dykxhoorn, D. M., et al., (2003) Nature Reviews Molecular Cell Biology 4: 457-67.

Accordingly, in some embodiments, the loop sequence can be selected from group consisting of, CCC, UUCG, CCACC, CCACACC, and UUCAAGAGA. A preferable loop sequence is UUCAAGAGA ("ttcaagaga" in DNA). Exemplary hairpin siRNA suitable for use in the context of the present invention include:
for TTK-siRNA; 
AACAAGAUGUGUUAAAGUGUUUU -[b]-
AAAACACUUUAACACAUCUUGUU(for target sequence of SEQ ID NO: 62)
for EGFR-siRNA;

AAGUAACAAGCUCACGCAGUUUU-[b]-
AAAACUGCGUGAGCUUGUUACUU(for target sequence of SEQ ID NO: 63) ,

The nucleotide sequence of suitable siRNAs can be designed using an siRNA design computer program available from the Ambion website on the worldwide web at ambion.com/techlib/misc/siRNA_finder.html. The computer program selects nucleotides sequences for siRNA synthesis based on the following protocol.

The regulatory sequences flanking the TTK or EGFR gene sequences can be identical or different, such that their expression can be modulated independently, or in a temporal or spatial manner. siRNAs are transcribed intracellularly by cloning the TTK or EGFR gene templates, respectively, into a vector containing, e.g., a RNA polymerase III transcription unit from the small nuclear RNA (snRNA) U6 or the human H1 RNA promoter. For introducing the vector into the cell, transfection-enhancing agent can be used. FuGENE (Roche diagnostics), Lipofectamine 2000 (Invitrogen), Oligofectamine (Invitrogen), and Nucleofector (Wako pure Chemical) are useful as the transfection-enhancing agent.

The siRNA of the present invention inhibits the expression of a polypeptide of the present invention and is thereby useful for suppressing the biological activity of a polypeptide of the invention. Also, expression-inhibitors, including the siRNA of the invention, are useful in the point that they can inhibit the biological activity of the polypeptide of the invention. Therefore, a composition composed of one or more siRNAs of the present invention is useful for treating a lung cancer. Alternatively, the present invention provides use of inhibitory nucleic acids including the siRNAs, or vector expressing the nucleic acids for manufacturing a pharmaceutical composition for treating or preventing lung cancer. Further, the present invention also provides such inhibitory nucleic acids including the siRNAs, or vector expressing the nucleic acids for treating or preventing lung cancer.

### Methods of treating or preventing lung cancer

Patients with tumors characterized as over-expressing TTK or EGFR are treated by administering siRNA of TTK or EGFR, respectively. siRNA therapy is used to inhibit expression of TTK or EGFR in patients suffering from or at risk of developing lung cancer. Such patients are identified by standard methods of the particular tumor type. Lung cancer cell is diagnosed for example, by CT, MRI, ERCP, MRCP, computer tomography, or ultrasound. Treatment is efficacious if the treatment leads to clinical benefit such as, a reduction in expression ofTTK or EGFR, or a decrease in size, prevalence, or metastatic potential of the tumor in the subject. When treatment is applied prophylactically, "efficacious" means that the treatment retards or prevents tumors from forming or prevents or alleviates a clinical symptom of the tumor. Efficaciousness is determined in association with any known method for diagnosing or treating the particular tumor type.

siRNA therapy is camped out by administering to a patient an siRNA by standard vectors encoding the siRNAs of the invention and/or gene delivery systems such as by delivering the synthetic siRNA molecules. Typically, synthetic siRNA molecules are chemically stabilized to prevent nuclease degradation in vivo. Methods for preparing chemically stabilized RNA molecules are well known in the art. Typically, such molecules comprise modified backbones and nucleotides to prevent the action of ribonucleases. Other modifications are also possible, for example, cholesterol-conjugated siRNAs have shown improved pharmacological properties. (Song et al. Nature Med. 9:347-351 (2003)).

Suitable gene delivery systems may include liposomes, receptor-mediated delivery systems, or viral vectors such as herpes viruses, retroviruses, adenoviruses and adeno-associated viruses, among others. A therapeutic nucleic acid composition is formulated in a pharmaceutically acceptable carrier. The therapeutic composition may also include a gene delivery system as described above. Pharmaceutically acceptable carriers are biologically compatible vehicles which are suitable for administration to an animal e.g., physiological saline. A therapeutically effective amount of a compound is an amount which is capable of producing a medically desirable result such as reduced production of a TTK or EGFR gene product, reduction of cell growth, e.g., proliferation, or a reduction in tumor growth in a treated animal.

Parenteral administration, such as intravenous, subcutaneous, intramuscular, and intraperitoneal delivery routes, may be used to deliver siRNA compositions of TTK or EGFR. For treatment of lung cancer, direct infusion into the tissue or near the site of cancer, is useful.

Dosages for any one patient depends upon many factors, including the patient's size, body surface area, age, the particular nucleic acid to be administered, sex, time and route of administration, general health, and other drugs being administered concurrently. Dosage for intravenous administration of nucleic acids is from approximately 106 to 1022 copies of the nucleic acid molecule.

The polynucleotides are administered by standard methods, such as by injection into the interstitial space of tissues such as muscles or skin, introduction into the circulation or into body cavities or by inhalation or insufflation. Polynucleotides are injected or otherwise delivered to the animal with a pharmaceutically acceptable liquid carrier, e.g., a liquid carrier, which is aqueous or partly aqueous. The polynucleotides are associated with a liposome (e.g., a cationic or anionic liposome). The polynucleotide includes genetic information necessary for expression by a target cell, such as a promoter.

### Pharmaceutical compositions comprising siRNA

Accordingly, the present invention includes medicaments and methods useful in either or both preventing and treating lung cancer. These medicaments and methods comprise a siRNA that inhibits expression of TKK (SEQ ID NO: 62) or EGFR (SEQ ID NO: 63) in an amount effective to achieve attenuation or arrest of disease cell proliferation. More specifically, in the context of the present invention, a therapeutically effective amount means an amount effective to prevent development of, or to alleviate existing symptoms of, the subject being treated.

Individuals to be treated with methods of the present invention include any individual afflicted with lung cancer. Such an individual can be, for example, a vertebrate such as a mammal, including a human, dog, cat, horse, cow, or goat; or any other animal, particularly a commercially important animal or a domesticated animal.

In the context of the present invention, suitable pharmaceutical formulations include those suitable for oral, rectal, nasal, topical (including buccal and sub-lingual), vaginal or parenteral (including intramuscular, sub-cutaneous and intravenous) administration, or for administration by inhalation or insufflation. Preferably, administration is intravenous. The formulations are optionally packaged in discrete dosage units.

Pharmaceutical formulations suitable for oral administration include capsules, cachets or tablets, each containing a predetermined amount of active ingredient. Suitable formulations also include powders, granules, solutions, suspensions and emulsions. The active ingredient is optionally administered as a bolus electuary or paste. Tablets and capsules for oral administration may contain conventional excipients" for example, fillers such as sugars, including lactose, sucrose, mannitol, or sorbitol, cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP), binding agents, lubricants, and/or wetting agents. If desired, disintegrating agents may be added, such as the cross-linked polyvinyl pyrrolidone, agar, or alginic acid or a salt thereof such as sodium alginate.

A tablet may be made by compression or molding, optionally with one or more formulational ingredients. Compressed tablets may be prepared by compressing in a suitable machine the active ingredients in a free-flowing form, such as a powder or granules, optionally mixed with a binder, lubricant, inert diluent, lubricating, surface active and/or dispersing agent. Molded tablets may be made by molding in a suitable machine a mixture of the powdered compound moistened with an inert liquid diluent. The tablets may be coated according to methods well known in the art. Oral fluid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), pH maintaining agents, and/or preservatives. The tablets may optionally be formulated so as to provide slow or controlled release of the active ingredient therein. A package of tablets may contain one tablet to be taken on each of the month.

Formulations suitable for parenteral administration include aqueous and non-aqueous sterile injection solutions, optionally contain anti-oxidants, buffers, bacteriostats and solutes which render the formulation isotonic with the blood of the intended recipient; as well as aqueous and non-aqueous sterile suspensions including suspending agents and/or thickening agents. The formulations may be presented in unit dose or multi-dose containers, for example as sealed ampoules and vials, and may be stored in a freeze-dried (lyophilized) condition requiring only the addition of the sterile liquid carrier, for example, saline, water-for-injection, immediately prior to use. Alternatively, the formulations may be presented for continuous infusion. Extemporaneous injection solutions and suspensions may be prepared from sterile powders, granules and tablets of the kind previously described.

Formulations suitable for rectal administration include suppositories with standard carriers such as cocoa butter or polyethylene glycol. Formulations suitable for topical administration in the mouth, for example buccally or sublingually, include lozenges, containing the active ingredient in a flavored base such as sucrose and acacia or tragacanth, and pastilles comprising the active ingredient in a base such as gelatin and glycerin or sucrose and acacia. For intra-nasal administration the compounds of the invention may be used as a liquid spray, a dispersible powder or in the form of drops. Drops may be formulated with an aqueous or non-aqueous base also comprising one or more dispersing agents, solubilizing agents and/or suspending agents.

For administration by inhalation the compounds can be conveniently delivered from an insufflator, nebulizer, pressurized packs or other convenient means of delivering an aerosol spray. Pressurized packs may comprise a suitable propellant such as dichlorodifluoromethane, trichlorofluoromethane, dichiorotetrafluoroethane, carbon dioxide or other suitable gas. In the case of a pressurized aerosol, the dosage unit may be determined by providing a valve to deliver a metered amount.

Alternatively, for administration by inhalation or insulation, the compounds may take the form of a dry powder composition, for example a powder mix of the compound and a suitable powder base such as lactose or starch. The powder composition may be presented in unit dosage form, for example, as capsules, cartridges, gelatin or blister packs from which the powder may be administered with the aid of an inhalator or insufflators.

Other formulations include implantable devices and adhesive patches; which release a therapeutic agent.

When desired, the above described formulations, adapted to give sustained release of the active ingredient, may be employed. The pharmaceutical compositions may also contain other active ingredients such as antimicrobial agents, immunosuppressants and/or preservatives.

It should be understood that in addition to the ingredients particularly mentioned above, the formulations of this invention may include other agents conventional in the art with regard to the type of formulation in question. For example, formulations suitable for oral administration may include flavoring agents.

It will be apparent to those persons skilled in the art that certain excipients may be more preferable depending upon, for instance, the route of administration, the concentration of test compound being administered, or whether the treatment uses a medicament that includes a protein, a nucleic acid encoding the test compound, or a cell capable of secreting a test compound as the active ingredient.

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes. Proper formulation is dependent upon the route of administration chosen.

### EXAMPLES

### EXAMPLES

### Example 1: Materials and Methods.

### (a) Lung Cancer Cell Lines and Tissue Samples. ,

Lung cancer cell lines were grown in monolayers in appropriate medium supplemented with 10% fetal calf serum (FCS) and were maintained at 37°C in an atmosphere of humidified air with 5% CO₂. Primary lung cancer and metastatic brain tumors derived from lung adenocarcinoma samples had been obtained earlier with informed consent (Kikuchi et al., Oncogene. 2003 Apr 10;22(14):2192-205., Int J Oncol. 2006 Apr;28(4):799-805., Taniwaki et al., Int J Oncol. 2006 Sep;29(3):567-75.). An independent set of 366 formalin-fixed primary tumors (234 adenocarcinomas (ADC), 104 squamous-cell carcinomas (SCC), and 28 large-cell carcinomas (LCC)) and adjacent normal lung tissue samples from patients undergoing surgery at Saitama Cancer Center (Saitama, Japan) were used in this invention. A total of 12 SCLC tissue samples were also obtained at Saitama Cancer Center.

### (b) Semi-quantitative RT-PCR analysis.

Total RNA was extracted from cultured cells and clinical tissues using Trizol reagent (Life Technologies, Inc.) according to the manufacturer's protocol. Extracted RNAs and normal human tissue poly(A) RNAs were treated with DNase I (Nippon Gene) and were reverse-transcribed using oligo(dT)20 primer and Superscript II reverse transcriptase (Invitrogen). Semiquantitative RT-PCR experiments were carried out with the following synthesized gene-specific primers or with beta-actin (ACTB)-specific primers as an internal control: TTK, 5'-TCTTGAATCCCTGTGGAAATC-3' (SEQ ID NO: 5) and 5'-TGCTATCCACCCACTATTCCA-3' (SEQ ID NO: 6); ACTB, 5'-GAGGTGATAGCATTGCTTTCG-3' (SEQ ID NO: 7) and 5'-CAAGTCAGTGTACAGGTAAGC-3' (SEQ ID NO: 8). PCR reactions were optimized for the number of cycles to ensure product intensity within the logarithmic phase of amplification.

### (c) Northern-blot Analysis.

Human multiple-tissue blots (BD Biosciences Clontech) were hybridized with a ³²P-labeled PCR product of TTK. The full-length cDNA of TTK was prepared by RT-PCR using primers. Prehybridization, hybridization, and washing were performed according to the supplier's recommendations. The blots were autoradiographed with intensifying screens at room temperature for 72 hours.

### (d) Western-blot Analysis

Cells were lysed with RIPA buffer [50 mM Tris-HCl (pH8.0), 150 mM NaCl, 1% NP-40, 0.5% deoxychorate-Na, 0.1% SDS] containing Protease Inhibitor Cocktail Set III (CALBIOCHEM) and phosphatase inhibitor (1 mM sodium fluoride, 1 mM sodium orthovanadate, 2 mM imidazole, 4 mM sodium tartrate). Cytoplasmic and nuclear extractions were isolated by NE-PER Nuclear and Cytoplasmic Extraction Reagents (PIERCE). Protein samples were separated by SDS-polyacrylamide gels and electroblotted onto Hybond-ECL nitrocellulose membranes (GE Healthcare Bio-sciences). Blots were incubated with a mouse monoclonal anti-Mpsl (TTK) antibody (Upstate), rabbit polyclonal anti-phospho-EGFR antibodies (Tyr-845, Tyr-992, Tyr-1045, Tyr-1068, Tyr-1148, and Tyr-1173; Cell Signaling Technology, Inc.), a rabbit polyclonal anti-EGFR antibody (Cell Signaling Technology, Inc.), a rabbit polyclonal anti-phospho-PLCgammal (Tyr-771) antibody (Cell Signaling Technology, Inc.), a rabbit polyclonal anti-PLCgammal antibody (Cell Signaling Technology, Inc.), a rabbit polyclonal anti-phospho- p44/42 MAPK (Thr202/Tyr204) antibody (Cell Signaling Technology, Inc.), a rabbit polyclonal anti-p44/42 MAPK antibody (Cell Signaling Technology, Inc.), a mouse monoclonal anti-Flag antibody (SIGMA) or a mouse monoclonal anti-beta-actin antibody (SIGMA). Anti-phospho-EGFR (Ser-967) antibodies were raised against Ser-967-phosphorylated synthetic peptides and purified with the phosphopeptide column. Antigen-antibody complexes were detected using secondary antibodies conjugated to horseradish peroxidase (GE Healthcare Bio-sciences). Protein bands were visualized by ECL Western Blotting Detection Reagents (GE Healthcare Bio-sciences).

### (e) Immunocytochemical analyses

Cultured cells were fixed with 4% paraformaldehyde solution for 15 min at 37°C, or 10% Trichloro acetic acid for 15 min on ice. Fixed cells were incubated in PBS(-) containing 0.1% Triton X-100 for 10 min and subsequently washed with PBS(-). Prior to the primary antibody reaction, fixed cells were covered with CAS-BLOCK (ZYMED Laboratories Inc.) for 10 min to block non-specific- antibody binding. Then the cells were incubated with a mouse monoclonal anti-Mps1 (TTK) antibody (Upstate) and a rabbit polyclonal anti-phospho-EGFR (Tyr-992) (Cell - Signaling Technology, Inc.), or a rabbit polyclonal anti-phospho-p44/42 MAPK (Thr202/Tyr204) antibody (Cell Signaling Technology, Inc.), or a mouse monoclonal anti-Flag antibody (SIGMA). Antibodies were stained with an anti-mouse secondary antibody conjugated to Alexa Fluor 488 (Molecular Probes) and an anti-rabbit secondary antibody conjugated to Alexa Fluor 594 (Molecular Probes). DNA was stained with 4',6-diamidino-2-phenylindole (DAPI). Images were viewed and assessed using a confocal microscope at wavelengths of 488, 594 nm (TCS SP2 AOBS: Leica Microsystems).

### (f) Immunohistochemistry and Tissue microarray

To investigate the presence of TTK or phospho-EGFR (Tyr-992) protein in clinical samples, the sections were stained using ENVISION+ Kit/horseradish peroxidase (HRP) (DakoCytomation). Briefly, anti-human TTK antibody (NOVUS Biologicals, Inc.) or anti-phospho-EGFR (Tyr-992) antibody (Cell Signaling Technology, Inc.) or anti-phospho-EGFR (Ser-967) antibody (described above), was added after blocking endogenous peroxidase and proteins, and the sections were incubated with HRP-labeled anti-rabbit IgG as the secondary antibody. Substrate-chromogen was added and the specimens were counterstained with hematoxylin.

The tumor tissue microarrays were constructed as published previously (Chin et al., Mol Pathol. 2003 Oct;56(5):275-9.; Callagy et al., Diagn Mol Pathol. 2003 Mar;12(1):27-34., J Pathol. 2005 Feb;205(3):388-96.). The tissue area for sampling was selected based on a visual alignment with the corresponding HE-stained section on a slide. Three, four, or five tissue cores (diameter 0.6 mm; height 3 - 4 mm) taken from the donor tumor blocks were placed into a recipient paraffin block using a tissue microarrayer (Beecher Instruments). A core of normal tissue was punched from each case. 5-micro-m sections of the resulting microarray block were used for immunohistochemical analysis. Positivity of TTK or phospho-EGFR (Tyr-992) or phospho-EGFR (Ser-967) protein was assessed semiquantitatively by three independent investigators without prior knowledge of the clinicopathological data, each of who recorded staining intensity as absent (score3211d as 0), weak (1+) or strongly positive (2+). Cases were accepted only as strongly positive (2+) if all reviewers defined them as such.

### (g) Statistical analysis

Using contingency tables, attempts were made to correlate clinicopathological variables such as age, gender, histology, smoking history, tumor size (pT), and lymph-node metastasis (pN) with the positivity of TTK and/or phospho-EGFR (Tyr-992) and/or phospho-EGFR (Ser-967) determined by tissue-microarray analysis. Tumor-specific survival curves were calculated from the date of surgery to the time of death related to NSCLC, or to the last follow-up observation. Kaplan-Meier curves were calculated for each relevant variable and for TTK and/or phospho-EGFR (Tyr-992) and/or phospho-EGFR (Ser-967) expression; differences in survival times among patient subgroups were analyzed using the log-rank test. Univariate analyses were performed with the Cox proportional-hazard regression model to determine associations between clinicopathological variables and cancer-related mortality.

### (h) RNA interference assay

### (i) vector based assay

A vector-based RNA interference (RNAi) system, psiH1BX3.0, had previously established to direct the synthesis of siRNAs in mammalian cells (Suzuki et al., 2003, 2005; Kato et al, 2005; Furukawa et al, 2005). 10 micro-g of siRNA-expression vector were transfected into NSCLC cell lines LC319 and A549 both of which over-expressed TTK endogenously, using 30 micro-1 of Lipofectamine 2000 (Invitrogen). The transfected cells were cultured for five days in the presence of appropriate concentrations of geneticin (G418), after which cell numbers and viability were measured by Giemsa staining and triplicate MTT assays. The target sequences of the synthetic oligonucleotides for RNAi were as follows: control 1 (Luciferase: Photinus pyralis luciferase gene), 5'-CGTACGCGGAATACTTCGA-3' (SEQ ID NO: 9); control 2 (Scramble: chloroplast Euglena gracilis gene coding for 5S and 16S rRNAs), 5'-GCGCGCTTTGTAGGATTCG-3' (SEQ ID NO: 10); siRNA-TTK (si-TTK-1), 5'-ACAGTGTTCCGCTAAGTGA-3' (SEQ ID NO: 11); siRNA-TTK (si-TTK-2), 5'-ATCACGGACCAGTACATCT-3'(SEQ ID NO: 12).

To validate our RNAi system, individual control siRNAs (Luciferase and Scramble) were initially confirmed using semiquantitative RT-PCR to decrease expression of the corresponding target genes that had been transiently transfected into COS-7 cells. Down-regulation ofTTK expression by si-TTK, but not by controls, was confirmed with semiquantitative RT-PCR in the cell lines used for this assay.

### (ii) oligo based assay

The oligo-siRNAs (Dharmacon, Inc., Lafayette, CO) (600 pM) were transfected into appropriate lung-cancer cell lines using 30 macro-1 of Lipofectamine 2000 (Invitrogen, Carlsbad, CA) following the manufacturer's protocol. The ribonucleotide sequences corresponding to following sequences were used as the oligo-siRNA.
RNAi-TTK (oligo):CAAGATGTGTTAAAGTGTTTT (SEQ ID NO: 62); and
RNAi-EGFR (oligo):GTAACAAGCTCACGCAGTTTT (SEQ ID NO: 63).

### (i) Cell Growth Assay

The growth effect of TTK on mammalian cells was also examined using COS-7 and NIH-3T3 cells transiently transfected with plasmids expressing TTK or mock plasmids. The cells were cultured in DMEM containing 10% FCS and geneticin for 14 days, and MTT assay were performed.

### (j) Dephosphorylation analysis

To obtain mitotically arrested cells, cultured cells were treated with colcemid (WAKO) for 24 hours before extracts were prepared for western-blot analysis. For phosphatase treatment, cell extract were incubated with lambda-phoshatase (New England Biolabs) in phosphatase buffer or buffer alone for 1 hour at 37°C, then analyzed by immunoblotting.

### (k) Preparation of recombinant TTK

The full-length cDNA of TTK was subcloned into pFastBac HT vector. The recombinant shuttle vector was transformed into the baculovirus genome (bacmid DNA) in DH10Bac competent cells (Invitrogen) by using Tn7 site-specific transposition according to the minufacture's instructions for the Bac-to-Bac expression system (Invitrogen). The recombinant bacmid DNA was isolated, purified, transfected into Sf9 cells (Invitrogen) (recombinant baculovirus), and used for next infection. Log-phase Sf-9 cells were infected with recombinant baculovirus at a multiplicity of infection (MOI) of 1, and then infected Sf-9 cells were grown in suspension at 27°C for 72 hours. Extracts of Sf-9 cells were collected and the His fusion proteins were purified using HiTrap HP column (GE Healthcare Bio-sciences) using standard protocols. To construct the catalytically inactive TTK (TTK-KD), a point mutation (Ala) was introduced at codon 647 (Asp). TTK-KD was cloned into pGEX6T vector (GE Healthcare Bio-sciences). GST fusion protein was expressed in E. coli stain BL21 and purified by Glutathione Sepharose 4B (GE Healthcare Bio-sciences) using standard protocols.

### (I) In vitro kinase assay

To investigate phosphorylation of proteins by TTK, purified recombinant His-tagged TTK was incubated with whole extracts prepared from cell lines or purified GST-tagged proteins containing various cytoplasmic region of EGFR in kinase assay buffer (50 mM Tris, pH7.4, 10 mM MgCl2, 2 mM dithiothreitol, 1 mM NaF, 0.2 mM ATP) for 60 min at 30°C. The reactions were stopped by addition of Laemmli sample buffer and heating at 95°C for 5 min. Proteins were resolved by SDS-PAGE and then western-blot or ³²P incorporation analysis.

### (m) Tumor growth in nude mouse xenograft model

TTK transfectants were harvested, and 5x10⁶ cells were suspended in growth factor-reduced, phenol red-free Mtrigel (BD Bioscience) and injected s.c. into the right dorsum of 6-weeks-old nu/nu BalbC female mice (Charles River Laboratories). Tumor size was measured using a ruler, and tumor volume was calculated using the formula V = (W/2)² x L, where W is the distance across and L is the measurement lengthwise of the tumor.

### (n) TTK-expressing stable transfectants

TTK-expressing stable transfectants were established according to a standard protocol. Using FuGENE 6 Transfection Reagent (Roche Diagnostics), we transfected HEK293 and NIH3T3 cells with plasmids expressing TTK (pCAGGS-n3FH-TTK) or mock plasmids (pCAGGS-n3FH). Transfected cells were cultured in DMEM containing 10% FCS and G418, then individual colonies were trypsinized and screened for stable transfectants by a limiting-dilution assay. Expression of TTK was determined in each clone by RT-PCR, western blotting, and immunostaining. HEK293 or NIH3T3 transfectants that stably expressed TTK were seeded onto 6-well plates (5 X 10⁴ cells/well), and maintained in medium containing 10% FCS and 0.4 mg/ml G418 for 7 days. At each time point, cell proliferation was evaluated by the MTT assay using Cell Counting Kits (WAKO). All experiments were done in triplicate.

### (o) TTK mutation analysis

Total RNA was extracted from NSCLC cell lines using RNeasy Mini Kit (Qiagen) according to the manufacturer's protocol. Extracted RNAs were treated with DNase I (Nippon Gene) and were reverse-transcribed using oligo (dT) primer and Superscript II reverse transcriptase (Invitrogen). Amplification of TTK was carried out with the synthesized TTK specific primers,
5'-GTGTTTGCGGAAAGGAGTTT-3' (SEQ ID NO: 13),
5'-CAACCAGTCCTCTGGGTTGT-3' (SEQ ID NO: 14);
5'-AACTCGGGAACTGTTAACCAAA-3' (SEQ ID NO: 15),
5'-GTGCATCATCTGGCTCTTGA-3' (SEQ ID NO: 16);
5'-TGTTCCGCTAAGTGATGCTC-3' (SEQ ID NO: 17),
5'-GCAAATTTCTTGCAGTTTGCTC-3' (SEQ ID NO: 18);
5'-TCAAGAGCCAGATGATGCAC-3' (SEQ ID NO: 19),
5'-TCTTTTCCTCCTCTGAAAGCA-3' (SEQ ID NO: 20);
S'-AAGCTGTAGAACGTGGAGCAG-3' (SEQ ID NO: 21),
5'-CATCTTGTGGTGGCATGTTC-3' (SEQ ID NO: 22);
5'-CGGTTCACTTGGGCATTTAC-3' (SEQ ID NO: 23),
5'-CCAAATCTCGGCATTCTGAT-3' (SEQ ID NO: 24);
5'-AGCCCAGATTGTGATGTGAA-3' (SEQ ID NO: 25),
5'-TTGATTCCGTTTTATTCTTCAGG-3' (SEQ ID NO: 26);
5'-TCAAGGAACCTCTGGTGTCA-3' (SEQ ID NO: 27),
S'-GACAGGTTGCTCAAAAGTGG-3' (SEQ ID NO: 28);
5'-ACTGGCAGATTCCGGAGTTA-3 (SEQ ID NO: 29)',
5'-CAACTGACAAGCAGGTGGAA-3' (SEQ ID NO: 30);
5'-GACACCAAGCAGCAATACCTTGG-3' (SEQ ID NO: 31),
5'-AACACCTGAAATACCTTGCTTGAAC-3' (SEQ ID NO: 32);
5'-ATGAATGCATTTCGGTTAAAGG-3' (SEQ ID NO: 33),
5'-TTTCCACACTCCATTACCATG-3' (SEQ ID NO: 34);
5'-ACAGTGATAAGATCATCCGAC-3' (SEQ ID NO: 35),
5'-ACACTTGTTGTATCTGGTTGC-3' (SEQ ID NO: 36);
5'-T'ITCTGATAGTTGATGGAATGC-3' (SEQ ID NO: 37),
5'-GAAATCTGATTAATTATCTGCTG-3' (SEQ ID NO: 38);
5'-TGATGTTTGGTCCTTAGGATG-3' (SEQ ID NO: 39),
5'-ATTTCTTCAGTGGTTCCCTTG-3' (SEQ ID NO: 40) and
5'-AGCTCCTGGCTCATCCCTAT-3' (SEQ ID NO: 41),
5'-TGCTATCCACCCACTATTCCA-3' (SEQ ID NO: 6).
Then, sequencing analysis of the PCR products were performed using an ABI Prism 3700 DNA sequencer (Applied Biosystems).

### (p) Mangel invasion assay

Using FuGENE 6 Transfection Reagent (Roche Diagnostics) according to the manufacturer's instructions, NIH-3T3 or COS-7 cells were transfected with plasmids expressing TTK (pCAGGS-n3FH-TTK), TTK-KD (D647A), mutant TTK (originated in RERF-LC-AI cells) or mock plasmids (pCAGGS-n3FH). Transfected cells were harvested and suspended in DMEM without FCS. Before the cell suspension was prepared, the dried layer of Matrigel matrix (Becton Dickinson Labware) was rehydrated with DMEM for 2 hours at room temperature. Then, DMEM containing 10% FCS was added to each lower chamber of 24-well Matrigel invasion chambers and cell suspension was added to each insert of the upper chamber. The plates of inserts were incubated for 22 hours at 37°C. After incubation, cells invading through the Matrigel-coated inserts were fixed and stained by Giemsa.

### (q) Synthesized cell-permeable peptide

17 ∼ 31 amino acid peptide sequences corresponding to a part of EGFR protein that contained possible TTK-interacting region were covalently linked at its NH2 terminus to a membrane transducing 11 poly-arginine sequence (Hayama, S, et al. Cancer Res. 66, 10339-48 (2006), Futaki S, et al. J Biol Chem. 276, 5836-5840 (2001)). Three cell-permeable peptides were synthesized:
11R-EGFR889-907: RRRRRRRRRRR-GGG-KPYDGIPASEISSILEKGE;
11R-EGFR899-917: RRRRRRRRRRR-GGG-ISSILEKGERLPQPPICTI(SEQ ID NO: 44);
11R-EGFR918-936: RRRRRRRRRRR-GGG-DVYMIMVKCWMIDADSRPK(SEQ ID NO: 45);
11R-EGFR937-955: RRRRRRRRRRR-GGG-FRELIIEFSKMARDPQRYL(SEQ ID NO: 46);
11R-EGFR958-976: RRRRRRRRRRR-GGG-QGDERMHLPSPTDSNFYRA;
11R-EGFR983-1001: RRRRRRRRRRR-GGG-MDDVVDADEYLIPQQGFFS; and
11R-EGFR965-994: RRRRRRRRRRR-GGG-LPSPTDSNFYRALMDEEDMDDWDADEYLI.
Scramble peptides derived from the most effective 11R-EGFR937-955 peptides were synthesized as a control: Scramble, RRRRRRRRRRR-GGG-EFMAELLRYFRPQSKRDII. Peptides were purified by preparative reverse-phase high-pressure liquid chromatography. A549. cells were incubated with the 11R peptides at the concentration of 2.5, 5, and 7.5 micro-mol/L for 5 days. The medium was exchanged at every 48 hours at the appropriate concentrations of each peptide and the viability of cells was evaluated by MTT assay at 5 days after the treatment.

### Examples 2: TTK expression in lung tumors and normal tissues

To search for novel molecular targets for development of therapeutic agents and/or diagnostic markers for lung cancer, genes that showed 5-fold higher expression in more than 50% of lung cancers analyzed by cDNA microarrays were screened first. Among 27,648 genes screened, the gene encoding TTK protein kinase was identified as frequently over-expressed in various types of lung cancers. This expression level was confirmed in 11 of 14 additional NSCLC cases (5 of 7 adenocarcinoma (ADCs) and in 6 of 7 squamous-cell carcinomas (SCCs) (**Figure 1a**). In addition, up-regulation of TTK was observed in all of 23 lung-cancer cell lines. (**Figure. 1b**). Furthermore, the expression of TTK in lung tumors was confirmed by examining expression of endogenous TTK protein on western blot analyses using anti-TTK antibody in all of 7 lung-cancer cell lines (**Figure 1c**). Northern blotting with TTK cDNA as a probe identified a 3.0-kb transcript specifically in testis among 24 normal human tissues examined (data not shown). The subcellular localization of TTK in NSCLC cell lines, A549 and LC319, were examined by immunocytochemical and western-blot analyses with anti-TTK antibody, and it was found to be mainly localized in the cytoplasm and nucleus (**Figures 1e and 1f**).

### Example 3: Association of TTK over-expression with poor prognosis

To verify the biological and clinicopathological significance of TTK, TTK protein expression was examined in clinical NSCLCs by means of tissue microarrays containing NSCLC tissues from 366 patients as well as SCLC tissues from 12 patients. Positive staining for TTK was observed in 68.6% of surgically-resected NSCLCs (251/366) and in 66.7% of SCLCs (8/12), while no staining was observed in any of normal lung tissues examined (**Figure 2a**). The correlation of its positive staining with various clinicopathological parameters was then examined in 366 NSCLC patients. The TTK expression level on the tissue array was classified as ranging from absent (scored as 0) to weak/strong positive (scored as 1+ ∼ 2+) (**Figure 2a****;** see Example 1). Of the 366 NSCLC cases examined, TTK was strongly stained in 135 (36.9%, score 2+), weakly stained in 116 (31.7%; score 1+), and not stained in 115 cases (31.4%; score 0). Gender (higher in male; P = 0.0006 by Fisher's exact test), histological classification (higher in SCC; P = 0.0206 by χ2-test), pT stage (higher in T2, T3, T4; P < 0.0001 by χ2-test), and-pN stage (higher in N1, N2; P = 0.0006 by χ2-test) were significantly associated with the strong TTK positivity (score 2+) (**Table 1a**). Kaplan-Meier method indicated significant association between strong positive staining of TTK in the NSCLCs and tumor-specific survival (P < 0.0001 by the Log-rank test) (**Figure 2b**). By univariate analysis, age (≥ 65 vs < 65), gender (male vs female), histological classification (non-ADC versus ADC), pT stage (T2, T3, T4 vs T1), pN stage (N1, N2 vs N0), and strong TTK positivity (score 2+ vs 1+, 0) were all significantly related to poor tumor-specific survival among NSCLC patients (**Table 1b**, upper). In multivariate analysis of the prognostic factors, age, pT stage, pN stage, and strong TTK positivity were indicated to be an independent prognostic factor (**Table 1b**, lower).

**Table 1a. Association between TTK-positivity in NSCLC tissues and patients' characteristics (n=366)**

| | | Total | TTK strong positive | TTK weak positive | TTK absent | P-value strong/weak vs absent |
|---|---|---|---|---|---|---|
| | | n = 366 | n = 135 | n= 116 | n = 115 | |
| Gender | | | | | | |
| | Male | 253 | 108 | 79 | 66 | 0.0006* |
| | Female | 113 | 27 | 37 | 49 | |
| Age (years) | | | | | | |
| | < 65 | 180 | 66 | 58 | 56 | N.S. |
| | ≧ 65 | 186 | 69 | 58 | 59 | |
| Histological | | | | | | |
| | type | | | | | |
| | ADC | 234 | 78 | 73 | 83 | 0.0206*,** |
| | SCC | 104 | 49 | 31 | 24 | |
| | LCC | 28 | 8 | 12 | 8 | |
| pT factor | | | | | | |
| | T1 | 124 | 24 | 37 | 63 | <0.0001* |
| | T2+T3+T4 | 242 | 111 | 79 | 52 | |
| pN factor | | | | | | |
| | N0 | 226 | 68 | 70 | 88 | 0.0006* |
| | N1+N2 | 140 | 67 | 46 | 27 | |

| | | | | | | |
|---|---|---|---|---|---|---|
| ADC, adenocarcinoma; SCC, squamous-cell carcinoma LCC, large-cell carcinoma P < 9.05 (Chi-square test) NS, no significance SCC vs others (ADC + LCC) | | | | | | |

**Table 1b. Cox's proportional hazards model analysis of prognostic factors in patients with NSCLC**

| Variables | Hazards ratio | 95% CI | Unfavorable/Favorable | P-value |
|---|---|---|---|---|
| Univariate analysis | | | | |
| TTK | 2.306 | 1.719- | Strong(+)/Weak(+)or(-) | <0.0001* |
| | | 3.094 | | |
| Age (years) | 1.448 | 1.076- | 65≧ / <65 | 0.0144* |
| | | 1.947 | | |
| Gender | 1.664 | 1.184- | Male/Female | 0.0033* |
| | | 2.338 | | |
| Histological type | 1.236 | 0.906- | SCC1/others | NS |
| | | 1.686 | | |
| pT factor | 2.693 | 1.861- | T2+T3+T4/T1 | <0.0001 * |
| | | 3.898 | | |
| pN factor | 2.536 | 1.890- | N1+N2/N0 | <0.0001* |
| | | 3.403 | | |

| Variables | Hazards ratio | 95% CI | Unfavorable/Favorable | P-value |
|---|---|---|---|---|
| Multivariate | | | | |
| analysis | | | | |
| TTK | 1.692 | 1-247- | Strong(+)/Weak(+)or(-) | 0.0007* |
| | | 2.296 | | |
| Age (years) | 1.606 | 1.189- | 65≧/<65 | 0.0020* |
| | | 2.169 | | |
| Gender | 1.322 | 0.932- | Male/Female | NS |
| | | 1.875 | | |
| pT factor | 1.956 | 1.331- | T2+T3+T4/T1 | 0.0006* |
| | | 2.873 | | |
| pN factor | 2.286 | 1.686- | N1+N2/NO | <0.0001* |
| | | 3.100 | | |

| | | | | |
|---|---|---|---|---|
| 1SCC, squamous-cell carcinoma * P < 0.05 NS, no significance | | | | |

### Example 4: Growth-suppression of lung cancer cells by siRNA against TTK

To assess whether TTK is essential for growth/survival of lung-cancer cells, plasmids were constructed to express siRNA against TTK (si-TTK-1 and -2) as well as two control plasmids (siRNAs for Luciferase (LUC), or Scramble (SCR)), and transfected each of them into LC319 and A549 cells (Representative data of LC319 was shown in **Figure 3a**)**.** The amount of TTK transcript in the two NSCLC cell lines transfected with si-TTK-1 was significantly decreased in comparison with the two control siRNAs. (**Figure 3a****, left upper panels**). Cell viability and colony numbers of the cells transfected with si-TTK-1, measured by MTT and colony-formation assays were reduced significantly in comparison with those with the two control siRNAs (**Figure 3a****, left lower and right panels**). si-TTK-2 revealed weaker reduction of the TTK expression and modest growth-suppressive effect. The results suggested the growth-promoting effect of TTK on NSCLC cells.

### Example 5: Growth promoting effect by TTK

To determine whether TTK plays an important role in the growth of cells, HEK293-derived transfectants that stably expressed TTK were established. Growth of HEK293 cells expressing exogenous TTK was promoted at a significant degree in accordance with the expression level of TTK compared their growth with control cells transfected with mock vector (**Figure 3b** and **3c**). The TTK-transfected HEK293 cells transplanted to subcutaneous of mice also exhibited higher growth rate compared to the control cells (**Figure 3d**). The results suggested the growth-promoting effect of TTK on NSCLC cells.

### Example 6: Activation of Cellular Invasive Activity by TTK

As the immunohistochemical analysis on tissue micro array had indicated that lung-cancer patients with TTK strong-positive tumors showed shorter cancer-specific survival period than patients whose tumors were negative for TTK, the present inventers performed Matrigel invasion assays to determine whether TTK might play a role in cellular invasive ability. Invasion of in NIH-3T3 cells transfected with TTK-expression vector through Matrigel was significantly enhanced, compared to the control cells transfected with mock or TTK-KD vector, suggesting that TTK could also contribute to the highly malignant phenotype of lung-cancer cells (**Figure 3e**).

### Example 7: Identification of EGFR as a novel substrate-protein for TTK

To elucidate the function of TTK kinase in carcinogenesis, the present inventers attempted to identify substrate proteins that would be phosphorylated by TTK and activate cell-proliferation signaling. Immunoblot-screening of kinase substrates for TTK was performed by using cell lysates of COS-7 cells transfected with TTK-expression vector and a series of antibodies specific for phospho-proteins related to cancer-cell signaling (see Example 1). A total of 31 phosphoproteins (**Table 2**) were screened. It was found that Tyr-992 of EGFR was significantly phosphorylated in the cells transfected with the TTK-expression vector, compared with those with mock vector (**Figure 4a**). In this screening, a total of seven phospho-specific antibodies were examined for EGFR that recognized various phospho-Tyr residues within the cytoplasmic domain of the EGFR (Tyr-845, Tyr-992, Tyr-1045, Tyr-1068, Tyr-1148, and Tyr-1173) (Amos S, et al., J Biol Chem. 2005 Mar 4;280(9):7729-38. Epub 2004 Dec 23; Biscardi JS, et al., J Biol Chem. 1999 Mar 19;274(12):8335-43; Honegger A, et al., EMBO J. 1988 Oct;7(10):3045-52; Sorkin A, et al., J Biol Chem. 1991 May 5;266(13):8355-62; Wang XQ, et al., J Biol Chem. 2003 Dec 5;278(49):48770-8. Epub 2003 Sep 25.) as well as one recognizing phosphor Ser-1046/1047 (Countaway JL, et al., J Biol Chem. 1992 Jan 15;267(2):1129-40; Gamou S & Shimizu N. J Cell Physiol. 1994 Jan;158(1):151-9.), the only Tyr-992 phosphorylation was found (data not shown). To confirm specific phosphorylation of Tyr-992 by the TTK kinase, the catalytically inactive TTK-KD-expression vector was transfected to COS-7 cells, and no enhancement of phosphorylation of EGFR at Tyr-992 was detected (**Figure 4a**). These data suggest that TTK kinase activity could very selectively regulate Tyr-992 phosphorylation in the EGFR signaling.

**Table 2. List of phospho-specific antibodies used for immunoblot-screening of substrate-protein(s) for TTK kinase.**

| | Antibody Name | Resource | Catalog number |
|---|---|---|---|
| 1 | Phospho-Akt (Ser473) | Cell Signaling Technology, Inc. | 9275 |
| 2 | Phospho-Akt (Thr309) | Cell Signaling Technology, Inc. | 9271 |
| 3 | Phospho-ATM (Ser1981) | Cell Signaling Technology, Inc. | 4526 |
| 4 | p-Bad (Ser136) | Santa Cruz Biotechnology, Inc. | sc-7999 |
| 5 | p-Bc1-2(Ser 87) | Santa Cruz Biotechnology, Inc. | sc-16323 |
| 6 | Phospho-cdc25C (Ser216) | Cell Signaling Technology, Inc. | 9528 |
| 7 | Phospho-Chk2 (Thr68) | Cell Signaling Technology, Inc. | 2661 |
| 8 | Phospho-EGF Receptor (Tyr845) | Cell Signaling Technology, Inc. | 2231 |
| 9 | Phospho-EGF Receptor (Tyr992) Phospho-EGF Receptor | Cell Signaling Technology, Inc. | 2235 |
| 10 | (Tyr1045) Phospho-EGF Receptor | Cell Signaling Technology, Inc. | 2237 |
| 11 | (Ser1046/1047) Phospho-EGF Receptor | Cell Signaling Technology, Inc. | 2238 |
| 12 | (Tyr1068) Phospho-EGF Receptor | Cell Signaling Technology, Inc. | 2234 |
| 13 | (Tyr1148) | Cell Signaling Technology, Inc. | 4404 |
| 14 | phospho-EGFR (Ty1173) | Upstate | 05-483 |
| 15 | phospho-Histone H2A.X (Ser139) | Upstate | 05-636 |
| 16 | p-IkappaB-alpha | Santa Cruz Biotechnology, Inc. | sc-8404 |
| 17 | p-IKK alpha/beta (Thr 23) | Santa Cruz Biotechnology, Inc. | sc-21660 |
| 18 | p-NIK (Thr 559)-R | Santa Cruz Biotechnology, Inc. | sc-12957 |
| 19 | Phospho-NPM (Thr199) | Cell Signaling Technology, Inc. | 3541 |
| 20 | Phospho-p53 (Ser15) | Cell Signaling Technology, Inc. | 9284 |
| 21 | Phospho-p53 (Ser20) | Cell Signaling Technology, Inc. | 9287 |
| 22 | Phospho-p53 (Ser46) | Cell Signaling Technology, Inc. | 2521 |
| 23 | p-Rb (Ser 249/Thr 252) | Santa Cruz Biotechnology, Inc. | sc-16671 |
| 24 | p-Rb (Ser 807/811) | Santa Cruz Biotechnology, Inc. | sc-16670-R |
| 25 | p-Rb (Thr 821/826) | Santa Cruz Biotechnology, Inc. | sc-16669 |
| 26 | p-Smad2/3 (Ser 433/435) | Santa Cruz Biotechnology, Inc. | sc-11769 |
| 27 | p-Smad1 (Ser 463/Ser 465) | Santa Cruz Biotechnology, Inc. | sc-12353 |
| 28 | Phospho-Stat1 (Tyr701) | Cell Signaling Technology, Inc. | 9171 |
| 29 | Phospho-Stat3 (Tyr705) | Cell Signaling Technology, Inc. | 9131 |
| 30 | Phospho-Stat3 (Ser727) | Cell Signaling Technology, Inc. | 9134 |
| 31 | Phospho-Stat5 (Tyr694) | Cell Signaling Technology, Inc. | 9351 |

The endogenous association between TTK and EGFR in lung cancer cells was also investigated by immunoprecipitation experiment using extracts from A549 cells, and the interaction between these two proteins regardless the EGF-stimulation was detected (**Figure 4b**). To further examine the relevance of TTK to EGFR signaling, TTK or EGFR function in A549 cells were suppressed and cell morphology was microscopically observed. A549 cells treated with EGFR tyrosine kinase inhibitor, AG1478 (tryphostin 4-(3-chloroanilino)-6,7-dimethoxyquinazoline), as well as the cells after transfection of RNAi-TTK (oligo) or RNAi-EGFR (oligo) became much larger with multiple nuclei (**Figure 4c**). These results support that TTK was involved in EGF-independent intracellular EGFR activation signals.

To further confirm specific phosphorylation of EGFR Tyr-992 by TTK, in vitro kinase assays were performed by incubating purified His-tagged TTK with whole cell extracts prepared from COS-7 cells. To investigate individual phosphorylation sites, the phospho-specific antibodies for EGFR (Tyr-845, Tyr-992, Tyr-1045, Tyr-1068, Tyr-1148, and Tyr-1173) corresponding to various phospho-Tyr residues within the cytoplasmic domain of the EGFR were applied. Western-blot analyses revealed phosphorylation of EGFR Tyr-992 by the recombinant TTK in a dose dependent manner, while phosphorylation of other Tyr residues in EGFR was not detected (**Figure 4d****, left panels**). On the other hand, these all Tyr residues were phosphorylated by stimulation of EGFR with its ligand EGF on COS-7 cells, suggesting that each phospho-EGFR specific antibody could precisely recognize each phosphorylation site (**Figure 4d****, right panels**). The evidence that EGF stimulation on COS-7 cells induces phosphorylation of all of the six Tyr residues further supports the specific kinase activity of TTK to Tyr-992 of EGFR.

The EGFR tyrosine phosphorylation sites were further evaluated by TTK using GST-tagged proteins containing various cytoplasmic region of EGFR as substrates (EGFR-DEL1, -DEL2, and -DEL3; **Figure 4e**). GST-tagged EGFR proteins were produced in E. coli, and subjected to in vitro kinase assays with purified recombinant TTK and subsequent western-blot analyses with anti--phospho-Tyr992 EGFR antibodies. Phosphorylation of EGFR Tyr-992 in GST-tagged EGFR-DEL2 protein (amino acid 889-1045) was promoted in a TTK-dose dependent manner, while other tyrosine residues in GST-tagged EGFR-DEL1 (amino acid 692-891), EGFR-DEL2 and EGFR-DEL3 (amino acid 1046-1186) proteins were not phosphorylated at all (**Figure 4f**). In vitro kinase assays using catalytically-active recombinant GST-tagged human EGFR (active-rhEGFR; Upstate) as substrates and subsequent western-blot analyses with anti-phospho-tyrosine antibodies demonstrated that the active-rhEGFR underwent autophosphorylation on tyrosine in the presence of ATP (**Figure 4g****, lane 2**). This phosphorylation was inhibited by EGFR tyrosine kinase inhibitor, AG1478 (tryphostin 4-(3-chloroanilino)-6,7-dimethoxyquinazoline) in a dose dependent manner (data not shown). However, as shown in **Figure 4g****, lane 4**, the phosphorylation of active-rhEGFR by TTK was not inhibited by AG1478. The result further confirmed the direct phosphorylation of EGFR by TTK.

Immunohistochemical analysis was further performed with anti-phospho-EGFR (Tyr-992) antibody using tissue microarrays composed of 366 NSCLC and 12 SCLC tissues. Of the 366 NSCLC cases, 123 (33.6%) revealed strong phospho-EGFR (Tyr-992) staining (score 2+), 121 (33.1%) were stained weakly (score 1+), and no staining (score 0) was observed in 122 cases (33.3%), while, no staining for phospho-EGFR (Tyr-992) was observed in any of normal lung tissues examined (**Figure 4h**). 3 of 12 SCLC (25%) were positive for phospho-EGFR (Tyr-992). 203 of the 366 tumors positive (scored as 1+ ∼ 2+) for both TTK and phospho-EGFR (Tyr-992), and 74 were negative for the both proteins. 48 of the 366 cases were positive for only TTK and 41 were positive for only phospho-EGFR (Tyr-992). The fact that the pattern af phvspha-EGFR (Tyr-992) positivity was significantly concordant with TTK positivity in these tumors (χ2 = 72.585; P < 0.0001) independently confirmed the results obtained by in vitro assays. It was found that strong expression ofphaspho-EGFR (Tyr-992) (score 2+) in NSCLCs was significantly associated with Gender (higher in male; P = 0.0086 by Fisher's exact test), histological classification (higher in SCC; P = 0.0483 by χ2-test), pT stage (higher'in T2, T3, T4; P = 0.0006 by χ2-test), pN stage (higher in N1, N2; P < 0001 by χ2-test), and tumor-specific survival (P < 0.0001 by the Log-rank test) (**Table 3a;** **Figure 4i**). In univariate and subsequent multivariate analyses of the prognostic factors, age, pT stage, pN stage, and strong phospho-EGFR (Tyr-992) positivity were indicated to be an independent prognostic factor (**Table 3b**, upper and lower). NSCLC patients whose tumors expressed neither TTK nor phospho-EGFR (Tyr-992) could receive the best survival benefit, while patients with strong positive values for both markers suffered the shortest tumor-specific survival (P < 0.0001 by the Log-rank test; (**Figure 4j**).

**Table 3a. Association between phosphoEGFR-positivity in NSCLC tissues and patients' characteristics (n=366)**

| | | Total | PhosphoEGF R strong positive | PhosphoEGFR weak positive | PhosphoEGF R absent | P-value strong/weak vs absent |
|---|---|---|---|---|---|---|
| | | n= 366 | n = J.23 | n = 121 | 122 | |
| Gender | | | | | | |
| | Male | 253 | 96 | 76 | 81 | 0.0086* |
| | Female | 113 | 27 | 45 | 41 | |
| Age (years) | | | | | | |
| | < 65 | 180 | 67 | 60 | 53 | NS |
| | ≧ 65 | 186 | 56 | 61 | 69 | |
| Histological type | | | | | | |
| | ADC | 234 | 69 | 88 | 77 | 0.0483* |
| | SCC | 104 | 43 | 29 | 32 | |
| | LCC | 28 | 11 | 4 | 13 | |
| pT factor | | | | | | |
| | T1 | 124 | 27 | 44 | 53 | 0.0006* |
| | T2+T3+T4 | 242 | 96 | 77 | 69 | |
| pN factor | | | | | | |
| | N0 | 226 | 55 | 86 | 85 | <0.0001* |
| | N1+N2 | 140 | 68 | 35 | 37 | |

| | | | | | | |
|---|---|---|---|---|---|---|
| ADC, adenocarcinoma; SCC, squamous-cell carcinoma LCC, large-cell carcinoma * P < 0.05 (Chi-square test) NS, no significance | | | | | | |

**Table 3b. Cox's proportional hazards model analysis of prognostic factors in patients with NSCLC,**

| Variables | Hazards ratio | 95% CI | Unfavorable/Favorable | P-value |
|---|---|---|---|---|
| Univariate analysis | | | | |
| | | | Strong(+) / Weak(+) | |
| Phospho-EGFR (Tyr-992) | 2.058 | 1.530-2.767 | | <0.0001 * |
| | | 1,530-2.767 | or (-) | |
| Age (years) | 1.448 | 1.076-1.947 | 65 ≧ / <65 | 0.0144 * |
| Gender | 1.664 | 1.184-2.338 | Male / Female | 0.0033 * |
| Histological type | 1.446 | 1.076-1.943 | SCC I/others | NS |
| pT factor | 2.693 | 1.851-3.898 | T2+T3+T4 / T1 | <0.0001 * |
| pN factor | 2.536 | 1.890-3.403 | N1+N2/N0 | <0.0001 * |
| | | | | |

| Variables | Hazards | 95% CI | Unfavorable/Favorable | P-value |
|---|---|---|---|---|
| | ratio | | | |
| Multivariate analysis | | | | |
| | | | Sarong(+) / Weak(+) | |
| Phospho-EGFR (Tyr-992) | 1.538 | 1.124-2.267 | | 0.0071* |
| | | | or (-) | |
| Age (years) | 1.677 | 1.241-2.267 | 65 ≧ / <65 | 0.0008 * |
| Gender | 1.382 | 0.977-1.956 | Male / Fernale | NS |
| pT factor | 2.028 | 1.383-2.973 | T2+T3+T4 / T1 | 0.0003 * |
| pN factor | 2.218 | 1.625-3.027 | N1+N2/N0 | <0.0001 * |

| | | | | |
|---|---|---|---|---|
| 1 SCC, squamous cell-carcinoma * P < 0.05 NS, no significance | | | | |

The requirement of EGF for the phosphorylation of EGFR by TTK- was then examined. TTK was exogenously over-expressed in serum-starved COS-7 cells and microscopically observed that phospho-EGFR (Tyr-992) was detected as small spots around the nucleus in only TTK-expressing cells (**Figure 4k** and **4l**). Endogenous phospho-EGFR (Tyr-992) was localized in the nucleus of serum-starved A549 cells that over-express TTK endogenously (**Figure 4m****, left panel**), whereas reduction of TTK protein by RNAi-TTK (oligo) significantly decreased the phospho-EGFR (Tyr-992) (**Figure 4m****, right pacel**). The data suggested the TTK-induced phosphorylation at Tyr-992 of EGFR and its internalization that occurred independently from EGF stimulation.

### Example 8: Activation of MAPK signals by phospho-EGFR (Tyr-992) in a TTK-dependent oncogenic pathway

The phosphorylation level of EGFR Tyr-992 was then examined in the mitotic phase of A549 cells, when the expression level of TTK was remarkably enhanced (**Figure 5a**). Phosphorylation levels of EGFR on Tyr-992 were in concordant with the expression levels of phospho-TTK. To assess whether the abundant expression of TTK is critical for phosphorylation of EGFR Tyr-992 in cancer cells, the expression of the TTK mRNA was selectively knocked down in A549 cells by using siRNA against TTK (RNAi-TTK (oligo)). Reduction of TTK protein by RNAi-TTK (oligo) decreased the phosphorylation level of EGFR Tyr-992 (**Figure 5b**), indicating that endogenous TTK in lung cancer cells induces the phosphorylation of EGFR. Tyr-992 of EGFR is known to be a binding site for two adaptor proteins, phospholipase Cγ (PLOγ) and Shc, and its phosphorylation is required for activation (phosphorylation) of these adaptor proteins15-17. Hence, we investigated the phosphorylation levels of these adaptor proteins after the down-regulation of TTK expression by RNAi-TTK (oligo). In accordance with the decrease of phospho-EGFR Tyr-992 by RNAi-TTK (oligo), the decrease of the phospho-PLCγ and phosphorylation of p44142 MAPK was observed, while this RNAi did not affect the amounts of PLCγ or p44/42 MAPK proteins (**Figure 5b**). Immunocytochemical analysis detected that phospho-p44/42 MAPK was accumulated in the nucleus of COS-7 cells that were transfected with TTK expression vector (**Figure 5c**). Furthermore, the interaction between PLCγ and EGFR was confirmed by immunoprecipitaion experiment using extracts from COS-7 cells transfected with the TTK-expression vector, while their association was scarcely observed in the cells transfected with the TTK-KD- or mock-vector (**Figure 5d**).

### Example 9: Identification of a novel phosphorylation site (Ser-967) in EGFR by TTK

GST-tagged EGFR-DEL2 protein phosphorylated by TTK was detected as double bands (**Figure 4f****, right panels**). To confirm whether EGFR Tyr-992 was the only phosphorylation site on EGFR, [gamma-³²P] ATP in vitro kinase assay was performed by using GST-tagged EGFR-DEL2 (Y992A) protein, in which Tyr-992 was substituted to alanine. This mutation remarkably reduced the intensity of lower-band, but did not reduce that of upper-band (**Figure 6a**). The fact prompted us to screen other TTK-dependent phosphorylation site(s) on EGFR. MALDI tandem mass spectrometric analysis was performed by using the GST-tagged EGFR-DEL2 protein that was in vitro phosphorylated by TTK, and phosphorylation of EGFR Ser-967 was identified (data not shown). Next, anti-phospho-EGFR (Ser-967) antibodies using Ser-967-ghosphorylated synthetic peptides were raised for immunization, and the expression pattern of TTK, phospho-EGFR (Ser-967), and total EGFR in lung-cancer cells was investigated by western blotting: Interestingly, phosphorylation level of EGFR Ser-967 in NSCLC cells were significantly correlated with protein expression levels of TTK (**Figure 6b**), indicating that EGFR Ser-967 was phosphorylated by TTK. Next TTK was over-expressed in COS-7 cells, and it was microscopically observed that phospho-EGFR (Ser -967) was increased in the nucleus of TTK-expressing cells (**Figure 6c**). Immunocytochemical analysis demonstrated that endogenous phospho-EGFR (Ser-967) was localized in the nucleus of A549 cells that over-expressed endogenous TTK (**Figure 6d**, left panel), whereas suppression of TTK protein expression by RNAi-TTK (oligo) decreased significantly the levels of phospho-EGFR Ser -967 (**Figure 6d**, right panel). These results suggest the TTK-induced phosphorylation at Ser -967 of EGFR and its nuclear transportation that occurred independently from EGF stimulation. Immunohistochemical analysis was also performed with anti-phospho-EGFR (Ser-967) antibody using tissue microarrays consisting of 374 NSCLC, and it was found that the pattern of phospho-EGFR (Ser-967) positivity was significantly concordant with TTK positivity in these tumors (P < 0.0001) independently confirmed the results obtained by in vitro assays. Strong expression of phospho-EGFR (Ser-967) in NSCLCs was significantly associated with tumor-specific survival (P < 0.0001 by the Log-rank test) (**Figure 6e** and **6f**; Details were shown in **Tables 4a** and **4b**. These evidences demonstrate that phosphorylation at Ser-967 of EGFR by TTK, could also significantly affect the growth and malignant nature of lung-cancer cells.

**Table 4a. Association between nuclear EGFR-positivity in NSCLC tissues and patients' characteristics (n-351)**

| | | Total | EGFR strong positive | EGFR 967N weak positive | EGFR 967 N assent | *P*-value strong vs weak/absent |
|---|---|---|---|---|---|---|
| | | n=351 | n=164 | n=158 | n=29 | |
| Gender | | | | | | |
| | Male | 246 | 121 | 90 | 35 | 0.0465* |
| | Fernale | 105 | 39 | 48 | 18 | |
| Age (years) | | | | | | |
| | < 65 | 173 | 75 | 79 | 19 | NS |
| | ≧ 65 | 178 | 89 | 79 | 10 | |
| Histological type | | | | | | |
| | ADC | 225 | 95 | 108 | 22 | |
| | SCC | 88 | 49 | 38 | 1 | 0.0261* |
| | Others | 38 | 20 | 12 | 6 | |
| pT factor | | | | | | |
| | T1 | 116 | 40 | 59 | 17 | 0.0014* |
| | T2-T4 | 235 | 124 | 99 | 12 | |
| pN factor | | | | | | |
| | N0 | 216 | 99 | 96 | 21 | NS |
| | N1+N2 | 135 | 65 | 62 | 8 | |
| Smoking history | | | | | | |
| | Never smoker | 106 | 41 | 51 | 14 | 0.0487* |
| | Smoker | 245 | 123 | 107 | 15 | |

| | | | | | | |
|---|---|---|---|---|---|---|
| ADC, adenocarcinoma; SCC, squamous-cell carcinoma Others, large-cell carcinoma plus adenosquamous.cell carcinoma, *ADC versus other histology + *P* < 0.05 (Fisher's exact test) NS, no significance | | | | | | |

**Table 4b. Cox's proportional hazards model analysis of prognostic factors in patients with NSCLCs**

| | Variables | Hazards ratio | 95% Cl | Unfavorable/Favorable | P-value |
|---|---|---|---|---|---|
| Univariate analysis | | | | | |
| | nuclear EGFR | 2.012 | 1.486-2.724 | Strong(+) or (- | <0.0001* |
| | 967 | | | / Weak(+) ) | |
| | Age (years) | 1.427 | 1.058-1.929 | 65≧ / <65 | 0.0206 |
| | Gender | 1.619 | 1.115-2.271 | Male / Female | 0.0052 * |
| | Histological type | 1.386 | 1.026-1.874 | others / ADC¹ | 0.0337* |
| | pT factor | 2.656 | 1.817-3.883 | T2-4 / T1 | <0.0001 * |
| | pN factor | 2.530 | 1.878-3.413 | N1+N2 / N0 | <0.0001* |

| Multivariate | | | | | |
|---|---|---|---|---|---|
| analysis | | | | | |
| | nuclear EGFR | 1.750 | 1.281-2.391 | Strong(+) / Weak(+) or (- | 0.0004* |
| | 967 | | | ) | |
| | Age (years) | 1.611 | 1.18a-2.190 | 65≧ / <65 | 0.0023* |
| | Gender | 1.361 | 0.928-1.995 | Male / Female | 0.1145 |
| | Histological type | 0.972 | 0.694-1.360 | others ADC¹ | 0.8675 |
| | pT factor | 2.042 | 1.383-3_016 | T2-4 T1 | 0.0003* |
| | pN factor | 2.446 | 1.798-3.328 | N1+N2/NO | 0.0004* |

| | | | | | |
|---|---|---|---|---|---|
| ¹ ADC, adenocarcinoma * *P*< 0.05 | | | | | |

### Examples 10: Inhibition of cell growth/invasion by targeting TTK-EGFR pathway

To further investigate the involvement of EGFR in TTK-induced cell proliferation/invasion, the present inventors next introduced RNAi-EGFR (oligo) to HEK29 3-derived transfectants that stably expressed TTK. The proliferation of HEK293 cells was scarcely reduced (P = 0.2439) by RNAi-EGFR (oligo) (control cells transfected with mock vector), whereas the TTK-induced cell proliferation was significantly reduced (P = 0.0032) by RNAi-EGFR (**Figure 7a**). The invasion of in TTK-transfected HEK293 cells through Matrigel was significantly enhanced (P < 0. 000 1) compared to the control cells, and the TTK-induced cell invasion was reduced to near the basal level by RNAi-EGFR (P < 0.0001). Invasion of the control cells was slightly reduced (P < 0.0001) by RNAi-EGFR (oligo)) (P < 0.0161) (**Figure 7b**).

The biological importance of the association of TTK and EGFR proteins and its potential as therapeutic targets for lung cancer were subsequently investigated. As shown in **Figure 3c**, phosphorylation, of EGFR Tyr-992 in GST-tagged EGFR-DEL2 protein (amino acid 889-1045) was promoted in a TTK-dose dependent manner, while N-terminal truncated form of EGFR-DEL2, termed EGFR-DEL4 (amino acid 977-1045) protein was not phosphorylated at Tyr-992 (data not shown). These experiments suggested that the 88 amino-acid polypeptide (amino acid 889-976) in EGFR should play an important role for the interaction with TTK. To investigate the functional significance of interaction between TTK and EGFR for growth or survival of lung-cancer cells, we developed bioactive cell-permeable peptides that were expected to inhibit the binding of these two proteins. 7 different peptides of 19 or 30 amino acid sequence that included in codons 889-1001 of EGFR were synthesized (see **Example 1**). These peptides were covalently linked at NH2-terminalus to a membrane transducing 11 arginine-residues (11R). The effect on growth was evaluated by addition of the seven 11 R-EGFR peptides into culture medium of A549 cells, the treatment with the 11R-EGFR899-917 (SEQ ID NO: 44), 11R-EGFR918-936 (SEQ ID NO: 45) or 11R-EGFR93 7-955 (SEQ ID NO: 46) peptides resulted in significant decreases in cell viability as measured by MTT assay (**Figure 7c**).

### Example 11: Activating mutation of TTK in lung cancer

Oncogenic protein kinase activation by somatic mutation in kinase domain is one of the common mechanism of turnorigenesis (Herbst, R.S., et al., Nat Rev Cancer. 4, 956-965. (2004); Pal, S.K. and Pegram, M., Anticancer Drugs. 16, 483-494. (2005)). To examine the presence of activating mutations of TTK, the TTK kinase domain was directly sequenced by using mRNAs prepared from 36 lung-cancer cell lines and 60 clinical lung cancer tissue samples (30 primary NSCLCs and 30 metastatic brain tumors derived from primary NSCLCs). A missense mutation was found at codon 574 (Y574C) in a RERF-LC-A1 cell line; which had not been reported in the SNP databases (JSNP: http://snp.ims.u-tokyo.ac.jp/index_ja_html; DBSNP: http://www.ncbi.nlm.nih.gov/projects/SNP/) (**Figure 8a****, left panels**). In addition, two missense mutations were identified in clinical samples of two metastatic brain tumors derived from primary lung adenocarcinoma. The mutations resulted in the amino acid substitution; valine to Phenylalanine at codon 610 (V610F) (Case 2; **Figure 8a**, middle panels) and Glutamine to Histidine at codon 753 (Q753H) (Case 8; **Figure 8a**, right panels). Matched normal brain tissues from these two patients showed only the wild-type DNA sequences, indicating that these two mutations had arisen somatically during tumor formation or progression.

To evaluate the functional properties of the mutant TTK identified by mutational analysis, two mutant-TTK proteins (Y574C or Q753H) were expressed in cultured mammalian cells. The level of autophosphorylated TTK (phospho-TTK) was significantly higher in the mutant-TTK-expressing NIH-3T3 cells than in the cells transfected with the wild type-TTK (wt-TTK)-expression vector, indicating that these mutations could promote the kinase activity of TTK protein (**Figure 8b**). Matrigel invasion assay was then performed by using NIH-3T3 cells transfected with the TTK-Y574C construct, because the invasive ability of NIH-3T3 cells was enhanced by wt-TTK transient-expression (**Figure 3e**). Transfection of mutant-TTK (Y574C or Q753H) into NIH-3T3 cells resulted in significant increase in the number of invaded cells compared to that of wt-TTK (**Figure 8c** and **8d**). These results indicate that the TTK mutation in the kinase domain appears to fall in the category of gain of function mutation that could contribute to lung cancer progression.

### Discussion

Mps1 (TTK- is its human homologue) was first discovered in budding yeast as a factor to be required in centrosome duplication (Winey M, et al., J Cell Biol. 1991 Aug;114(4):745-54.) and was subsequently shown to have a critical function in the spindle checkpoint (Weiss E & Winey M. J Cell Biol. 1996 Jan;132(1-2):111-23.). In human, over-expression of TTK was found in several cancers, but their functional significance in carcinogenesis has been remained unclear (Stucke VM, et al., EMBO J. 2002 Apr 2;21(7):1723-32.).

It is herein demonstrated that the treatment of NSCLC cells with specific siRNA to TTK reduces its expression and caused growth suppression. The growth-promotive effect by introduction of TTK in mammalian cells also supports its oncogenic function. The results obtained by in vitro and in vivo assays suggest an important role of TTK in human cancer, and that screening of molecules targeting the TTK pathway presents a promising therapeutic approach for treating lung cancers. EGFR whose pathway was known to be involved in carcinogenesis of various tissues is herein revealed as a novel intracellular target molecule of TTK kinase. Also revealed herein is the discovery by tissue microarray analysis that NSCLC patients showing high levels of TTK and phospho-EGFR (Tyr-992 or Ser-967) revealed a shorter tumor-specific survival period. EGFR autophosphorylation has been shown to play a critical role in the activation of the MAPK cascade following EGF stimulation. Of the phosphorylation sites in EGFR, Tyr-992 was proven to be a high binding-affinity site to PLCgamma, and is required for PLCgamma activation by EGF stimulation (Rotin D, et al., EMBO J. 1992 Feb;11(2):559-67.). PLCgamma activates the Ras/MAPK cascade through inositol 1,4,5-triphosphate production and oscillations in cytosolic Ca²⁺ (Schmidt-Ullrich RK, Oncogene. 2003 Sep 1;22(37):5855-65.). The activation of the MAP kinase pathway is associated with cell division and its aberrant activity is supposed to be involved in the uncontrolled cell proliferation that occurs in tumors (Pal SK & Pegram M. Anticancer Drugs. 2005 Jun;16(5):483-94.). Interestingly, the data herein indicate that phosphorylation, of EGFR at Tyr-992 and Ser-967 by TTK is independent from the EGF stimulation. Phosphorylation of EGFR Ser-967 has been reported to be constitutive phosphorylation (Elisabetta et al., 2005), but any role of its function has not been implicated. Phosphorylation of EGFR at Ser-967 was mainly detected in nuclear EGFR. Nuclear EGFR was recently reported as a transcription factor or transcriptional coactivator (Lin et al., Nat Cell Biol. 3:802-8 (2001)). Our data indicated that phosphorylation of EGFR at Ser-967 may be important for nuclear localization of EGFR during lung carcinogenesis.

DNA sequences of the TTK kinase domain in 36 lung-cancer cell lines and 60 primary and metastatic NSCLCs were also examined, from which one lung squamous-cell carcinoma cell line that has an activating mutation promoting cellular invasive activity was identified. Point mutations were also found in 2 cases of brain metastatic lesion of adenocarcinoma. Interestingly, abundant expression of TTK protein kinase was observed in the great majority of lung-cancer samples of various histological types and much higher expression in advanced stage tumor and especially in brain metastasis (**Figure 9**). Previous reports also demonstrated that the EGFR-PLCgamma signaling pathway plays significant roles in tumor progression, especially in the invasive and metastatic state of prostate carcinoma, breast carcinoma, and head-and-neck squamous cell carcinoma (Chen P, et al., J Cell Biol. 1994 Feb;124(4):547-55.; Chen P, et al., J Cell Biol. 1994 Nov;127(3):847-57.; Thomas SM, et al., Cancer Res. 2003 Sep 1;63(17):5629-35.). These in vitro and in vivo data indicated that TTK through activation of the EGFR-PLCgamma signaling pathway plays an important role as a key molecule associated with brain metastasis, and that the specific subset of patients with lung cancer carrying the TTK mutations is more likely to suffer the metastatic disease to the brain.

In summary, the present invention strongly demonstrates for the first time that EGFR signaling is intracellularly regulated by the activated TTK kinase and that targeting of the enzymatic activity of TTK holds promise for development of a new strategy for treatment of lung-cancer patients.

### Industrial Applicability

As demonstrated herein, TTK has kinase activity for EGFR, and the suppression of this activity leads to the inhibition of cell proliferation of lung cancer cells. Thus, agents that inhibit the kinase activity of TTK for EGFR find therapeutic utility as anti-cancer agents for the treatment of lung cancer. For example, the phosphorylation site of EGFR by TTK is Try922 or Ser967, which is an EGF-independent phosphorylation.

In addition, the present invention provides a screening method for anti-cancer agents that inhibit the kinase activity of TTK for EGFR. EGFR has been recognized as an important mediator of growth signaling pathways. Accordingly, it is expected that candidate compounds that inhibit the critical step for cell proliferation can be isolated by the present invention.

In addition, the present invention demonstrates that treatment of lung cancer cells with siRNA against TTK suppresses its expression as well as the kinase activity of TTK for EGFR at Tyr-992 or Ser-967, and, thus, suppresses growth of cancer cells. This data implies that up-regulation of TTK function and enhancement of kinase activity of TTK for EGFR are common features of pulmonary carcinogenesis. Accordingly, the selective suppression of TTK kinase activity may be a promising therapeutic strategy for treatment of lung-cancer patients.

Alternatively, lung cancer can be detected using the kinase activity of TTK for EGFR as a diagnostic marker.

Furthermore, it was revealed herein that a high level of TTK expression and/or phospho-EGFR is significantly associated with poor prognosis for patients with lung cancer. Accordingly, a prognosis of lung cancer can be assessed or determined by measuring a TTK expression level and/or phosphor-EGFR (Tyr992 or Ser967).

In addition, in the present invention, it is revealed that TKK mutations are associated with a high risk of metastasis of lung cancer. Accordingly, risk assessment for metastasis of lung cancer can be achieved by detecting such mutations. Furthermore, method for detecting a TKK mutant is also provided by the present invention.

All patents, patent applications, and publications cited herein are incorporated by reference in their entirety. However, nothing herein should be construed as an admission that the invention is not entitled to antedate such disclosure by virtue of prior invention.
While the invention has been described in detail and with reference to specific embodiments thereof, it is to be understood that the foregoing description is exemplary and explanatory in nature and is intended to illustrate the invention and its preferred embodiments. Through routine experimentation, one skilled in the art will readily recognize that various changes and modifications can be made therein without departing from the spirit and scope of the invention. Further advantages and features will become apparent from the claims filed hereafter, with the scope of such claims to be determined by their reasonable equivalents, as would be understood by those skilled in the art. Thus, the invention is intended to be defined not by the above description, but by the following claims and their equivalents.

Furthermore, the present invention relates to the following items:
1. A method of assessing lung cancer prognosis, said method comprising the steps of:
   a. detecting either or both of a TTK expression level and a phosphorylation level of EGFR in a specimen collected from a subject whose lung cancer prognosis is to be assessed, and
   b. indicating a poor prognosis when an elevated either or both of TTK expression level and a phosphorylation level of EGFR, as compared to a control level, is detected.
2. The method of item 1, wherein the TTK expression level is detected by any one of the methods selected from the group consisting of:
   (a) detecting the presence of an mRNA encoding the amino acid sequence of SEQ ID NO: 2,
   (b) detecting the presence of a protein comprising the amino acid sequence of SEQ ID NO: 2, and
   (c) detecting a biological activity of a protein comprising the amino acid sequence, of SEQ ID NO: 2.
3. The method of item 2, wherein the biological activity of (c) is a kinase activity for EGFR.
4. The method of item 1, wherein the phosphorylation level of EGFR is detected at tyrosine of 992 amino acid residue or serine of 967 amino acid residue in SEQ ID NO: 42.
5. A kit for assessing lung cancer prognosis, wherein the kit comprises any one component selected from the group consisting of:
   (a) a reagent for detecting an mRNA encoding the amino acid sequence of SEQ ID NO: 2,
   (b) a reagent for detecting a protein comprising the amino acid sequence of SEQ ID NO: 2 or tyrosine of 992 amino acid residue or serine of 967 amino acid residue in SEQ ID NO: 42, and
   (c) a reagent for detecting a biological activity of a protein comprising the amino acid sequence of SEQ ID NO: 2.
6. A method of diagnosing lung cancer or a predisposition for developing lung cancer in a subject, comprising determining TTK expression level or phosphorylation level of EGFR in a biological sample from a subject, wherein an increase of said expression level or pohsphorylation level in said sample as compared to a normal control level of said gene indicates that said subject suffers from or is at risk of developing lung cancer.
7. The method of item 6, wherein said sample expression level is at least 10% greater than said normal control level.
8. The method of item 6, wherein TTK expression level is determined by a method selected from the group consisting of:
   a) detecting an RNA encoding the amino acid sequence of SEQ ID NO: 2,
   b) detecting a protein comprising the amino acid sequence of SEQ ID NO: 2, and
   c) detecting a biological activity of a protein comprising the amino acid sequence of SEQ ID NO: 2.
9. The method of item 8, wherein the biological activity is a kinase activity for EGFR.
10. The method of item 6, wherein the phosphorylation level of EGFR is detected at tyrosine of 992 amino acid residue or serine of 967 amino acid residue in SEQ ID NO: 42.
11. The method of item 6, wherein said subject-derived biological sample comprises an epithelial cell.
12. The method of item 6, wherein said biological sample comprises a lung cell.
13. The method of item 6 wherein said biological sample comprises an epithelial cell from a lung tissue.
14. A kit for diagnosing lung cancer or a predisposition for developing lung cancer in a subject, wherein the kit comprises a reagent for detecting the kinase activity of TTK for EGFR.
15. The kit of item 14, wherein the kinase activity of TTK for EGFR is an EGF-independent phosphorylation of EGFR by TTK.
16. A method of measuring a kinase activity of TTK for EGFR, said method comprising the steps of:
   a. incubating EGFR or functional equivalent thereof and TTK under conditions suitable for the EGFR phosphorylation, by TTK, wherein the TTK is selected from the group consisting of:
      i. a polypeptide comprising the amino acid sequence of SEQ ID NO: 2 (TTK);
      ii. a polypeptide comprising the amino acid sequence of SEQ ID NO: 2 wherein one or more amino acids are substituted, deleted, or inserted, provided the resulting polypeptide has a biological activity equivalent to the polypeptide consisting of the amino acid sequence of SEQ ID NO: 2;
      iii. a polypeptide encoded by a polynucleotide that hybridizes under stringent conditions to a polynucleotide consisting of the nucleotide sequence of SEQ ID NO: 1, provided the resulting polypeptide has a biological activity equivalent to a polypeptide consisting of the amino acid sequence of SEQ ID NO: 2;
   b. detecting a phospho-EGFR level; and
   c. measuring the kinase activity of TTK by correlating the phosphor-EGFR level detected in step (b).
17. The method of item 16, wherein the functional equivalent of EGFR is a fragment comprising amino acid sequence of SEQ ID NO: 43
18. The method of item 16, wherein the phospho-EGFR level is detected at tyrosine of 992 amino acid residue or serine of 967 amino acid residue in SEQ ID NO: 42.
19. A method of identifying an agent that modulates a kinase activity of TTK for EGFR, said method comprising the steps of:
   a. incubating EGFR or functional equivalent thereof and TTK in the presence of a test compound under conditions suitable for the phosphorylation of EGFR by TTK, wherein the TTK is a polypeptide selected from the group consisting of:
      i. a polypeptide comprising the amino acid sequence of SEQ ID NO: 2 (TTK);
      ii. a polypeptide comprising the amino acid sequence of SEQ ID NO: 2 wherein one or more amino acids are substituted, deleted, or inserted, provided the resulting polypeptide has a biological activity equivalent to the polypeptide consisting of the amino acid sequence of SEQ ID NO: 2;
      iii. a polypeptide encoded by a polynucleotide that hybridizes under stringent conditions to a polynucleotide, consisting of the nucleotide sequence of SEQ ID NO: 1, provided the resulting polypeptide has a biological activity equivalent to a polypeptide consisting of the amino acid sequence of SEQ ID NO: 2;
   b. detecting a phospho-EGFR level; and
   c. comparing the phospho-EGFR level to a control level, wherein an increase or decrease in the phospho-EGFR level as compared to said control level indicates that the test compound modulates the kinase activity of TTK for EGFR.
20. The method of item 19, wherein the functional equivalent of EGFR is a fragment comprising amino acid sequence of SEQ ID NO: 43
21. The method of item 19, wherein the phospho-EGFR level is detected at tyrosine of 992 amino acid residue or serine of 967 amino acid residue in SEQ ID NO: 42.
22. A method of screening for a compound for treating and/or preventing lung cancer, said method comprising the steps of:
   a. incubating EGFR or functional equivalent thereof and TTK in the presence of a test compound under conditions suitable for the phosphorylation of EGFR by TTK, wherein the TTK is a polypeptide selected from the group consisting of:
      i. a polypeptide comprising the amino acid sequence of SEQ ID NO: 2 (TTK);
      ii. a polypeptide comprising the amino acid sequence of SEQ ID NO: 2 wherein one or more amino acids are substituted, deleted, or inserted, provided said polypeptide has a biological activity equivalent to the polypeptide consisting of the amino acid sequence of SEQ ID NO: 2;
      iii. a polypeptide encoded by a polynucleotide that hybridizes under stringent conditions to a polynucleotide consisting of the nucleotide sequence of SEQ ID NO: 1, provided the polypeptide has a biological activity equivalent to a polypeptide consisting of the amino acid sequence of SEQ ID NO: 2;
   b. detecting a phospho-EGFR level; and
   c. selecting a compound that decreases the phospho-EGFR level as compared to a control level.
23. The method of item 22, wherein or functional equivalent of EGFR is a fragment comprising amino acid sequence of SEQ ID NO: 43.
24. The method of item 22, wherein the phospho-EGFR level is detected at tyrosine of 992 amino acid residue or serine of 967 amino acid residue in SEQ ID NO: 42.
25. A kit for detecting the ability of a test compound to modulate kinase activity of TTK for EGFR, said kit comprising the components of:
   A) a polypeptide selected from the group consisting of:
      i. a polypeptide comprising the amino acid sequence of SEQ ID NO: 2 (TTK);
      ii. a polypeptide comprising the amino acid sequence of SEQ ID NO: 2 wherein one or more amino acids are substituted, deleted, or inserted, provided the resulting polypeptide has a biological activity equivalent to the polypeptide consisting of the amino acid sequence of SEQ ID NO: 2;
      iii. a polypeptide encoded by a polynucleotide that hybridizes under stringent conditions to a polynucleotide consisting of the nucleotide sequence of SEQ ID NO: 1, provided the resulting polypeptide has a biological activity equivalent to a polypeptide consisting of the amino acid sequence of SEQ ID NO: 2;
   B) EGFR or EGFR fragment comprising the amino acid sequence of SEQ ID NO: 43; and
   C) a reagent for detecting a phospho-EGFR.
26. A kit for detecting for the ability of a test compound to modulate kinase activity of TTK for EGFR, said kit comprising the components of:
   A) a cell expressing EGFR or EGFR fragment comprising the amino acid sequence of SEQ ID NO: 43 and a polypeptide selected from the group consisting of:
      i. a polypeptide comprising the amino acid sequence of SEQ ID NO: 2 (TTK);
      ii. a polypeptide comprising the amino acid sequence of SEQ ID NO: 2 wherein one or more amino acids are substituted, deleted, or inserted, provided the resulting polypeptide has a biological activity equivalent to the polypeptide consisting of the amino acid sequence of SEQ ID NO: 2;
      iii. a polypeptide encoded by a polynucleotide that hybridizes under stringent conditions to a polynucleotide consisting of the nucleotide sequence of SEQ ID NO: 1, provided the resulting polypeptide has a biological activity equivalent to a polypeptide consisting of the amino acid sequence of SEQ ID NO: 2; and
   B) a reagent for detecting a phospho-EGFR
27. A method of predicting a metastasis of lung cancer, said method comprising the steps of:
   a. detecting one or more mutations of TTK at kinase domain,
   b. indicating a high risk of metastasis of lung cancer when a mutation is detected.
28. The method of item 27, wherein one or more mutations of TTK at kinase domain are selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2.
29.A method for detecting one or more mutation of TTK, wherein the mutation is at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, the method comprises steps of:
   a) contacting a subject polypeptide or cDNA encoding them with binding agent recognizing any one of the mutation of the polypeptide or cDNA encoding them,
   b) detecting the binding agent with the polypeptide or cDNA encoding them, and
   c) showing the mutation of TTK when the binding of the agent of step b) is detected.
30. The method of item 29, wherein the binding agent is an antibody that binds to polypeptide comprising at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, and substantially does not binds to a polypeptide consisting of the amino acid sequence of SEQ ID NO: 2.
31. A reagent for detecting one or more mutation of TTK, wherein the mutation is at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, wherein the reagent comprises a binding agent recognizing any one of the mutation of the polypeptide or cDNA encoding them.
32. The reagent of item 31, wherein the binding agent is an antibody that binds to polypeptide comprising at least one mutation selected from the group consisted of Valine to Phenylalanine at codon 610 (V610F), Glutamine to Histidine at codon 753 (Q753H) and Tyrosine to Cysteine at codon 574 (Y574C) of SEQ ID NO: 2, and substantially does not binds to a polypeptide consisting of the amino acid sequence of SEQ ID NO: 2.
33. An isolated polynucleotide comprising a mutated nucleotide sequence of SEQ ID NO: 1, wherein the nucleotide sequence comprises one or more mutations selected from the group consisting of A1870G (for V610F), G1977T (for Q753H) and G2408C (for Y574C), or fragment thereof comprising the one or more mutations.
34. An isolated polypeptide comprising a mutated amino acid sequence of SEQ ID NO: 2, wherein the amino acid sequence comprises one or more mutations selected from the group consisting of V610F, Q753H and for Y574C, or fragment thereof comprising the one or more mutations.
35. A method for treating or preventing lung cancer comprising the step of administering at least any one polypeptide selected from the group consisting of;
   (a) a polypeptide comprising ISSILEKGERLPQPPICTI (SEQ ID NO: 44), DVYMIMVKCWMIDADSRPK (SEQ ID NO: 45) or FRELIIEFSKMARDPQRYL (SEQ ID NO: 46), and
   (b) a polypeptide functionally equivalent to the polypeptide selected from the group consisting of ISSILEKGERLPQPPICTI (SEQ ID NO: 44), DVYMIMVKCWMIDADSRPK (SEQ ID NO: 45) and FRELIIEIEFSKMARDPQRYL (SEQ ID NO: 46),
   wherein the polypeptide lacks the biological function of a polypeptide consisting of the amino acid sequence of SEQ ID NO: 2,
   or a polynucleotide encoding the polypeptide selected from the polypeptide of (a) and (b).
36. A composition for treating or preventing lung cancer comprising a pharmaceutically effective amount of at least any one polypeptide selected from the group consisting of;
   (a) a polypeptide comprising ISSILEKGERLPQPPICTI (SEQ ID NO: 44), DVYMIMVKCWMIDADSRPK (SEQ ID NO: 45) or FRELIIEFSKMARDPQRYL (SEQ ID NO: 46), and
   (b) a polypeptide functionally equivalent to the polypeptide selected from the group consisting of ISSILEKGERLPQPPICTI (SEQ ID NO: 44), DVYMIMVKCWMIDADSRPK (SEQ ID NO: 45) and FRELIIEFSKMARDPQRYL (SEQ ID NO: 46),
   wherein the polypeptide lacks the biological function of a polypeptide consisting of the amino acid sequence of SEQ ID NO: 2
   or a polynucleotide encoding the polypeptide, selected from the polypeptide of (a) and (b) and a pharmaceutically acceptable carrier.
37. A polypeptide selected from the group consisting of;
   (a) a polypeptide comprising ISSILEKGERLPQPPICTI (SEQ ID NO: 44), DVYMIMVKCWMIDADSRPK (SEQ ID NO: 45) or FRELIIEFSKMARDPQRYL (SEQ ID NO: 46), and
   (b) a polypeptide having an amino acid sequence of a polypeptide functionally equivalent to the polypeptide selected from the group consisting of ISSILEKGERLPQPPICTI (SEQ ID NO: 44), DVYMIMVKCWMIDADSRPK (SEQ ID NO: 45) and FRELIIEFSKMARDPQRYL (SEQ ID NO: 46),
   wherein the polypeptide lacks the biological function of a polypeptide consisting of the amino acid sequence of SEQ ID NO: 2.
38. A polynucleotide encoding the polypeptide of item 37.
39. The polypeptide of the item 37, wherein the biological function is cell proliferation activity.
40. The polypeptide of item 37, wherein the polypeptide consists of 19 to 57 residues.
41. The polypeptide of item 37, wherein the polypeptide is modified with a cell-membrane permeable substance,
42. The polypeptide of item 41, which has the following general formula:

   [R]-[D];

   wherein [R] represents the cell-membrane permeable substance; and [D] represents the amino acid sequence of a fragment sequence which comprises ISSILEIGGERLPQPPICTI (SEQ ID NO: 44) or DVYMIMVKCWMIDADSRPK (SEQ ID NO: 45) or FRELIIEFSKMARDPQRYL (SEQ ID NO: 46); or the amino acid sequence of a polypeptide functionally equivalent to the polypeptide comprising said fragment sequence, wherein the polypeptide lacks the biological function of a polypeptide consisting of the amino acid sequence of SEQ ID NO: 2, wherein [R] and [D] can be linked directly or indirectly through a linker comprising amino acid sequences consisting of GGG.
43. The polypeptide of item 42, wherein the cell-membrane permeable substance is any one selected from the group consisting of:
   poly-arginine;
   Tat / RKKRRQRRR/SEQ ID NO: 47;
   Penetratin / RQIKIWFQNRRMKWKK/SEQ ID NO: 48;
   Buforin II / TRSSRAGLQFPVGRVHRLLRK/SEQ ID NO: 49;
   Transportan / GWTLNSAGYLLGKINLKALAALAKKIL/SEQ ID NO: 50;
   MAP (model amphipathic peptide) / KLALKLALKALKAALKLAJSEQ ID NO:51;
   K-FGF / AAVALLPAVLLALLAP/SEQ ID NO: 52;
   Ku70 / VPMLK/SEQ ID NO: 53
   Ku70 / PMLKE/SEQ ID NO: 61;
   Prion / MANLGYWLLALFVTMWTDVGLCKKRPKP/SEQ ID NO: 54;
   pVEC / LLIILRRRIRKQAHAHSK/SEQ ID NO: 55;
   Pep-1 / KETWWETWWTEWSQPKKKRKV/SEQ ID NO: 56;
   SynB1 / RGGRLSYSRRRFSTSTGR/SEQ ID NO: 57;
   Pep-7 / SDLWEMMMVSLACQY/SEQ ID NO: 58; and
   HN-1 / TSPLNIHNGQKL/SEQ ID NO: 59.
44. The polypeptide of item 43, wherein the poly-arginine is Arg 11 (RRRRRRRRRRR/SEQ ID NO: 60).
45. A method for treating or preventing lung cancer comprising administering to subject a composition comprising a double-stranded molecule which reduces TTK (SEQ ID NO: 1) or EGFR (SEQ ID NO: 3) gene expression, wherein the double-stranded molecule comprises a sense nucleic acid and an anti-sense nucleic acid, wherein the sense nuclei acid comprises a ribonucleotide sequence corresponding to a sequence of SEQ ID NO: 62 or 63 as the target sequence.
46. The method of item 45, wherein said composition comprises a transfection-enhancing agent.
47. A composition for treating or preventing lung cancer comprising a pharmaceutically effective amount of a double-stranded molecule which reduces TTK (SEQ ID NO: 1) or EGFR (SEQ ID NO: 3) gene expression, and a pharmaceutically acceptable carrier, wherein the double-stranded molecule comprises a sense nucleic acid and an anti-sense nucleic acid, wherein the sense nucleic acid comprises a ribonucleotide sequence corresponding to a sequence of SEQ ID NO: 62 or 63 as the target sequence.

## Claims

1. A double-stranded molecule which reduces TTK (SEQ ID NO: 1) or EGFR (SEQ ID NO: 3) gene expression for use in treating or preventing lung cancer, wherein the double-stranded molecule comprises a sense nucleic acid and an anti-sense nucleic acid, wherein the sense nucleic acid comprises a ribonucleotide sequence corresponding to a sequence of SEQ ID NO: 62 or 63 as the target sequence.

2. A composition for use in treating or preventing lung cancer comprising a pharmaceutically effective amount of a double-stranded molecule which reduces TTK (SEQ ID NO: 1) or EGFR (SEQ ID NO: 3) gene expression, and a pharmaceutically acceptable carrier, wherein the double-stranded molecule comprises a sense nucleic acid and an anti-sense nucleic acid, wherein the sense nucleic acid comprises a ribonucleotide sequence corresponding to a sequence of SEQ ID NO: 62 or 63 as the target sequence.

3. The composition of claim 2, wherein said composition further comprises a transfection-enhancing agent.

4. A double-stranded molecule which reduces TTK (SEQ ID NO: 1) or EGFR (SEQ ID NO: 3) gene expression, wherein the double-stranded molecule comprises a sense nucleic acid and an anti-sense nucleic acid, wherein the sense nucleic acid comprises a ribonucleotide sequence corresponding to a sequence of SEQ ID NO: 62 or 63 as the target sequence.


SEQUENCE LISTING

<110>  ONCOTHERAPY SCIENCE, INC.

<120>  TTK AS TUMOR MARKER AND THERAPEUTIC TARGET FOR LUNG CANCER

<130>  ONC-A0619P

<150>  60/874,791
<151>  2006-12-13

<160>  63

<170>  PatentIn version 3.4

<210>  1
<211>  2984
<212>  DNA
<213>  Homo sapiens

<220>
<221>  CDS
<222>  (75)..(2648)

<400>  1
ggaaattcaa acgtgtttgc ggaaaggagt ttgggttcca tcttttcatt tccccagcgc      60

agctttctgt agaa atg gaa tcc gag gat tta agt ggc aga gaa ttg aca      110
                Met Glu Ser Glu Asp Leu Ser Gly Arg Glu Leu Thr
                1               5                   10

att gat tcc ata atg aac aaa gtg aga gac att aaa aat aag ttt aaa 158
Ile Asp Ser Ile Met Asn Lys Val Arg Asp Ile Lys Asn Lys Phe Lys
        15                  20                  25

aat gaa gac ctt act gat gaa cta agc ttg aat aaa att tct gct gat 206
Asn Glu Asp Leu Thr Asp Glu Leu Ser Leu Asn Lys Ile Ser Ala Asp
    30                  35                  40

act aca gat aac tcg gga act gtt aac caa att atg atg atg gca aac 254
Thr Thr Asp Asn Ser Gly Thr Val Asn Gln Ile Met Met Met Ala Asn
45                  50                  55                  60

aac cca gag gac tgg ttg agt ttg ttg ctc aaa cta gag aaa aac agt 302
Asn Pro Glu Asp Trp Leu Ser Leu Leu Leu Lys Leu Glu Lys Asn Ser
                65                  70                  75

gtt ccg cta agt gat gct ctt tta aat aaa ttg att ggt cgt tac agt 350
Val Pro Leu Ser Asp Ala Leu Leu Asn Lys Leu Ile Gly Arg Tyr Ser
            80                  85                  90

caa gca att gaa gcg ctt ccc cca gat aaa tat ggc caa aat gag agt 398
Gln Ala Ile Glu Ala Leu Pro Pro Asp Lys Tyr Gly Gln Asn Glu Ser
        95                  100                 105

ttt gct aga att caa gtg aga ttt gct gaa tta aaa gct att caa gag 446
Phe Ala Arg Ile Gln Val Arg Phe Ala Glu Leu Lys Ala Ile Gln Glu
    110                 115                 120

cca gat gat gca cgt gac tac ttt caa atg gcc aga gca aac tgc aag 494
Pro Asp Asp Ala Arg Asp Tyr Phe Gln Met Ala Arg Ala Asn Cys Lys
125                 130                 135                 140

aaa ttt gct ttt gtt cat ata tct ttt gca caa ttt gaa ctg tca caa 542
Lys Phe Ala Phe Val His Ile Ser Phe Ala Gln Phe Glu Leu Ser Gln
                145                 150                 155

ggt aat gtc aaa aaa agt aaa caa ctt ctt caa aaa gct gta gaa cgt 590
Gly Asn Val Lys Lys Ser Lys Gln Leu Leu Gln Lys Ala Val Glu Arg
            160                 165                 170

gga gca gta cca cta gaa atg ctg gaa att gcc ctg cgg aat tta aac 638
Gly Ala Val Pro Leu Glu Met Leu Glu Ile Ala Leu Arg Asn Leu Asn
        175                 180                 185

ctc caa aaa aag cag ctg ctt tca gag gag gaa aag aag aat tta tca 686
Leu Gln Lys Lys Gln Leu Leu Ser Glu Glu Glu Lys Lys Asn Leu Ser
    190                 195                 200

gca tct acg gta tta act gcc caa gaa tca ttt tcc ggt tca ctt ggg 734
Ala Ser Thr Val Leu Thr Ala Gln Glu Ser Phe Ser Gly Ser Leu Gly
205                 210                 215                 220

cat tta cag aat agg aac aac agt tgt gat tcc aga gga cag act act 782
His Leu Gln Asn Arg Asn Asn Ser Cys Asp Ser Arg Gly Gln Thr Thr
                225                 230                 235

aaa gcc agg ttt tta tat gga gag aac atg cca cca caa gat gca gaa 830
Lys Ala Arg Phe Leu Tyr Gly Glu Asn Met Pro Pro Gln Asp Ala Glu
            240                 245                 250

ata ggt tac cgg aat tca ttg aga caa act aac aaa act aaa cag tca 878
Ile Gly Tyr Arg Asn Ser Leu Arg Gln Thr Asn Lys Thr Lys Gln Ser
        255                 260                 265

tgc cca ttt gga aga gtc cca gtt aac ctt cta aat agc cca gat tgt 926
Cys Pro Phe Gly Arg Val Pro Val Asn Leu Leu Asn Ser Pro Asp Cys
    270                 275                 280

gat gtg aag aca gat gat tca gtt gta cct tgt ttt atg aaa aga caa 974
Asp Val Lys Thr Asp Asp Ser Val Val Pro Cys Phe Met Lys Arg Gln
285                 290                 295                 300

acc tct aga tca gaa tgc cga gat ttg gtt gtg cct gga tct aaa cca 1022
Thr Ser Arg Ser Glu Cys Arg Asp Leu Val Val Pro Gly Ser Lys Pro
                305                 310                 315

agt gga aat gat tcc tgt gaa tta aga aat tta aag tct gtt caa aat 1070
Ser Gly Asn Asp Ser Cys Glu Leu Arg Asn Leu Lys Ser Val Gln Asn
            320                 325                 330

agt cat ttc aag gaa cct ctg gtg tca gat gaa aag agt tct gaa ctt 1118
Ser His Phe Lys Glu Pro Leu Val Ser Asp Glu Lys Ser Ser Glu Leu
        335                 340                 345

att att act gat tca ata acc ctg aag aat aaa acg gaa tca agt ctt 1166
Ile Ile Thr Asp Ser Ile Thr Leu Lys Asn Lys Thr Glu Ser Ser Leu
    350                 355                 360

cta gct aaa tta gaa gaa act aaa gag tat caa gaa cca gag gtt cca 1214
Leu Ala Lys Leu Glu Glu Thr Lys Glu Tyr Gln Glu Pro Glu Val Pro
365                 370                 375                 380

gag agt aac cag aaa cag tgg caa tct aag aga aag tca gag tgt att 1262
Glu Ser Asn Gln Lys Gln Trp Gln Ser Lys Arg Lys Ser Glu Cys Ile
                385                 390                 395

aac cag aat cct gct gca tct tca aat cac tgg cag att ccg gag tta 1310
Asn Gln Asn Pro Ala Ala Ser Ser Asn His Trp Gln Ile Pro Glu Leu
            400                 405                 410

gcc cga aaa gtt aat aca gag cag aaa cat acc act ttt gag caa cct 1358
Ala Arg Lys Val Asn Thr Glu Gln Lys His Thr Thr Phe Glu Gln Pro
        415                 420                 425

gtc ttt tca gtt tca aaa cag tca cca cca ata tca aca tct aaa tgg 1406
Val Phe Ser Val Ser Lys Gln Ser Pro Pro Ile Ser Thr Ser Lys Trp
    430                 435                 440

ttt gac cca aaa tct att tgt aag aca cca agc agc aat acc ttg gat 1454
Phe Asp Pro Lys Ser Ile Cys Lys Thr Pro Ser Ser Asn Thr Leu Asp
445                 450                 455                 460

gat tac atg agc tgt ttt aga act cca gtt gta aag aat gac ttt cca 1502
Asp Tyr Met Ser Cys Phe Arg Thr Pro Val Val Lys Asn Asp Phe Pro
                465                 470                 475

cct gct tgt cag ttg tca aca cct tat ggc caa cct gcc tgt ttc cag 1550
Pro Ala Cys Gln Leu Ser Thr Pro Tyr Gly Gln Pro Ala Cys Phe Gln
            480                 485                 490

cag caa cag cat caa ata ctt gcc act cca ctt caa aat tta cag gtt 1598
Gln Gln Gln His Gln Ile Leu Ala Thr Pro Leu Gln Asn Leu Gln Val
        495                 500                 505

tta gca tct tct tca gca aat gaa tgc att tcg gtt aaa gga aga att 1646
Leu Ala Ser Ser Ser Ala Asn Glu Cys Ile Ser Val Lys Gly Arg Ile
    510                 515                 520

tat tcc att tta aag cag ata gga agt gga ggt tca agc aag gta ttt 1694
Tyr Ser Ile Leu Lys Gln Ile Gly Ser Gly Gly Ser Ser Lys Val Phe
525                 530                 535                 540

cag gtg tta aat gaa aag aaa cag ata tat gct ata aaa tat gtg aac 1742
Gln Val Leu Asn Glu Lys Lys Gln Ile Tyr Ala Ile Lys Tyr Val Asn
                545                 550                 555

tta gaa gaa gca gat aac caa act ctt gat agt tac cgg aac gaa ata 1790
Leu Glu Glu Ala Asp Asn Gln Thr Leu Asp Ser Tyr Arg Asn Glu Ile
            560                 565                 570

gct tat ttg aat aaa cta caa caa cac agt gat aag atc atc cga ctt 1838
Ala Tyr Leu Asn Lys Leu Gln Gln His Ser Asp Lys Ile Ile Arg Leu
        575                 580                 585

tat gat tat gaa atc acg gac cag tac atc tac atg gta atg gag tgt 1886
Tyr Asp Tyr Glu Ile Thr Asp Gln Tyr Ile Tyr Met Val Met Glu Cys
    590                 595                 600

gga aat att gat ctt aat agt tgg ctt aaa aag aaa aaa tcc att gat 1934
Gly Asn Ile Asp Leu Asn Ser Trp Leu Lys Lys Lys Lys Ser Ile Asp
605                 610                 615                 620

cca tgg gaa cgc aag agt tac tgg aaa aat atg tta gag gca gtt cac 1982
Pro Trp Glu Arg Lys Ser Tyr Trp Lys Asn Met Leu Glu Ala Val His
                625                 630                 635

aca atc cat caa cat ggc att gtt cac agt gat ctt aaa cca gct aac 2030
Thr Ile His Gln His Gly Ile Val His Ser Asp Leu Lys Pro Ala Asn
            640                 645                 650

ttt ctg ata gtt gat gga atg cta aag cta att gat ttt ggg att gca 2078
Phe Leu Ile Val Asp Gly Met Leu Lys Leu Ile Asp Phe Gly Ile Ala
        655                 660                 665

aac caa atg caa cca gat aca aca agt gtt gtt aaa gat tct cag gtt 2126
Asn Gln Met Gln Pro Asp Thr Thr Ser Val Val Lys Asp Ser Gln Val
    670                 675                 680

ggc aca gtt aat tat atg cca cca gaa gca atc aaa gat atg tct tcc 2174
Gly Thr Val Asn Tyr Met Pro Pro Glu Ala Ile Lys Asp Met Ser Ser
685                 690                 695                 700

tcc aga gag aat ggg aaa tct aag tca aag ata agc ccc aaa agt gat 2222
Ser Arg Glu Asn Gly Lys Ser Lys Ser Lys Ile Ser Pro Lys Ser Asp
                705                 710                 715

gtt tgg tcc tta gga tgt att ttg tac tat atg act tac ggg aaa aca 2270
Val Trp Ser Leu Gly Cys Ile Leu Tyr Tyr Met Thr Tyr Gly Lys Thr
            720                 725                 730

cca ttt cag cag ata att aat cag att tct aaa tta cat gcc ata att 2318
Pro Phe Gln Gln Ile Ile Asn Gln Ile Ser Lys Leu His Ala Ile Ile
        735                 740                 745

gat cct aat cat gaa att gaa ttt ccc gat att cca gag aaa gat ctt 2366
Asp Pro Asn His Glu Ile Glu Phe Pro Asp Ile Pro Glu Lys Asp Leu
    750                 755                 760

caa gat gtg tta aag tgt tgt tta aaa agg gac cca aaa cag agg ata 2414
Gln Asp Val Leu Lys Cys Cys Leu Lys Arg Asp Pro Lys Gln Arg Ile
765                 770                 775                 780

tcc att cct gag ctc ctg gct cat ccc tat gtt caa att caa act cat 2462
Ser Ile Pro Glu Leu Leu Ala His Pro Tyr Val Gln Ile Gln Thr His
                785                 790                 795

cca gtt aac caa atg gcc aag gga acc act gaa gaa atg aaa tat gtt 2510
Pro Val Asn Gln Met Ala Lys Gly Thr Thr Glu Glu Met Lys Tyr Val
            800                 805                 810

ctg ggc caa ctt gtt ggt ctg aat tct cct aac tcc att ttg aaa gct 2558
Leu Gly Gln Leu Val Gly Leu Asn Ser Pro Asn Ser Ile Leu Lys Ala
        815                 820                 825

gct aaa act tta tat gaa cac tat agt ggt ggt gaa agt cat aat tct 2606
Ala Lys Thr Leu Tyr Glu His Tyr Ser Gly Gly Glu Ser His Asn Ser
    830                 835                 840

tca tcc tcc aag act ttt gaa aaa aaa agg gga aaa aaa tga 2648
Ser Ser Ser Lys Thr Phe Glu Lys Lys Arg Gly Lys Lys
845                 850                 855

tttgcagtta ttcgtaatgt caaataccac ctataaaata tattggactg ttatactctt 2708

gaatccctgt ggaaatctac atttgaagac aacatcactc tgaagtgtta tcagcaaaaa 2768

aaattcagta gattatcttt aaaagaaaac tgtaaaaata gcaaccactt atggtactgt 2828

atatattgta gacttgtttt ctctgtttta tgctcttgtg taatctactt gacatcattt 2888

tactcttgga atagtgggtg gatagcaagt atattctaaa aaactttgta aataaagttt 2948

tgtggctaaa atgacactaa aaaaaaaaaa aaaaaa 2984

<210> 2
<211> 857
<212> PRT
<213> Homo sapiens

<400> 2

Met Glu Ser Glu Asp Leu Ser Gly Arg Glu Leu Thr Ile Asp Ser Ile
1               5                   10                  15

Met Asn Lys Val Arg Asp Ile Lys Asn Lys Phe Lys Asn Glu Asp Leu
            20                  25                  30

Thr Asp Glu Leu Ser Leu Asn Lys Ile Ser Ala Asp Thr Thr Asp Asn
        35                  40                  45

Ser Gly Thr Val Asn Gln Ile Met Met Met Ala Asn Asn Pro Glu Asp
    50                  55                  60

Trp Leu Ser Leu Leu Leu Lys Leu Glu Lys Asn Ser Val Pro Leu Ser
65                  70                  75                  80

Asp Ala Leu Leu Asn Lys Leu Ile Gly Arg Tyr Ser Gln Ala Ile Glu
                85                  90                  95

Ala Leu Pro Pro Asp Lys Tyr Gly Gln Asn Glu Ser Phe Ala Arg Ile
            100                 105                 110

Gln Val Arg Phe Ala Glu Leu Lys Ala Ile Gln Glu Pro Asp Asp Ala
        115                 120                 125

Arg Asp Tyr Phe Gln Met Ala Arg Ala Asn Cys Lys Lys Phe Ala Phe
    130                 135                 140

Val His Ile Ser Phe Ala Gln Phe Glu Leu Ser Gln Gly Asn Val Lys
145                 150                 155                 160

Lys Ser Lys Gln Leu Leu Gln Lys Ala Val Glu Arg Gly Ala Val Pro
                165                 170                 175

Leu Glu Met Leu Glu Ile Ala Leu Arg Asn Leu Asn Leu Gln Lys Lys
            180                 185                 190

Gln Leu Leu Ser Glu Glu Glu Lys Lys Asn Leu Ser Ala Ser Thr Val
        195                 200                 205

Leu Thr Ala Gln Glu Ser Phe Ser Gly Ser Leu Gly His Leu Gln Asn
    210                 215                 220

Arg Asn Asn Ser Cys Asp Ser Arg Gly Gln Thr Thr Lys Ala Arg Phe
225                 230                 235                 240
Leu Tyr Gly Glu Asn Met Pro Pro Gln Asp Ala Glu Ile Gly Tyr Arg
                245                 250                 255
Asn Ser Leu Arg Gln Thr Asn Lys Thr Lys Gln Ser Cys Pro Phe Gly
            260                 265                 270
Arg Val Pro Val Asn Leu Leu Asn Ser Pro Asp Cys Asp Val Lys Thr
        275                 280                 285
Asp Asp Ser Val Val Pro Cys Phe Met Lys Arg Gln Thr Ser Arg Ser
    290                 295                 300
Glu Cys Arg Asp Leu Val Val Pro Gly Ser Lys Pro Ser Gly Asn Asp
305                 310                 315                 320
Ser Cys Glu Leu Arg Asn Leu Lys Ser Val Gln Asn Ser His Phe Lys
                325                 330                 335
Glu Pro Leu Val Ser Asp Glu Lys Ser Ser Glu Leu Ile Ile Thr Asp
            340                 345                 350

Ser Ile Thr Leu Lys Asn Lys Thr Glu Ser Ser Leu Leu Ala Lys Leu
        355                 360                 365

Glu Glu Thr Lys Glu Tyr Gln Glu Pro Glu Val Pro Glu Ser Asn Gln
    370                 375                 380

Lys Gln Trp Gln Ser Lys Arg Lys Ser Glu Cys Ile Asn Gln Asn Pro
385                 390                 395                 400

Ala Ala Ser Ser Asn His Trp Gln Ile Pro Glu Leu Ala Arg Lys Val
                405                 410                 415

Asn Thr Glu Gln Lys His Thr Thr Phe Glu Gln Pro Val Phe Ser Val
            420                 425                 430

Ser Lys Gln Ser Pro Pro Ile Ser Thr Ser Lys Trp Phe Asp Pro Lys
        435                 440                 445

Ser Ile Cys Lys Thr Pro Ser Ser Asn Thr Leu Asp Asp Tyr Met Ser
    450                 455                 460

Cys Phe Arg Thr Pro Val Val Lys Asn Asp Phe Pro Pro Ala Cys Gln
465                 470                 475                 480

Leu Ser Thr Pro Tyr Gly Gln Pro Ala Cys Phe Gln Gln Gln Gln His
                485                 490                 495

Gln Ile Leu Ala Thr Pro Leu Gln Asn Leu Gln Val Leu Ala Ser Ser
            500                 505                 510

Ser Ala Asn Glu Cys Ile Ser Val Lys Gly Arg Ile Tyr Ser Ile Leu
        515                 520                 525

Lys Gln Ile Gly Ser Gly Gly Ser Ser Lys Val Phe Gln Val Leu Asn
    530                 535                 540

Glu Lys Lys Gln Ile Tyr Ala Ile Lys Tyr Val Asn Leu Glu Glu Ala
545                 550                 555                 560

Asp Asn Gln Thr Leu Asp Ser Tyr Arg Asn Glu Ile Ala Tyr Leu Asn
                565                 570                 575

Lys Leu Gln Gln His Ser Asp Lys Ile Ile Arg Leu Tyr Asp Tyr Glu
            580                 585                 590

Ile Thr Asp Gln Tyr Ile Tyr Met Val Met Glu Cys Gly Asn Ile Asp
        595                 600                 605

Leu Asn Ser Trp Leu Lys Lys Lys Lys Ser Ile Asp Pro Trp Glu Arg
    610                 615                 620

Lys Ser Tyr Trp Lys Asn Met Leu Glu Ala Val His Thr Ile His Gln
625                 630                 635                 640

His Gly Ile Val His Ser Asp Leu Lys Pro Ala Asn Phe Leu Ile Val
                645                 650                 655

Asp Gly Met Leu Lys Leu Ile Asp Phe Gly Ile Ala Asn Gln Met Gln
            660                 665                 670

Pro Asp Thr Thr Ser Val Val Lys Asp Ser Gln Val Gly Thr Val Asn
        675                 680                 685

Tyr Met Pro Pro Glu Ala Ile Lys Asp Met Ser Ser Ser Arg Glu Asn
    690                 695                 700

Gly Lys Ser Lys Ser Lys Ile Ser Pro Lys Ser Asp Val Trp Ser Leu
705                 710                 715                 720

Gly Cys Ile Leu Tyr Tyr Met Thr Tyr Gly Lys Thr Pro Phe Gln Gln
                725                 730                 735

Ile Ile Asn Gln Ile Ser Lys Leu His Ala Ile Ile Asp Pro Asn His
            740                 745                 750

Glu Ile Glu Phe Pro Asp Ile Pro Glu Lys Asp Leu Gln Asp Val Leu
        755                 760                 765

Lys Cys Cys Leu Lys Arg Asp Pro Lys Gln Arg Ile Ser Ile Pro Glu
    770                 775                 780

Leu Leu Ala His Pro Tyr Val Gln Ile Gln Thr His Pro Val Asn Gln
785                 790                 795                 800

Met Ala Lys Gly Thr Thr Glu Glu Met Lys Tyr Val Leu Gly Gln Leu
                805                 810                 815

Val Gly Leu Asn Ser Pro Asn Ser Ile Leu Lys Ala Ala Lys Thr Leu
            820                 825                 830

Tyr Glu His Tyr Ser Gly Gly Glu Ser His Asn Ser Ser Ser Ser Lys
        835                 840                 845

Thr Phe Glu Lys Lys Arg Gly Lys Lys
    850                 855

<210>  3
<211>  5616
<212>  DNA
<213>  Homo sapiens

<220>
<221>  CDS
<222>  (247)..(3879)

<400>  3
ccccggcgca gcgcggccgc agcagcctcc gcccccgca cggtgtgagc gcccgacgcg      60

gccgaggcgg ccggagtccc gagctagccc cggcggccgc cgccgcccag accggacgac     120

aggccacctc gtcggcgtcc gcccgagtcc ccgcctcgcc gccaacgcca caaccaccgc     180

gcacggcccc ctgactccgt ccagtattga tcgggagagc cggagcgagc tcttcgggga     240

gcagcg atg cga ccc tcc ggg acg gcc ggg gca gcg ctc ctg gcg ctg       288
       
       1               5                   10

ctg gct gcg ctc tgc ccg gcg agt cgg gct ctg gag gaa aag aaa gtt      336

15                  20                  25                  30

tgc caa ggc acg agt aac aag ctc acg cag ttg ggc act ttt gaa gat      384

                35                  40                  45

cat ttt ctc agc ctc cag agg atg ttc aat aac tgt gag gtg gtc ctt      432


            50                  55                  60

ggg aat ttg gaa att acc tat gtg cag agg aat tat gat ctt tcc ttc 480

        65                  70                  75

tta aag acc atc cag gag gtg gct ggt tat gtc ctc att gcc ctc aac 528

    80                  85                  90

aca gtg gag cga att cct ttg gaa aac ctg cag atc atc aga gga aat 576

95                  100                 105                 110

atg tac tac gaa aat tcc tat gcc tta gca gtc tta tct aac tat gat 624

                115                 120                 125

gca aat aaa acc gga ctg aag gag ctg ccc atg aga aat tta cag gaa 672

            130                 135                 140

atc ctg cat ggc gcc gtg cgg ttc agc aac aac cct gcc ctg tgc aac 720

        145                 150                 155

gtg gag agc atc cag tgg cgg gac ata gtc agc agt gac ttt ctc agc 768

    160                 165                 170

aac atg tcg atg gac ttc cag aac cac ctg ggc agc tgc caa aag tgt 816


175                 180                 185                 190

gat cca agc tgt ccc aat ggg agc tgc tgg ggt gca gga gag gag aac 864

                195                 200                 205

tgc cag aaa ctg acc aaa atc atc tgt gcc cag cag tgc tcc ggg cgc 912

            210                 215                 220

tgc cgt ggc aag tcc ccc agt gac tgc tgc cac aac cag tgt gct gca 960

        225                 230                 235

ggc tgc aca ggc ccc cgg gag agc gac tgc ctg gtc tgc cgc aaa ttc 1008

    240                 245                 250

cga gac gaa gcc acg tgc aag gac acc tgc ccc cca ctc atg ctc tac 1056

255                 260                 265                 270

aac ccc acc acg tac cag atg gat gtg aac ccc gag ggc aaa tac agc 1104

                275                 280                 285

ttt ggt gcc acc tgc gtg aag aag tgt ccc cgt aat tat gtg gtg aca 1152

            290                 295                 300

gat cac ggc tcg tgc gtc cga gcc tgt ggg gcc gac agc tat gag atg 1200


        305                 310                 315

gag gaa gac ggc gtc cgc aag tgt aag aag tgc gaa ggg cct tgc cgc 1248

    320                 325                 330

aaa gtg tgt aac gga ata ggt att ggt gaa ttt aaa gac tca ctc tcc 1296

335                 340                 345                 350

ata aat gct acg aat att aaa cac ttc aaa aac tgc acc tcc atc agt 1344

                355                 360                 365

ggc gat ctc cac atc ctg ccg gtg gca ttt agg ggt gac tcc ttc aca 1392

            370                 375                 380

cat act cct cct ctg gat cca cag gaa ctg gat att ctg aaa acc gta 1440

        385                 390                 395

aag gaa atc aca ggg ttt ttg ctg att cag gct tgg cct gaa aac agg 1488

    400                 405                 410

acg gac ctc cat gcc ttt gag aac cta gaa atc ata cgc ggc agg acc 1536

415                 420                 425                 430

aag caa cat ggt cag ttt tct ctt gca gtc gtc agc ctg aac ata aca 1584


                435                 440                 445

tcc ttg gga tta cgc tcc ctc aag gag ata agt gat gga gat gtg ata 1632

            450                 455                 460

att tca gga aac aaa aat ttg tgc tat gca aat aca ata aac tgg aaa 1680

        465                 470                 475

aaa ctg ttt ggg acc tcc ggt cag aaa acc aaa att ata agc aac aga 1728

    480                 485                 490

ggt gaa aac agc tgc aag gcc aca ggc cag gtc tgc cat gcc ttg tgc 1776

495                 500                 505                 510

tcc ccc gag ggc tgc tgg ggc ccg gag ccc agg gac tgc gtc tct tgc 1824

                515                 520                 525

cgg aat gtc agc cga ggc agg gaa tgc gtg gac aag tgc aac ctt ctg 1872

            530                 535                 540

gag ggt gag cca agg gag ttt gtg gag aac tct gag tgc ata cag tgc 1920

        545                 550                 555

cac cca gag tgc ctg cct cag gcc atg aac atc acc tgc aca gga cgg 1968


    560                 565                 570

gga cca gac aac tgt atc cag tgt gcc cac tac att gac ggc ccc cac 2016

575                 580                 585                 590

tgc gtc aag acc tgc ccg gca gga gtc atg gga gaa aac aac acc ctg 2064

                595                 600                 605

gtc tgg aag tac gca gac gcc ggc cat gtg tgc cac ctg tgc cat cca 2112

            610                 615                 620

aac tgc acc tac gga tgc act ggg cca ggt ctt gaa ggc tgt cca acg 2160

        625                 630                 635

aat ggg cct aag atc ccg tcc atc gcc act ggg atg gtg ggg gcc ctc 2208

    640                 645                 650

ctc ttg ctg ctg gtg gtg gcc ctg ggg atc ggc ctc ttc atg cga agg 2256

655                 660                 665                 670

cgc cac atc gtt cgg aag cgc acg ctg cgg agg ctg ctg cag gag agg 2304

                675                 680                 685

gag ctt gtg gag cct ctt aca ccc agt gga gaa gct ccc aac caa gct 2352


            690                 695                 700

ctc ttg agg atc ttg aag gaa act gaa ttc aaa aag atc aaa gtg ctg 2400

        705                 710                 715

ggc tcc ggt gcg ttc ggc acg gtg tat aag gga ctc tgg atc cca gaa 2448

    720                 725                 730

ggt gag aaa gtt aaa att ccc gtc gct atc aag gaa tta aga gaa gca 2496

735                 740                 745                 750

aca tct ccg aaa gcc aac aag gaa atc ctc gat gaa gcc tac gtg atg 2544

                755                 760                 765

gcc agc gtg gac aac ccc cac gtg tgc cgc ctg ctg ggc atc tgc ctc 2592

            770                 775                 780

acc tcc acc gtg cag ctc atc acg cag ctc atg ccc ttc ggc tgc ctc 2640

        785                 790                 795

ctg gac tat gtc cgg gaa cac aaa gac aat att ggc tcc cag tac ctg 2688

    800                 805                 810

ctc aac tgg tgt gtg cag atc gca aag ggc atg aac tac ttg gag gac 2736


815                 820                 825                 830

cgt cgc ttg gtg cac cgc gac ctg gca gcc agg aac gta ctg gtg aaa 2784

                835                 840                 845

aca ccg cag cat gtc aag atc aca gat ttt ggg ctg gcc aaa ctg ctg 2832

            850                 855                 860

ggt gcg gaa gag aaa gaa tac cat gca gaa gga ggc aaa gtg cct atc 2880

        865                 870                 875

aag tgg atg gca ttg gaa tca att tta cac aga atc tat acc cac cag 2928

    880                 885                 890

agt gat gtc tgg agc tac ggg gtg acc gtt tgg gag ttg atg acc ttt 2976

895                 900                 905                 910

gga tcc aag cca tat gac gga atc cct gcc agc gag atc tcc tcc atc 3024

                915                 920                 925

ctg gag aaa gga gaa cgc ctc cct cag cca ccc ata tgt acc atc gat 3072

            930                 935                 940

gtc tac atg atc atg gtc aag tgc tgg atg ata gac gca gat agt cgc 3120


        945                 950                 955

cca aag ttc cgt gag ttg atc atc gaa ttc tcc aaa atg gcc cga gac 3168

    960                 965                 970

ccc cag cgc tac ctt gtc att cag ggg gat gaa aga atg cat ttg cca 3216

975                 980                 985                 990

agt cct aca gac tcc aac ttc tac cgt gcc ctg atg gat gaa gaa gac 3264

                995                 1000                1005

atg gac gac gtg gtg gat gcc gac gag tac ctc atc cca cag cag 3309

            1010                1015                1020

ggc ttc ttc agc agc ccc tcc acg tca cgg act ccc ctc ctg agc 3354

            1025                1030                1035

tct ctg agt gca acc agc aac aat tcc acc gtg gct tgc att gat 3399

            1040                1045                1050

aga aat ggg ctg caa agc tgt ccc atc aag gaa gac agc ttc ttg 3444

            1055                1060                1065

cag cga tac agc tca gac ccc aca ggc gcc ttg act gag gac agc 3489


            1070                1075                1080

ata gac gac acc ttc ctc cca gtg cct gaa tac ata aac cag tcc 3534

            1085                1090                1095

gtt ccc aaa agg ccc gct ggc tct gtg cag aat cct gtc tat cac 3579

            1100                1105                1110

aat cag cct ctg aac ccc gcg ccc agc aga gac cca cac tac cag 3624

            1115                1120                1125

gac ccc cac agc act gca gtg ggc aac ccc gag tat ctc aac act 3669

            1130                1135                1140

gtc cag ccc acc tgt gtc aac agc aca ttc gac agc cct gcc cac 3714

            1145                1150                1155

tgg gcc cag aaa ggc agc cac caa att agc ctg gac aac cct gac 3759

            1160                1165                1170

tac cag cag gac ttc ttt ccc aag gaa gcc aag cca aat ggc atc 3804

            1175                1180                1185

ttt aag ggc tcc aca gct gaa aat gca gaa tac cta agg gtc gcg 3849


            1190                1195                1200

cca caa agc agt gaa ttt att gga gca tga ccacggagga tagtatgagc 3899

            1205                1210

cctaaaaatc cagactcttt cgatacccag gaccaagcca cagcaggtcc tccatcccaa 3959

cagccatgcc cgcattagct cttagaccca cagactggtt ttgcaacgtt tacaccgact 4019

agccaggaag tacttccacc tcgggcacat tttgggaagt tgcattcctt tgtcttcaaa 4079

ctgtgaagca tttacagaaa cgcatccagc aagaatattg tccctttgag cagaaattta 4139

tctttcaaag aggtatattt gaaaaaaaaa aaaagtatat gtgaggattt ttattgattg 4199

gggatcttgg agtttttcat tgtcgctatt gatttttact tcaatgggct cttccaacaa 4259

ggaagaagct tgctggtagc acttgctacc ctgagttcat ccaggcccaa ctgtgagcaa 4319

ggagcacaag ccacaagtct tccagaggat gcttgattcc agtggttctg cttcaaggct 4379

tccactgcaa aacactaaag atccaagaag gccttcatgg ccccagcagg ccggatcggt 4439

actgtatcaa gtcatggcag gtacagtagg ataagccact ctgtcccttc ctgggcaaag 4499

aagaaacgga ggggatggaa ttcttcctta gacttacttt tgtaaaaatg tccccacggt 4559

acttactccc cactgatgga ccagtggttt ccagtcatga gcgttagact gacttgtttg 4619

tcttccattc cattgttttg aaactcagta tgctgcccct gtcttgctgt catgaaatca 4679

gcaagagagg atgacacatc aaataataac tcggattcca gcccacattg gattcatcag 4739

catttggacc aatagcccac agctgagaat gtggaatacc taaggatagc accgcttttg 4799

ttctcgcaaa aacgtatctc ctaatttgag gctcagatga aatgcatcag gtcctttggg 4859

gcatagatca gaagactaca aaaatgaagc tgctctgaaa tctcctttag ccatcacccc 4919

aacccccaa aattagtttg tgttacttat ggaagatagt tttctccttt tacttcactt 4979

caaaagcttt ttactcaaag agtatatgtt ccctccaggt cagctgcccc caaacccct 5039

ccttacgctt tgtcacacaa aaagtgtctc tgccttgagt catctattca agcacttaca 5099

gctctggcca caacagggca ttttacaggt gcgaatgaca gtagcattat gagtagtgtg 5159

gaattcaggt agtaaatatg aaactagggt ttgaaattga taatgctttc acaacatttg 5219

cagatgtttt agaaggaaaa aagttccttc ctaaaataat ttctctacaa ttggaagatt 5279

ggaagattca gctagttagg agcccacctt ttttcctaat ctgtgtgtgc cctgtaacct 5339

gactggttaa cagcagtcct ttgtaaacag tgttttaaac tctcctagtc aatatccacc 5399

ccatccaatt tatcaaggaa gaaatggttc agaaaatatt ttcagcctac agttatgttc 5459

agtcacacac acatacaaaa tgttcctttt gcttttaaag taatttttga ctcccagatc 5519

agtcagagcc cctacagcat tgttaagaaa gtatttgatt tttgtctcaa tgaaaataaa 5579

actatattca tttccactct aaaaaaaaaa aaaaaaa 5616

<210> 4
<211> 1210
<212> PRT
<213> Homo sapiens

<400> 4

<210> 5
<211> 21
<212> DNA

<213>  Artificial

<220>
<223>  An artificially synthesized primer sequence for RT-PCR

<400>  5
tcttgaatcc ctgtggaaat c                                                21

<210>  6
<211>  21
<212>  DNA
<213>  Artificial

<220>
<223>  An artificially synthesized primer sequence for RT-PCR

<400>  6
tgctatccac ccactattcc a                                                21

<210>  7
<211>  21
<212>  DNA
<213>  Artificial

<220>
<223>  An artificially synthesized primer sequence for RT-PCR

<400>  7
gaggtgatag cattgctttc g                                                21

<210> 8
<211> 21
<212> DNA
<213> Artificial

<220>
<223> An artificially synthesized primer sequence for RT-PCR

<400> 8
caagtcagtg tacaggtaag c 21

<210> 9
<211> 19
<212> DNA
<213> Artificial

<220>
<223> An artificially synthesized target sequence for siRNA

<400> 9
cgtacgcgga atacttcga 19

<210> 10
<211> 19
<212> DNA
<213> Artificial

<220>
<223> An artificially synthesized target sequence for siRNA

<400> 10
gcgcgctttg taggattcg 19

<210> 11
<211> 19
<212> DNA
<213> Artificial

<220>
<223> An artificially synthesized target sequence for siRNA

<400> 11
acagtgttcc gctaagtga 19

<210> 12
<211> 19
<212> DNA
<213> Artificial

<220>
<223> An artificially synthesized target sequence for siRNA

<400> 12
atcacggacc agtacatct 19

<210> 13
<211> 20
<212> DNA

<213>  Artificial

<220>
<223>  An artificially synthesized primer sequence for PCR

<400>  13
gtgtttgcgg aaaggagttt                                                    20


<210>  14
<211>  20
<212>  DNA
<213>  Artificial

<220>
<223>  An artificially synthesized primer sequence for PCR

<400>  14
caaccagtcc tctgggttgt                                                    20


<210>  15
<211>  22
<212>  DNA
<213>  Artificial

<220>
<223>  An artificially synthesized primer sequence for PCR

<400>  15
aactcgggaa ctgttaacca aa                                                 22

<210> 16
<211> 20
<212> DNA
<213> Artificial

<220>
<223> An artificially synthesized primer sequence for PCR

<400> 16
gtgcatcatc tggctcttga 20

<210> 17
<211> 20
<212> DNA
<213> Artificial

<220>
<223> An artificially synthesized primer sequence for PCR

<400> 17
tgttccgcta agtgatgctc 20

<210> 18
<211> 22
<212> DNA
<213> Artificial

<220>
<223> An artificially synthesized primer sequence for PCR

<400> 18
gcaaatttct tgcagtttgc tc 22

<210> 19
<211> 20
<212> DNA
<213> Artificial

<220>
<223> An artificially synthesized primer sequence for PCR

<400> 19
tcaagagcca gatgatgcac 20

<210> 20
<211> 21
<212> DNA
<213> Artificial

<220>
<223> An artificially synthesized primer sequence for PCR

<400> 20
tcttttcctc ctctgaaagc a 21

<210> 21
<211> 21
<212> DNA

<213>  Artificial

<220>
<223>  An artificially synthesized primer sequence for PCR

<400>  21
aagctgtaga acgtggagca g                                                21

<210>  22
<211>  20
<212>  DNA
<213>  Artificial

<220>
<223>  An artificially synthesized primer sequence for PCR

<400>  22
catcttgtgg tggcatgttc                                                  20

<210>  23
<211>  20
<212>  DNA
<213>  Artificial

<220>
<223>  An artificially synthesized primer sequence for PCR

<400>  23
cggttcactt gggcatttac                                                  20

<210> 24
<211> 20
<212> DNA
<213> Artificial

<220>
<223> An artificially synthesized primer sequence for PCR

<400> 24
ccaaatctcg gcattctgat 20

<210> 25
<211> 20
<212> DNA
<213> Artificial

<220>
<223> An artificially synthesized primer sequence for PCR

<400> 25
agcccagatt gtgatgtgaa 20

<210> 26
<211> 23
<212> DNA
<213> Artificial

<220>
<223> An artificially synthesized primer sequence for PCR

<400> 26
ttgattccgt tttattcttc agg 23

<210> 27
<211> 20
<212> DNA
<213> Artificial

<220>
<223> An artificially synthesized primer sequence for PCR

<400> 27
tcaaggaacc tctggtgtca 20

<210> 28
<211> 20
<212> DNA
<213> Artificial

<220>
<223> An artificially synthesized primer sequence for PCR

<400> 28
gacaggttgc tcaaaagtgg 20

<210> 29
<211> 20
<212> DNA

<213>  Artificial

<220>
<223>  An artificially synthesized primer sequence for PCR

<400>  29
actggcagat tccggagtta                                                    20

<210>  30
<211>  20
<212>  DNA
<213>  Artificial

<220>
<223>  An artificially synthesized primer sequence for PCR

<400>  30
caactgacaa gcaggtggaa                                                    20

<210>  31
<211>  23
<212>  DNA
<213>  Artificial

<220>
<223>  An artificially synthesized primer sequence for PCR

<400>  31
gacaccaagc agcaatacct tgg                                                23

<210> 32
<211> 25
<212> DNA
<213> Artificial

<220>
<223> An artificially synthesized primer sequence for PCR

<400> 32
aacacctgaa ataccttgct tgaac 25

<210> 33
<211> 22
<212> DNA
<213> Artificial

<220>
<223> An artificially synthesized primer sequence for PCR

<400> 33
atgaatgcat ttcggttaaa gg 22

<210> 34
<211> 21
<212> DNA
<213> Artificial

<220>
<223> An artificially synthesized primer sequence for PCR

<400> 34
tttccacact ccattaccat g                                                    21

<210> 35
<211> 21
<212> DNA
<213> Artificial

<220>
<223> An artificially synthesized primer sequence for PCR

<400> 35
acagtgataa gatcatccga c                                                    21

<210> 36
<211> 21
<212> DNA
<213> Artificial

<220>
<223> An artificially synthesized primer sequence for PCR

<400> 36
acacttgttg tatctggttg c                                                    21

<210> 37
<211> 22
<212> DNA

<213>  Artificial

<220>
<223>  An artificially synthesized primer sequence for PCR

<400>  37
tttctgatag ttgatggaat gc                                                22

<210>  38
<211>  23
<212>  DNA
<213>  Artificial

<220>
<223>  An artificially synthesized primer sequence for PCR

<400>  38
gaaatctgat taattatctg ctg                                               23

<210>  39
<211>  21
<212>  DNA
<213>  Artificial

<220>
<223>  An artificially synthesized primer sequence for PCR

<400>  39
tgatgtttgg tccttaggat g                                                 21

<210> 40
<211> 21
<212> DNA
<213> Artificial

<220>
<223> An artificially synthesized primer sequence for PCR

<400> 40
atttcttcag tggttccctt g 21

<210> 41
<211> 20
<212> DNA
<213> Artificial

<220>
<223> An artificially synthesized primer sequence for PCR

<400> 41
agctcctggc tcatccctat 20

<210> 42
<211> 1186
<212> PRT
<213> Homo sapiens

<400> 42

Leu Glu Glu Lys Lys Val Cys Gln Gly Thr Ser Asn Lys Leu Thr Gln
1               5                   10                  15

Leu Gly Thr Phe Glu Asp His Phe Leu Ser Leu Gln Arg Met Phe Asn
            20                  25                  30

Asn Cys Glu Val Val Leu Gly Asn Leu Glu Ile Thr Tyr Val Gln Arg
        35                  40                  45

Asn Tyr Asp Leu Ser Phe Leu Lys Thr Ile Gln Glu Val Ala Gly Tyr
    50                  55                  60

Val Leu Ile Ala Leu Asn Thr Val Glu Arg Ile Pro Leu Glu Asn Leu
65                  70                  75                  80

Gln Ile Ile Arg Gly Asn Met Tyr Tyr Glu Asn Ser Tyr Ala Leu Ala
                85                  90                  95

Val Leu Ser Asn Tyr Asp Ala Asn Lys Thr Gly Leu Lys Glu Leu Pro
            100                 105                 110

Met Arg Asn Leu Gln Glu Ile Leu His Gly Ala Val Arg Phe Ser Asn
        115                 120                 125

Asn Pro Ala Leu Cys Asn Val Glu Ser Ile Gln Trp Arg Asp Ile Val
    130                 135                 140
Ser Ser Asp Phe Leu Ser Asn Met Ser Met Asp Phe Gln Asn His Leu
145                 150                 155                 160
Gly Ser Cys Gln Lys Cys Asp Pro Ser Cys Pro Asn Gly Ser Cys Trp
                165                 170                 175
Gly Ala Gly Glu Glu Asn Cys Gln Lys Leu Thr Lys Ile Ile Cys Ala
            180                 185                 190
Gln Gln Cys Ser Gly Arg Cys Arg Gly Lys Ser Pro Ser Asp Cys Cys
        195                 200                 205
His Asn Gln Cys Ala Ala Gly Cys Thr Gly Pro Arg Glu Ser Asp Cys
    210                 215                 220
Leu Val Cys Arg Lys Phe Arg Asp Glu Ala Thr Cys Lys Asp Thr Cys
225                 230                 235                 240
Pro Pro Leu Met Leu Tyr Asn Pro Thr Thr Tyr Gln Met Asp Val Asn
                245                 250                 255

Pro Glu Gly Lys Tyr Ser Phe Gly Ala Thr Cys Val Lys Lys Cys Pro
            260                 265                 270

Arg Asn Tyr Val Val Thr Asp His Gly Ser Cys Val Arg Ala Cys Gly
        275                 280                 285

Ala Asp Ser Tyr Glu Met Glu Glu Asp Gly Val Arg Lys Cys Lys Lys
    290                 295                 300

Cys Glu Gly Pro Cys Arg Lys Val Cys Asn Gly Ile Gly Ile Gly Glu
305                 310                 315                 320

Phe Lys Asp Ser Leu Ser Ile Asn Ala Thr Asn Ile Lys His Phe Lys
                325                 330                 335

Asn Cys Thr Ser Ile Ser Gly Asp Leu His Ile Leu Pro Val Ala Phe
            340                 345                 350

Arg Gly Asp Ser Phe Thr His Thr Pro Pro Leu Asp Pro Gln Glu Leu
        355                 360                 365

Asp Ile Leu Lys Thr Val Lys Glu Ile Thr Gly Phe Leu Leu Ile Gln
    370                 375                 380

Ala Trp Pro Glu Asn Arg Thr Asp Leu His Ala Phe Glu Asn Leu Glu
385                 390                 395                 400

Ile Ile Arg Gly Arg Thr Lys Gln His Gly Gln Phe Ser Leu Ala Val
                405                 410                 415

Val Ser Leu Asn Ile Thr Ser Leu Gly Leu Arg Ser Leu Lys Glu Ile
            420                 425                 430

Ser Asp Gly Asp Val Ile Ile Ser Gly Asn Lys Asn Leu Cys Tyr Ala
        435                 440                 445

Asn Thr Ile Asn Trp Lys Lys Leu Phe Gly Thr Ser Gly Gln Lys Thr
    450                 455                 460

Lys Ile Ile Ser Asn Arg Gly Glu Asn Ser Cys Lys Ala Thr Gly Gln
465                 470                 475                 480

Val Cys His Ala Leu Cys Ser Pro Glu Gly Cys Trp Gly Pro Glu Pro
                485                 490                 495

Arg Asp Cys Val Ser Cys Arg Asn Val Ser Arg Gly Arg Glu Cys Val
            500                 505                 510

Asp Lys Cys Asn Leu Leu Glu Gly Glu Pro Arg Glu Phe Val Glu Asn
        515                 520                 525

Ser Glu Cys Ile Gln Cys His Pro Glu Cys Leu Pro Gln Ala Met Asn
    530                 535                 540

Ile Thr Cys Thr Gly Arg Gly Pro Asp Asn Cys Ile Gln Cys Ala His
545                 550                 555                 560

Tyr Ile Asp Gly Pro His Cys Val Lys Thr Cys Pro Ala Gly Val Met
                565                 570                 575

Gly Glu Asn Asn Thr Leu Val Trp Lys Tyr Ala Asp Ala Gly His Val
            580                 585                 590

Cys His Leu Cys His Pro Asn Cys Thr Tyr Gly Cys Thr Gly Pro Gly
        595                 600                 605

Leu Glu Gly Cys Pro Thr Asn Gly Pro Lys Ile Pro Ser Ile Ala Thr
    610                 615                 620

Gly Met Val Gly Ala Leu Leu Leu Leu Leu Val Val Ala Leu Gly Ile
625                 630                 635                 640

Gly Leu Phe Met Arg Arg Arg His Ile Val Arg Lys Arg Thr Leu Arg
                645                 650                 655

Arg Leu Leu Gln Glu Arg Glu Leu Val Glu Pro Leu Thr Pro Ser Gly
            660                 665                 670

Glu Ala Pro Asn Gln Ala Leu Leu Arg Ile Leu Lys Glu Thr Glu Phe
        675                 680                 685

Lys Lys Ile Lys Val Leu Gly Ser Gly Ala Phe Gly Thr Val Tyr Lys
    690                 695                 700

Gly Leu Trp Ile Pro Glu Gly Glu Lys Val Lys Ile Pro Val Ala Ile
705                 710                 715                 720

Lys Glu Leu Arg Glu Ala Thr Ser Pro Lys Ala Asn Lys Glu Ile Leu
                725                 730                 735

Asp Glu Ala Tyr Val Met Ala Ser Val Asp Asn Pro His Val Cys Arg
            740                 745                 750

Leu Leu Gly Ile Cys Leu Thr Ser Thr Val Gln Leu Ile Thr Gln Leu
        755                 760                 765

Met Pro Phe Gly Cys Leu Leu Asp Tyr Val Arg Glu His Lys Asp Asn
    770                 775                 780

Ile Gly Ser Gln Tyr Leu Leu Asn Trp Cys Val Gln Ile Ala Lys Gly
785                 790                 795                 800

Met Asn Tyr Leu Glu Asp Arg Arg Leu Val His Arg Asp Leu Ala Ala
                805                 810                 815

Arg Asn Val Leu Val Lys Thr Pro Gln His Val Lys Ile Thr Asp Phe
            820                 825                 830

Gly Leu Ala Lys Leu Leu Gly Ala Glu Glu Lys Glu Tyr His Ala Glu
        835                 840                 845

Gly Gly Lys Val Pro Ile Lys Trp Met Ala Leu Glu Ser Ile Leu His
    850                 855                 860

Arg Ile Tyr Thr His Gln Ser Asp Val Trp Ser Tyr Gly Val Thr Val
865                 870                 875                 880

Trp Glu Leu Met Thr Phe Gly Ser Lys Pro Tyr Asp Gly Ile Pro Ala
                885                 890                 895

Ser Glu Ile Ser Ser Ile Leu Glu Lys Gly Glu Arg Leu Pro Gln Pro
            900                 905                 910

Pro Ile Cys Thr Ile Asp Val Tyr Met Ile Met Val Lys Cys Trp Met
        915                 920                 925

Ile Asp Ala Asp Ser Arg Pro Lys Phe Arg Glu Leu Ile Ile Glu Phe
    930                 935                 940

Ser Lys Met Ala Arg Asp Pro Gln Arg Tyr Leu Val Ile Gln Gly Asp
945                 950                 955                 960

Glu Arg Met His Leu Pro Ser Pro Thr Asp Ser Asn Phe Tyr Arg Ala
                965                 970                 975

Leu Met Asp Glu Glu Asp Met Asp Asp Val Val Asp Ala Asp Glu Tyr
            980                 985                 990

Leu Ile Pro Gln Gln Gly Phe Phe Ser Ser Pro Ser Thr Ser Arg Thr
        995                 1000                1005

Pro Leu Leu Ser Ser Leu Ser Ala Thr Ser Asn Asn Ser Thr Val
    1010                1015                1020

Ala Cys Ile Asp Arg Asn Gly Leu Gln Ser Cys Pro Ile Lys Glu
    1025                1030                1035

Asp Ser Phe Leu Gln Arg Tyr Ser Ser Asp Pro Thr Gly Ala Leu
    1040                1045                1050

Thr Glu Asp Ser Ile Asp Asp Thr Phe Leu Pro Val Pro Glu Tyr
    1055                1060                1065

Ile Asn Gln Ser Val Pro Lys Arg Pro Ala Gly Ser Val Gln Asn
    1070                1075                1080

Pro Val Tyr His Asn Gln Pro Leu Asn Pro Ala Pro Ser Arg Asp
    1085                1090                1095

Pro His Tyr Gln Asp Pro His Ser Thr Ala Val Gly Asn Pro Glu
    1100                1105                1110

Tyr Leu Asn Thr Val Gln Pro Thr Cys Val Asn Ser Thr Phe Asp
    1115                1120                1125

Ser Pro Ala His Trp Ala Gln Lys Gly Ser His Gln Ile Ser Leu
    1130                1135                1140

Asp Asn Pro Asp Tyr Gln Gln Asp Phe Phe Pro Lys Glu Ala Lys
    1145                1150                1155

Pro Asn Gly Ile Phe Lys Gly Ser Thr Ala Glu Asn Ala Glu Tyr
    1160                1165                1170

Leu Arg Val Ala Pro Gln Ser Ser Glu Phe Ile Gly Ala
    1175                1180                1185

<210> 43
<211> 157
<212> PRT
<213> Artificial

<220>
<223> An artificially synthesized EGFP fragment

<400> 43

Lys Pro Tyr Asp Gly Ile Pro Ala Ser Glu Ile Ser Ser Ile Leu Glu
1               5                   10                  15

Lys Gly Glu Arg Leu Pro Gln Pro Pro Ile Cys Thr Ile Asp Val Tyr
            20                  25                  30

Met Ile Met Val Lys Cys Trp Met Ile Asp Ala Asp Ser Arg Pro Lys
        35                  40                  45

Phe Arg Glu Leu Ile Ile Glu Phe Ser Lys Met Ala Arg Asp Pro Gln
    50                  55                  60

Arg Tyr Leu Val Ile Gln Gly Asp Glu Arg Met His Leu Pro Ser Pro
65                  70                  75                  80

Thr Asp Ser Asn Phe Tyr Arg Ala Leu Met Asp Glu Glu Asp Met Asp
                85                  90                  95

Asp Val Val Asp Ala Asp Glu Tyr Leu Ile Pro Gln Gln Gly Phe Phe
            100                 105                 110

Ser Ser Pro Ser Thr Ser Arg Thr Pro Leu Leu Ser Ser Leu Ser Ala
        115                 120                 125

Thr Ser Asn Asn Ser Thr Val Ala Cys Ile Asp Arg Asn Gly Leu Gln
    130                 135                 140

Ser Cys Pro Ile Lys Glu Asp Ser Phe Leu Gln Arg Tyr
145                 150                 155

<210> 44
<211> 19

<212>  PRT
<213>  Artificial

<220>
<223>  An artificially synthesised dominant negative peptide

<400>  44

Ile Ser Ser Ile Leu Glu Lys Gly Glu Arg Leu Pro Gln Pro Pro Ile
1               5                   10                  15

Cys Thr Ile

<210>  45
<211>  19
<212>  PRT
<213>  Artificial

<220>
<223>  An artificially synthesised dominant negative peptide

<400>  45

Asp Val Tyr Met Ile Met Val Lys Cys Trp Met Ile Asp Ala Asp Ser
1               5                   10                  15

Arg Pro Lys

<210> 46
<211> 19
<212> PRT
<213> Artificial

<220>
<223> An artificially synthesised dominant negative peptide

<400> 46

Phe Arg Glu Leu Ile Ile Glu Phe Ser Lys Met Ala Arg Asp Pro Gln
1               5                   10                  15

Arg Tyr Leu

<210> 47
<211> 9
<212> PRT
<213> Artificial

<220>
<223> an artificially synthesized Tat sequence

<400> 47

Arg Lys Lys Arg Arg Gln Arg Arg Arg
1               5

<210> 48
<211> 16
<212> PRT
<213> Artificial

<220>
<223> an artificially synthesized Penetratin sequence

<400> 48

Arg Gln Ile Lys Ile Trp Phe Gln Asn Arg Arg Met Lys Trp Lys Lys
1               5                   10                  15

<210> 49
<211> 21
<212> PRT
<213> Artificial

<220>
<223> an artificially synthesized Buforin II sequence

<400> 49

Thr Arg Ser Ser Arg Ala Gly Leu Gln Phe Pro Val Gly Arg Val His
1               5                   10                  15

Arg Leu Leu Arg Lys
            20

<210>  50
<211>  27
<212>  PRT
<213>  Artificial

<220>
<223>  an artificially synthesized Transportan sequence

<400>  50

Gly Trp Thr Leu Asn Ser Ala Gly Tyr Leu Leu Gly Lys Ile Asn Leu
1               5                   10                  15

Lys Ala Leu Ala Ala Leu Ala Lys Lys Ile Leu
            20                  25

<210>  51
<211>  18
<212>  PRT
<213>  Artificial

<220>
<223>  an artificially synthesized MAP (model amphipathic peptide)
       sequence

<400>  51

Lys Leu Ala Leu Lys Leu Ala Leu Lys Ala Leu Lys Ala Ala Leu Lys

1                   5                   10                  15

Leu Ala

<210>  52
<211>  16
<212>  PRT
<213>  Artificial

<220>
<223>  an artificially synthesized K-FGF sequence

<400>  52

Ala Ala Val Ala Leu Leu Pro Ala Val Leu Leu Ala Leu Leu Ala Pro
1                   5                   10                  15

<210>  53
<211>  5
<212>  PRT
<213>  Artificial

<220>
<223>  an artificially synthesized Ku70 sequence

<400>  53

Val Pro Met Leu Lys

1               5

<210>  54
<211>  28
<212>  PRT
<213>  Artificial

<220>
<223>  an artificially synthesized Prion sequence

<400>  54

Met Ala Asn Leu Gly Tyr Trp Leu Leu Ala Leu Phe Val Thr Met Trp
1               5                   10                  15

Thr Asp Val Gly Leu Cys Lys Lys Arg Pro Lys Pro
            20                  25

<210>  55
<211>  18
<212>  PRT
<213>  Artificial

<220>
<223>  an artificially synthesized pVEC sequence

<400>  55

Leu Leu Ile Ile Leu Arg Arg Arg Ile Arg Lys Gln Ala His Ala His

1               5                   10                  15

Ser Lys

<210>  56
<211>  21
<212>  PRT
<213>  Artificial

<220>
<223>  an artificially synthesized Pep-1 sequence

<400>  56

Lys Glu Thr Trp Trp Glu Thr Trp Trp Thr Glu Trp Ser Gln Pro Lys
1               5                   10                  15

Lys Lys Arg Lys Val
            20

<210>  57
<211>  18
<212>  PRT
<213>  Artificial

<220>
<223>  an artificially synthesized SynB1 sequence

<400>  57

Arg Gly Gly Arg Leu Ser Tyr Ser Arg Arg Arg Phe Ser Thr Ser Thr
1               5                   10                  15

Gly Arg

<210>  58
<211>  15
<212>  PRT
<213>  Artificial

<220>
<223>  an artificially synthesized Pep-7 sequence

<400>  58

Ser Asp Leu Trp Glu Met Met Met Val Ser Leu Ala Cys Gln Tyr
1               5                   10                  15

<210>  59
<211>  12
<212>  PRT
<213>  Artificial

<220>
<223>  an artificially synthesized HN-1 sequence

<400>  59

Thr Ser Pro Leu Asn Ile His Asn Gly Gln Lys Leu
1               5                   10

<210>  60
<211>  11
<212>  PRT
<213>  Artificial

<220>
<223>  an artificially synthesized 11 mer poly-arginine sequence

<400>  60

Arg Arg Arg Arg Arg Arg Arg Arg Arg Arg Arg
1               5                   10

<210>  61
<211>  5
<212>  PRT
<213>  Artificial

<220>
<223>  an artificially synthesized Ku70 sequence

<400>  61

Pro Met Leu Lys Glu

1               5

<210>  62
<211>  21
<212>  DNA
<213>  Artificial

<220>
<223>  An artificially synthesized target sequence for siRNA

<400>  62
caagatgtgt taaagtgttt t                                                    21

<210>  63
<211>  21
<212>  DNA
<213>  Artificial

<220>
<223>  An artificially synthesized target sequence for siRNA

<400>  63
gtaacaagct cacgcagttt t                                                    21